# EUROPEAN PATENT SPECIFICATION

(11) **EP 1 952 293 B1**
(45) Date of publication and mention of the grant of the patent: **05.01.2011**
(21) Application number: 06829959.3
(22) Date of filing: 08.11.2006
(51) Int. Cl.: G06F 19/00, C12N 15/11, C12N 15/63, C12N 5/10, C12Q 1/68, G01N 33/50, A61K 31/7105

(54) **METHODS FOR THE IDENTIFICATION OF MICRORNA AND THEIR APPLICATIONS IN RESEARCH AND HUMAN HEALTH**
VERFAHREN ZUR MIKRORNA-IDENTIFIKATION UND IHRER ANWENDUNGEN BEI DER FORSCHUNG UND MENSCHLICHEN GESUNDHEIT
PROCEDES D'IDENTIFICATION DU MICROARN ET LEURS APPLICATIONS EN TERMES DE RECHERCHE ET DE SANTE HUMAINE

(30) Priority: 08.11.2005 EP 05292359
(43) Date of publication of application: 06.08.2008
(60) Divisional application: 10014063.1
(73) Proprietor: Cepheid, Sunnyvale, CA 94089 (US)
(72) Inventor: DELFOUR, Olivier, 31460 Caraman (FR); CIUTI, Jérôme, F-31400 Toulouse (FR); DENOUAL, Florent, F-31400 Toulouse (FR); VILANOVA, David, F-31300 Toulouse (FR); MICHOT, Bernard, F-46170 Pern (FR)
(74) Representative: Campbell, Patrick John Henry
(86) International application number: PCT/EP2006/068246
(87) International publication number: WO 2007/054520

(56) References cited:
- LAI E C ET AL: "COMPUTATIONAL IDENTIFICATION OF DROSOPHILA MICRORNA GENES" GENOME BIOLOGY (ONLINE), GB, vol. 4, no. 7, 2003, page R42, XP002370305 ISSN: 1465-6914
- SMALHEISER NEIL R ET AL: "Mammalian microRNAs derived from genomic repeats." TRENDS IN GENETICS : TIG JUN 2005, vol. 21, no. 6, June 2005 (2005-06), pages 322-326, XP004911546 ISSN: 0168-9525
- LLAVE CESAR ET AL: "Endogenous and silencing-associated small RNAs in plants" PLANT CELL, AMERICAN SOCIETY OF PLANT PHYSIOLOGISTS, ROCKVILLE, MD, US, vol. 14, no. 7, July 2002 (2002-07), pages 1605-1619, XP002387504 ISSN: 1040-4651
- GRAD Y ET AL: "COMPUTATIONAL AND EXPERIMENTAL IDENTIFICATION OF C. ELEGANS MICRORNAS" MOLECULAR CELL, CELL PRESS, CAMBRIDGE, MA, US, vol. 11, no. 5, 1 May 2003 (2003-05-01), pages 1253-1263, XP009032050 ISSN: 1097-2765
- BENTWICH I ET AL: "IDENTIFICATION OF HUNDREDS OF CONSERVED AND NONCONSERVED HUMAN MICRORNAS" NATURE GENETICS, NATURE AMERICA, NEW YORK, US, vol. 37, no. 7, July 2005 (2005-07), pages 766-770, XP009059361 ISSN: 1061-4036
- BEREZIKOV EUGENE ET AL: "Phylogenetic shadowing and computational identification of human microRNA genes" CELL, vol. 120, no. 1, 14 January 2005 (2005-01-14), pages 21-24, XP002370262 ISSN: 0092-8674
- SEWER ALAIN ET AL: "Identification of clustered microRNAs using an ab initio prediction method -" BMC BIOINFORMATICS, vol. 6, 7 November 2005 (2005-11-07), pages 1-15, XP002370286 ISSN: 1471-2105
- XIE XIAOHUI ET AL: "Systematic discovery of regulatory motifs in human promoters and 3' UTRs by comparison of several mammals" NATURE (LONDON), vol. 434, no. 7031, 17 March 2005 (2005-03-17), pages 338-345, XP002370287 ISSN: 0028-0836

### SEQUENCE LISTING

<110> CEPHEID
<120> Methods for the identification of microRNA and their applications in research and human health
<130> N104759
<160> 605
<170> PatentIn version 3.3
<210> 3
   <211> 89
   <212> DNA
   <213> Homo sapiens
<400> 3
   ccgggcgccc ccgagagccg gccctccctt cctccgtgac aggtgggcct ggagttgggg 60
   aaagtttgga gccggcgagg ggcgccggg 89
<210> 5
   <211> 80
   <212> DNA
   <213> Homo sapiens
<400> 5
   agctctgcac ctcctccagg ggatgaatct catcttcaga caatgttaaa ctcattctct 60
   tgagcagagg tgaaaaagct 80
<210> 8
   <211> 78
   <212> DNA
   <213> Homo sapiens
<400> 8
   ggatgcaacc gtggaagccg gtgccgttga ggatctgcca caggcggaag gcagctgagt 60
   tgacatccac gggcatcc 78
<210> 16
   <211> 87
   <212> DNA
   <213> Homo sapiens
<400> 16
   ggcatacaca aggatgacat agatttgaca aaaaattaat gatttgtaca tcactttaat 60
   gcctcaaatc tgcaattcat gtatgct 87
<210> 22

   <211> 84
   <212> DNA
   <213> Homo sapiens
<400> 22
   tgtttccagc attcccaggt aggccaaggt gtcctacaga aaaaccttgg gttagaccta 60
   cagggggtct ggctggtgtt aaca 84
<210> 27
   <211> 71
   <212> DNA
   <213> Homo sapiens
<400> 27
   ggtctcctgc tctgctgatc agtgtctcat agggcaagtc ctgagggatc tgggacaaca 60
   ggtggtggac c 71
<210> 32
   <211> 87
   <212> DNA
   <213> Homo sapiens
<400> 32
   actttttctg gtgggagggg agagcggagc aggctcacgt gtaaccgcgc aggagcctcc 60
   tctggcttga gccctttctt ggtaagt 87
<210> 34
   <211> 80
   <212> DNA
   <213> Homo sapiens
<400> 34
   ggtccagata acagaagaga gagcaaagga aaaagaattt tttgaagatc aaaagtggct 60
   gttcattttg ttatctgacc 80
<210> 35
   <211> 86
   <212> DNA

   <213> Homo sapiens
<400> 35
   gatggtgggc atgggtcaga aggactccta cgtgggcgac aagggcccag agcaagagag 60
   gcattctgac cctgaagtac cgcatc 86
<210> 36
   <211> 73
   <212> DNA
   <213> Homo sapiens
<400> 36
   attccaaggg tctgacactt gtctaaatcc tgtagaaatg agatcaaggg caaagggcac 60
   agatcctggg aat 73
<210> 60
   <211> 85
   <212> DNA
   <213> Homo sapiens
<400> 60
   accttctgcc tttgagacct ggaagccaca gcactggtgg acataaattc tactgcctgc      60
   tgctccagat ccaaggggag agggt 85
<210> 69
   <211> 89
   <212> DNA
   <213> Homo sapiens
<400> 69
   acccagagtc aattactcac tgtgtgaatc acccccaaaa gattgctttt tccttttcca 60
   ttagtatcac acataattgc actctgtgt 89
<210> 70
   <211> 88
   <212> DNA
   <213> Homo sapiens
<400> 70
   cagttagcca gagtggtctg gacacacaga tttatcttcc ctcttcttca gtgaagaaca 60
   tctatcttcc atgacctctt gattactg 88
<210> 77
   <211> 83
   <212> DNA
   <213> Homo sapiens
<400> 77
   atggcagaga tattgacata gtgagaaaac attgccttca acaatagggc tttgttgaca 60
   aaacagtgaa atgtctagac cat 83
<210> 81
   <211> 86
   <212> DNA
   <213> Homo sapiens
<400> 81
   gtgtattgtg agtgtccctt gccatatttc tgctaatgtg tttcatctgg agtacataaa 60
   gaggcaggga tgctgatttt agacac 86
<210> 82
   <211> 82
   <212> DNA
   <213> Homo sapiens
<400> 82
   tgaaaatcat aagccctaag ctttagagag agttgtgcaa ttgacaatca aatggagctt 60
   aaagggaatt aggaggtttt ca 82
<210> 90
   <211> 72
   <212> DNA
   <213> Homo sapiens
<400> 90
   cccacgcacg gagggtcgcc aggaaagtgg acattaccgc tttaattaac ttcgagatgc 60
   tccggcggcg gg 72
<210> 93
   <211> 82
   <212> DNA
   <213> Homo sapiens
<400> 93
   ggaagaaacg gatcgcatgg ctcatttatc ttgcagccat taattaatgg tggttaattg 60
   tagttatgag ctgttgaatt cc 82
<210> 97
   <211> 90
   <212> DNA
   <213> Homo sapiens
<400> 97
   gaataccatt aatctgttca ctaggagatt aatttgcaat ttgttggcaa atcacatgtg 60
   ggtcttgtag caagagcgtg ggtggtgttt 90
<210> 139
   <211> 89
   <212> DNA
   <213> Homo sapiens
<400> 139
   gctggctcct gctcagagtg acagcaccct gtggagtctg ctggtactga cctaaccacc 60
   acagagcccc actggagcat ggagccagc 89
<210> 141
   <211> 83
   <212> DNA
   <213> Homo sapiens
<400> 141
   tgatcaggcg cttgatatga caggctgtgg gagtcaggac cccttgaaca gagtgggcct 60
   tgtctgtcac agtccgcctg aca 83
<210> 151
   <211> 74
   <212> DNA
   <213> Homo sapiens
<400> 151
   agtgtagcat tttaagtagg tgttataaat ctctgccaaa ggcacattca tagccctgct 60
   tgaatagttt catt 74
<210> 152
   <211> 80
   <212> DNA
   <213> Homo sapiens
<400> 152
   ttccattttc ctttaattaa ggctctgtgg aggtgtgttt gaaaacagag ggtctcctat 60
   gcctttgagg agggtgagaa 80
<210> 158
   <211> 71
   <212> DNA
   <213> Homo sapiens
<400> 158
   tgacgactgg ccccgcctct tcctctcggt cccatattga actcgagttg gaagaggcga 60
   gtccggtctc a 71
<210> 163
   <211> 79
   <212> DNA
   <213> Homo sapiens
<400> 163
   tgtgagtggg tttggggtga ggctatgggg agggcggggt gccgccttgc ccagcccctg 60
   agggccccag cccagtaca 79
<210> 170
   <211> 88
   <212> DNA
   <213> Homo sapiens
<400> 170
   tggagattgt gtgtctggct atgtcaggcc agggtggccc tttttaggca gaccattaag 60
   gcaatctggc tgccagatga agtcttca 88
<210> 175
   <211> 77
   <212> DNA
   <213> Homo sapiens
<400> 175
   cacggggacc ttctggaggt aaatctgcag aatggcattt tgtttgtgaa ttctcggatc 60
   gaccgcgaga agctgtg 77
<210> 176
   <211> 85
   <212> DNA
   <213> Homo sapiens
<400> 176
   tgcaggaaaa tcagtaacat tagtcatctt aaaagggtta ttactcaaga tgatttaaat 60
   tgtgaaaatg tcagtggagc ctgca 85
<210> 178
   <211> 76
   <212> DNA
   <213> Homo sapiens
<400> 178
   tgttttgaat tgcaaatatt cagttttgca tatatttaaa agttattttg acagtatgta 60
   atgtagattt aaaaca 76
<210> 183
   <211> 84
   <212> DNA
   <213> Homo sapiens
<400> 183
   ttgattgatt tgtaacactt taaaaaacat tttctaggca aagcacatta ggagagtgtt 60
   ttataatgta ttcagtttaa ttaa 84
<210> 184
   <211> 73
   <212> DNA
   <213> Homo sapiens
<400> 184
   cattgtctga attactgaag gtcaacttat ctgataaaat aggtgagctt tgtggtaatg 60
   aagtcagata atg 73
<210> 187
   <211> 88
   <212> DNA
   <213> Homo sapiens
<400> 187
   gaatttgccc tacggtgtga ccccagcctc tccctctggc cacagccagg gccggcgggg 60
   ggcctctggg agcatcttca gcaagttc 88
<210> 189
   <211> 71
   <212> DNA
   <213> Homo sapiens
<400> 189
   ttggtgacgt ctccatgttg tattgtctca agcacatatg gctggacagc aaacatggag 60
   agaatcatga a 71
<210> 191
   <211> 74
   <212> DNA
   <213> Homo sapiens
<400> 191
   tctggcaggc ggtaattgct ggccagctgc agggattaca gccctgtgag ctgtgttcag 60
   ggccctgtgc cagg 74
<210> 192
   <211> 80
   <212> DNA
   <213> Homo sapiens
<400> 192
   gttggaaggc acagttaaag ggtcatgtga ccaaggaggg aaatgccttt gtcacatgac 60
   tttttggtgg cctgattagc 80
<210> 200
   <211> 84
   <212> DNA
   <213> Homo sapiens
<400> 200
   tggtgctttg aattaatgtg caagctgtta atctccgtag acctggattt aaaattagac 60
   aaagcaataa ttagtttggc acca 84
<210> 205
   <211> 73
   <212> DNA
   <213> Homo sapiens
<400> 205
   gtgcataagg gaagaagaca agaaaatgat attgtcgttt aatagttcac tttagatctt 60
   catctcttat cac 73
<210> 207
   <211> 72
   <212> DNA
   <213> Homo sapiens
<400> 207
   ggctcggttt caaatctctc ctaatccact aatgaacctt tattaaagtg ggagagagag 60
   gttgaatcag tc 72
<210> 219
   <211> 62
   <212> DNA
   <213> Homo sapiens
<400> 219
   gggccgggca gcgtggtgcc ctggctgggt ccggccgcgg ggcgcccgtc ccgcccgcgc 60
   cc 62
<210> 221
   <211> 77
   <212> DNA
   <213> Homo sapiens
<400> 221
   ggctctgcac caggcgtttc ttcttgtgtt tcctcttctc ttctggagag ggatgaagga 60
   gatcctttgt gagaggc 77
<210> 223
   <211> 83
   <212> DNA
   <213> Homo sapiens
<400> 223
   agtgggggca cgttcaagaa tgctaatgaa ttatcagttt tctttaagcc tcattagatt 60
   tcttgatagc aaaacctccc att 83
<210> 224
   <211> 77
   <212> DNA
   <213> Homo sapiens
<400> 224
   gtctgtcacc cttgattgga aagtaataat tcataaaact tgatgttatt atcgttcctc 60
   tgcctggagt gatagat 77
<210> 229
   <211> 71
   <212> DNA
   <213> Homo sapiens
<400> 229
   gggagtgggg ggatctgttt ggcaactgga gtgaagggat tgccctcccc ctgctgggat 60
   ccccccagcc c 71
<210> 248
   <211> 78
   <212> DNA
   <213> Homo sapiens
<400> 248
   tggcctgacg tgaggaggag ggacttttcg aagttttata ggaaagtttc cgctttccag 60
   tccccctccc ccgtccca 78
<210> 251
   <211> 78
   <212> DNA
   <213> Home sapiens
<400> 251
   agcacccgga gctggcagac aagaatgtgc ccaaccttca tgtcatgaag gccatgcagt 60
   cttccaagtc ccggggct 78
<210> 256
   <211> 74
   <212> DNA
   <213> Homo sapiens
<400> 256
   ggctctggga aagccttcct ttcccggctg gcctggcatt caaagccaga caaagggggg 60
   ctttctctct cgcc 74
<210> 259
   <211> 82
   <212> DNA
   <213> Homo sapiens
<400> 259
   gcccatttcc ctaatggcag ccgattgcca tttgctattc aaatcagact agataaatta 60
   cagtacatgc cagggaacag gc 82
<210> 261
   <211> 87
   <212> DNA
   <213> Homo sapiens
<400> 261
   ggcgatgcca gggcccctcc cccaccattg ttctggagag accataggtc catctgttgc 60
   cccatggtgg ggcccctagg cagtgcc 87
<210> 275
   <211> 74
   <212> DNA
   <213> Homo sapiens
<400> 275
   aattttaatt agtatttttg gctttaatga aaagtgatta caaaattggc attttagcta 60
   atactgttga gatt 74
<210> 276
   <211> 76
   <212> DNA
   <213> Homo sapiens
<400> 276
   tctgcctggt gcatctcttg ccaatcaaat cagtgctgtc tgcagtgtga ttgctgcttt 60
   agtggcacca gggaga 76
<210> 316
   <211> 73
   <212> DNA
   <213> Homo sapiens
<400> 316
   gtaaatttca acctattttt aagggttatt ttcactcaag tgaaatctat caaggaagga 60
   gggttatttt tac 73
<210> 323
   <211> 86
   <212> DNA
   <213> Homo sapiens
<400> 323
   gcacagattt taattttatc ccagttatgg atatctggcg tgtgcgctga tactctgggc 60
   agactggtgt gaaatttaat ttgagc 86
<210> 328
   <211> 80
   <212> DNA
   <213> Homo sapiens
<400> 328
   ggctggccag gctccgcccc cggccctccc tgcgcccggc cggtgcgcag ggaggtcgga 60
   ggagcgggca ctgcccaccc 80
<210> 333
   <211> 74
   <212> DNA
   <213> Homo sapiens
<400> 333
   ggtcccagaa ctgtttgctt caattggcca tttaagtcta atagtaaaag actggttaat 60
   gataacagtg gatc 74
<210> 340
   <211> 75
   <212> DNA
   <213> Homo sapiens
<400> 340
   aaacgggtgg cagttattgc tggagtccaa gtggctgaat cgcatgcttg gaaataggag 60
   tatattcatc tgttt 75
<210> 344
   <211> 90
   <212> DNA
   <213> Homo sapiens
<400> 344
   tacctctcac ctggcagtga ccttagtatg ttttcatgtt tatctaaaaa agataaaagg 60
   tatgattgct caggaatgaa aacatgggta 90
<210> 357
   <211> 76
   <212> DNA
   <213> Homo sapiens
<400> 357
   gctctattat acagaacaga aggaacattt ggtgggttga atacttgtta atttatttct 60
   ctgtttgtta atgggc 76
<210> 363
   <211> 77
   <212> DNA
   <213> Homo sapiens
<400> 363
   gctggggttc atcggagaaa ctccctgcga tgagccacta gggtcacgga cagggaactt 60
   tttgatgagc gccgagt 77
<210> 366
   <211> 85
   <212> DNA
   <213> Homo sapiens
<400> 366
   gtacttggtt tttctaattt gacagaggat aaaagaaggc tttcgcggct ttgttttatt 60
   tctaaactca tttagcacca agtgc 85
<210> 368
   <211> 84
   <212> DNA
   <213> Homo sapiens
<400> 368
   gtgatgtgat ttctgcccag tgctctgaat gtcaaactga agaaattcag tgaaatgcgg 60
   gtaaacggcg ggagtaacta tgac 84
<210> 384
   <211> 77
   <212> DNA
   <213> Homo sapiens
<400> 384
   cccaagctcc ttcctggagg acttaacact gtgttgagca gttgttttga atttccgggc 60
   agggggctgc aaaaggg 77
<210> 385
   <211> 79
   <212> DNA
   <213> Homo sapiens
<400> 385
   ctttaatatt caatgacata acttcattgc gggtttgaca acactatata gaagttgtgc 60
   ttaccactga aaattaaag 79
<210> 390
   <211> 84
   <212> DNA
   <213> Homo sapiens
<400> 390
   ttccccaggc agcagcaggc gcacggccgt ctggatctcc ctggaggtga tggtcgagcg 60
   cttgtcataa tgcgccaggc ggga 84
<210> 400
   <211> 81
   <212> DNA
   <213> Homo sapiens
<400> 400
   ccttttatat cgacagttgg tagcacaagc cttttaactc acaaagggta gcagctcttc 60
   agccaatctg tgataaaagg g 81
<210> 406
   <211> 69
   <212> DNA
   <213> Homo sapiens
<400> 406
   gggagtgtgc tgggaactgg gcagataaaa agggctggaa cttattattt ggctagactc 60
   tcaattccc 69
<210> 407
   <211> 78
   <212> DNA
   <213> Homo sapiens

<400> 407
   ggagcccttg atgtcctgca aatgaaggag gaggatgtcc ttaagttcct tgcagcagga 60
   acccacttag gtggcacc 78
<210> 425
   <211> 72
   <212> DNA
   <213> Homo sapiens
<400> 425
   tgggctactg ttgtgaatga taatcaagta gagctctatc atttgattac aagcctcatt 60
   cagtacagcc ca 72
<210> 453
   <211> 86
   <212> DNA
   <213> Homo sapiens
<400> 453
   acagaccagg cacagaaagc tgaaggtgct ggagatgcca agtgaagcgt gtgcattttt 60
   ggtgactgtg tacttctggt gactgt 86
<210> 458
   <211> 85
   <212> DNA
   <213> Homo sapiens
<400> 458
   ggagcccgag aaactaagag aaagtgtcag gagcatgtta atcagactcg ttacactgta 60
   acaataacgt ctctctcggg tctcc 85
<210> 472
   <211> 85
   <212> DNA
   <213> Homo sapiens
<400> 472
   ggtgtgtctg cctctctttc tgccccccta taccccttga ccccaggggg aaggcactcg 60
   ggcagcacaa agggagcaga tgccc 85
<210> 481
   <211> 81
   <212> DNA
   <213> Homo sapiens
<400> 481
   tgtgaagcca caggaagggg ctctgtgaca tcacaggtag gggcagtgtg aagtcacagg 60
   aaggggctgt gggaagtcac a 81
<210> 489
   <211> 76
   <212> DNA
   <213> Homo sapiens
<400> 489
   agcccaggtt cacatggtta ccacattatt tggctaaagc tattaatctc tgtggtcagg 60
   cctgtaaagc tggtct 76
<210> 494
   <211> 77
   <212> DNA
   <213> Homo sapiens
<400> 494
   cttgttagac aatcacagct gggggccagg ggtaaattta gcctttgttc agcccccagt 60
   tatgtgtggg atgcagg 77
<210> 500
   <211> 81
   <212> DNA
   <213> Homo sapiens
<400> 500
   atgctgtttg aagggtctct gttttctctc ttaatttggg tcattactta gaaagcaagc 60
   gtgagatcat ttcatcagcg t 81
<210> 501
   <211> 87
   <212> DNA
   <213> Homo sapiens
<400> 501
   gatagccttt gttaaaaagt ttaatgttct taattagttg aatcggttgg gctaattaga 60
   ataccaatta gaactggcag ggctatc 87
<210> 503
   <211> 77
   <212> DNA
   <213> Homo sapiens
<400> 503
   ccccattttc agaagatgcc atggtttatt aattctctgc agaagcaata agaactgggt 60
   attggcttaa tttgggg 77
<210> 512
   <211> 81
   <212> DNA
   <213> Homo sapiens
<400> 512
   gaccacttgt tcaaggttac tttacccacc ctcctttgtg atccagagcc aaaggatcca 60
   aatgggtaat tacctttggt c 81
<210> 517
   <211> 85
   <212> DNA
   <213> Homo sapiens
<400> 517
   ggagccagca gagggagctg tcgtcccaga actttcttag agctgctaag aaattctgat 60
   tttgaaaaag atcttcctag gctcc 85
<210> 520
   <211> 68
   <212> DNA
   <213> Homo sapiens
<400> 520
   gggcgctcca gtctcgacgt tcccggggta gagagcaacg tccggggacg aacgggacgg 60
   gtgcgccc 68
<210> 527
   <211> 87
   <212> DNA
   <213> Homo sapiens
<400> 527
   aatctgtagt cataagcagt gtgggtgaaa tacaaagtgt atatttagac tttagtgttg 60
   ctcatacata tgcattgttt ttagatt 87
<210> 528
   <211> 73
   <212> DNA
   <213> Homo sapiens
<400> 528
   ggattggccg agcaagcctg gaaaatggta aatgatcatt tggatcaatt acaggctttt 60
   agctggcttg tct 73
<210> 530
   <211> 90
   <212> DNA
   <213> Homo sapiens
<400> 530
   aggttgcata aatggtgaca aaatttacat ctttgtattc aagaagagta tagagttact 60
   tggtgaagat cactacatat gttaaatcct 90
<210> 531
   <211> 70
   <212> DNA
   <213> Homo sapiens
<400> 531
   gattcctgct cccagagcca taaagtggga gcccccattt attaattggg ctgggactgg 60
   ggcgggggtc 70
<210> 534
   <211> 73
   <212> DNA
   <213> Homo sapiens
<400> 534
   ggcgtttctt cttgtgtttc ctcttctcct tttctggaga gggatgaagg agaccctttg 60
   caagaggcat gtt 73
<210> 541
   <211> 74
   <212> DNA
   <213> Homo sapiens
<400> 541
   cagtttgtac tggggggtca aaggggagtt cttttccgaa gaattctggg agagacgcca 60
   actagagcaa gctg 74
<210> 542
   <211> 88
   <212> DNA
   <213> Homo sapiens
<400> 542
   ccaatgcctc tcacctcctc acttgtggca ccttcgcttt tgatccgaag tgcggggtta 60
   ttgtgagtga cggtgtggga ggagaggg 88
<210> 562
   <211> 80
   <212> DNA
   <213> Homo sapiens
<400> 562
   gtctttgctg atatagagga aggaagggga aaaatgagcg cattagttct cttttattaa 60
   aagagttatt tcagcatgac 80
<210> 563
   <211> 77
   <212> DNA
   <213> Homo sapiens
<400> 563
   gtctagcctt gttcatggta attatttcta tccattatcc atcatgctaa gaaatgacag 60
   ctcagggaca aggagac 77
<210> 579
   <211> 74
   <212> DNA
   <213> Homo sapiens
<400> 579
   cacaggttgt gtggtaataa aagacccccg aacaaaatgt tccaggggct ttggttttgt 60
   gacctactct tgtg 74
<210> 608
   <211> 88
   <212> DNA
   <213> Homo sapiens
<400> 608
   ggccctgaga gcaatactca tattgattgc atttatttca ctctagggag gagagataat 60
   tcttcaatgt gggacacatc tgggagcc 88
<210> 633
   <211> 86
   <212> DNA
   <213> Homo sapiens
<400> 633
   aagatagagg tcatttctat gcagagaaaa acaaatgagc tgtgtcattg cagaaatgtt 60
   ctgcatggtt tgctgccatc tgtctt 86
<210> 638
   <211> 83
   <212> DNA
   <213> Homo sapiens
<400> 638
   gcctcccctt ccaggtcatt acctgcggcc gcggaggcaa cagctgccac catggcctga 60
   tgagtgatct ggtgggcgac ggc 83
<210> 640
   <211> 85
   <212> DNA
   <213> Homo sapiens
<400> 640
   agttgctata acaactttga tatttcttta ttatttttag cacttcagtt gccataacaa 60
   taagatatcc taaaaaatag caatt 85
<210> 651
   <211> 83
   <212> DNA
   <213> Homo sapiens
<400> 651
   ttccagccag gccacctctc ctttatctgc agtgtctatt ttaagactgc ttcactgcaa 60
   ggagtatggg gcccgggcag gaa 83
<210> 653
   <211> 74
   <212> DNA
   <213> Homo sapiens
<400> 653
   ccccaccaac caccagtgct caggacttct gcaaatccca ttcggatctg ggagcagctg 60
   atgagggggt gggg 74
<210> 664
   <211> 74
   <212> DNA
   <213> Homo sapiens
<400> 664
   ctcccacggc ctgaagctgc tgccaagcta tttttggttc tgcacagtta aaaatagctt 60
   cacggaggtg ggag 74
<210> 669
   <211> 79
   <212> DNA
   <213> Homo sapiens
<400> 669
   caggcacgga aagctgaagg tgctggagat gccacatgaa gtgtgtgcat ttttggtaac 60
   tgtgtgcttc tgatgactg 79
<210> 671
   <211> 86
   <212> DNA
   <213> Homo sapiens
<400> 671
   tcccccaacc catccattag gccagcaacg cttgtagagc tcactgtggg ctgtaatgtg 60
   gcactggtgg gctgggacac caggga 86
<210> 678
   <211> 69
   <212> DNA
   <213> Homo sapiens
<400> 678
   ggctttgccc ttttcggtga cacaggctgt tgctattcca agcagcctat cacaggcagg 60
   gggagggcc 69
<210> 712
   <211> 89
   <212> DNA
   <213> Homo sapiens
<400> 712
   gccatttgcc ccaacttagg tttagaaatt acaagtttca gtaatagctg aacctgttca 60
   aaatgttaat aaaggtttcg ttgaatggt 89
<210> 716
   <211> 79
   <212> DNA
   <213> Homo sapiens
<400> 716
   gttggcggca cggtaggttc attagtcaat aaagcattgc cacgcgtttt attgaattgt 60
   aaccctattt tgccctgat 79
<210> 728
   <211> 75
   <212> DNA
   <213> Homo sapiens
<400> 728
   ctctctacca ccaaaataaa ttcaattact aactttgagt aattgcgggt aatttatttc 60
   ttggagggca gagag 75
<210> 738
   <211> 87
   <212> DNA
   <213> Homo sapiens
<400> 738
   ggccacttgg gtgcattgga cttgaacttc ttttttgtct cccctttagg ggggatatag 60
   attttcattt ctctttcata acgggcc 87
<210> 744
   <211> 78
   <212> DNA
   <213> Homo sapiens
<400> 744
   ggcttaatag tgcataaaat gtcaccactt catttaacaa aatgggttct gaagttatgt 60
   aatcatgcat tatgagtc 78
<210> 753
   <211> 76
   <212> DNA
   <213> Homo sapiens
<400> 753
   cagcttttat tgaaaatagt tactgcagcc aacgttggaa aagtttactg cagcttttga 60
   cttcttcaaa gagctg 76
<210> 770
   <211> 75
   <212> DNA
   <213> Homo sapiens
<400> 770
   ctatgcgtct tgacggattt ggggttggca gagcaggctg cccctgcttt ctatccccat 60
   tcagtccact tatag 75
<210> 784
   <211> 80
   <212> DNA
   <213> Homo sapiens
<400> 784
   cgactttatc acccatcggt tatctgtgtc gcctgaagga actccgggta aggtacagga 60
   aaaaggtggg ggtattgttg 80
<210> 785
   <211> 80
   <212> DNA
   <213> Homo sapiens
<400> 785
   tttctgtata tgtttctgga gtcctgagcc tgagctaaac aaaagcagga ggctgacggg 60
   gctgctggag tttgcagaga 80
<210> 786
   <211> 78
   <212> DNA
   <213> Homo sapiens
<900> 786
<210> 826
   <211> 83
   <212> DNA
   <213> Homo sapiens
<400> 826
   agtgcagatg caaataatcc taacaaatta ttctataaat ataccacaaa tagcaatact 60
   ttgtgaggtt aatagctctt act 83
<210> 827
   <211> 83
   <212> DNA
   <213> Homo sapiens
<400> 827
   gtgctgggtg ggtggttttt tatcttcacg gatttatgga gtccttaaaa catctgttcc 60
   gttctgattc ccccgctcag tac 83
<210> 832
   <211> 80
   <212> DNA
   <213> Homo sapiens
<400> 832
   catgtgattt ctgcccagtg ctctgaatgt caaagtgaag aaattcaatg aagcacgggt 60
   aaacggcggg agtaactatg 80
<210> 837
   <211> 77
   <212> DNA
   <213> Homo sapiens
<400> 837
   ggaaatggga gaaggataaa gtggaaatct aattttgaga aataaggatt aaaggttcca 60
   ttattcatgc tgttttc 77
<210> 838
   <211> 74
   <212> DNA
   <213> Home sapiens
<400> 838
   tctgttttta tcagtttaat atatgataca tcttctatcc aaggacaata tattaaatgg 60
   atttttggag caga 74
<210> 852
   <211> 80
   <212> DNA
   <213> Homo sapiens
<400> 852
   tgtgtcatcc tgtgtgctga agctgctttt taaaggggcc aagcctcctt taaacaattc 60
   taaatatttt tgaagataca 80
<210> 858
   <211> 84
   <212> DNA
   <213> Homo sapiens
<400> 858
   gcctgctaat ggttctactg cttttgaagg ttccaggtct gtgcagcaca gtgtgggacc 60
   ccaggagtgt ggatctgtgg aggc 84
<210> 874
   <211> 77
   <212> DNA
   <213> Homo sapiens
<400> 874
   gttccgaggc aggctttcct cctctctgca ggggagaggc tccctcacac aagaggagga 60
   ttacactggc tctgagc 77
<210> 876
   <211> 78
   <212> DNA
   <213> Homo sapiens
<400> 876
   aaaatagatt tttcacctgt caggcagtta tttttacaaa tgtatgatat gggtactgat 60
   tagtgaaaat gctctttt 78
<210> 878
   <211> 86
   <212> DNA
   <213> Homo sapiens
<400> 878
   gttccagaag gcggcgcgtg cggttgggaa cgcggagcgg acggattcga ttcaacgggg 60
   ttccggaccg cgctgcgcta tggagc 86
<210> 903
   <211> 87
   <212> DNA
   <213> Homo sapiens
<400> 903
   gctggattct gcccttggat acacacaaca aaacccccat tgaagtcaat ggaaattgtg 60
   catgcatatc caggggcaga atttggc 87
<210> 906
   <211> 78
   <212> DNA
   <213> Homo sapiens
<400> 906
   actgcttagc actcctccac ttgcgaatgc acttaagaca gtaggtgtgg ttgcagttgg 60
   agaggatccc gaagcggt 78
<210> 909
   <211> 75
   <212> DNA
   <213> Homo sapiens
<400> 909
   tagtgctaaa ctcatttgca caactacagt taattggcta caacaattaa ttaatgtgtt 60
   ggaaatttgg cactg 75
<210> 911
   <211> 80
   <212> DNA
   <213> Homo sapiens
<400> 911
   ggtatttgaa gatgcggttg accatggtgt gtacgcttta tttgtgacgt aggacacatg 60
   gtctacttct tctcaatatc 80
<210> 926
   <211> 84
   <212> DNA
   <213> Homo sapiens
<400> 926
   gcttggtgag aggaggagga ggcagggccg accgccaccc gcctgtctgc catctggtcc 60
   ccttcccctc cctcctctca ttgc 84
<210> 927
   <211> 70
   <212> DNA
   <213> Homo sapiens
<400> 927
   tctctaaaag gtttctaaat gatgttgatg ttaaaaaata tagtatcacc atttaacagc 60
   cttttaaaga 70
<210> 940
   <211> 73
   <212> DNA
   <213> Homo sapiens
<400> 940
   ctcagtatct tcagcttggg aaactgacct cgttaatttt aatgagggga aaaattctcc 60
   agctggggct gag 73
<210> 942
   <211> 76
   <212> DNA
   <213> Homo sapiens
<400> 942
   gtactctgta attactgtac attgtaacaa atgacctcat aaatacattg taatgtccca 60
   tgtaattaca tggtac 76
<210> 944
   <211> 71
   <212> DNA
   <213> Homo sapiens
<400> 944
   gcacattata aactctaatt cattaacgtc atcataaatg gtaatgtcct gtgaaaaaga 60
   gaggtggtgg c 71
<210> 946
   <211> 85
   <212> DNA
   <213> Homo sapiens
<400> 946
   aatacataaa tgaacgggta ttggtgcctc tttattttaa aaaatttgaa gaaaagagcc 60
   acctcatatt catagggtgt gtatt 85
<210> 947
   <211> 81
   <212> DNA
   <213> Homo sapiens
<400> 947
   tgtgctctca ttgttattcc aaaagtctct gtctagatca ctggaggggc agagagagag 60
   gggagaaaac agggagatac a 81
<210> 949
   <211> 78
   <212> DNA
   <213> Homo sapiens
<400> 949
   gttctagttc caggatgctg atactttaag cccgaggctc taacttgagc aggaagagtt 60
   tattttggga tgaagaat 78
<210> 951
   <211> 72
   <212> DNA
   <213> Homo sapiens
<400> 951
   acttagagga gtgacaaatt tggtacttaa aaaatattta ggttaaaaac aaccctgtca 60
   cttctactaa gt 72
<210> 955
   <211> 86
   <212> DNA
   <213> Homo sapiens
<400> 955
   caggctgaca actgatatca caagaccaca gctaagagtg gtttattact tttagtggga 60
   tttatttaat tcagtctcac agcctg 86
<210> 963
   <211> 87
   <212> DNA
   <213> Homo sapiens
<400> 963
   ccctcccagt tcccatagca actgggctgt agcagccaga acttgattga gcccagcagt 60
   ggcccgactg aggtggggaa aggaggg 87
<210> 967
   <211> 81
   <212> DNA
   <213> Homo sapiens
<400> 967
   agtgcaattt gtgtataaca ttttagtact taaatcaact atgaaaagca ttttaaatta 60
   aaatccaata cactttgcat t 81
<210> 975
   <211> 90
   <212> DNA
   <213> Homo sapiens
<400> 975
   ttagagagaa accaattctt tggtctattt tcttggtagc tttactgatt gccagggaaa 60
   atgaaaacca gaaaattaat taatctctaa 90
<210> 978
   <211> 74
   <212> DNA
   <213> Homo sapiens
<400> 978
   gccacctttc atggtgagga tgcctgccac cttcaggatc acatctttgg gtgaggtcca 60
   accagagagg gagc 74
<210> 1039
   <211> 70
   <212> DNA
   <213> Homo sapiens
<400> 1039
   gggaatctat tctccccatt tccaaaccat gatctgaatg ggcactgaca agtggagaga 60
   atggatatct 70
<210> 1045
   <211> 86
   <212> DNA
   <213> Homo sapiens
<400> 1045
   tcatggcagc gacccacctc cagtcccctg gacaatcggg tacaagagac ttaaggttgg 60
   gcatgggaag ggtggggttt ccatga 86
<210> 1055
   <211> 69
   <212> DNA
   <213> Homo sapiens
<400> 1055
   atgcactctg ctgtcatttg cagcctgacc tgcaggtcag gggtgatgtg agctggctca 60
   gggatgcat 69
<210> 1060
   <211> 75
   <212> DNA
   <213> Homo sapiens
<400> 1060
   gatgtcttgc aaaaattgct ggaatcttta caggagaacc acttccaaga agatgagcag 60
   tttctgggag ccgtt 75
<210> 1065
   <211> 84
   <212> DNA
   <213> Homo sapiens
<400> 1065
   caggggcgcg ggccggagag cgggtgtgca aagtgggcgc agggccctgg ggccgcgccc 60
   cttgctctgc cggctcgact cttg 84
<210> 1083
   <211> 88
   <212> DNA
   <213> Homo sapiens
<400> 1083
   ggagatgtgc gccccctctg ccacgccccc accttcctgg caggtggtgg gccaccaggg 60
   tggtgggaag tggccggggt ttctctcc 88
<210> 1100
   <211> 90
   <212> DNA
   <213> Homo sapiens
<400> 1100
   gcaattagaa tgcagggagg ttcagaagct atttaactgg gtgacccctg aggtcgctgc 60
   atctgactcc catccctgga taaatattgt 90
<210> 1117
   <211> 90
   <212> DNA
   <213> Homo sapiens
<400> 1117
   gcctctgtgc gcatggatat aatcagcttt gataggcaga ggctgaggct gtttttccaa 60
   ttagagctgt tagaggattc tggcaggggc 90
<210> 1118
   <211> 85
   <212> DNA
   <213> Homo sapiens
<400> 1118
   gggggcgagg ctatgtcgcg gtggcagccc ggatgggccg gcagggccgg gagtaacggg 60
   acgtcgccgc ggagcttctt ccccc 85
<210> 1129
   <211> 89
   <212> DNA
   <213> Homo sapiens
<400> 1129
   gaagtgtttg ttaggcttcg catgctggtc tccatatgcc tcttttcaat tacatgtgcc 60
   gccacaagta tggctaaacg aaacgcttc 89
<210> 1136
   <211> 86
   <212> DNA
   <213> Homo sapiens
<400> 1136
   ctcaaacctg gcatggtggt cacctttgct ccagtcaatg ttacaactga agtgaagtct 60
   gttgaaatgc accatgaagc tttgag 86
<210> 1137
   <211> 70
   <212> DNA
   <213> Homo sapiens
<400> 1137
   tgaagaattt cttctggatg actgaccaag aggctattca agatctctgg cagtggagga 60
   agtctcttta 70
<210> 1145
   <211> 80
   <212> DNA
   <213> Homo sapiens
<400> 1145
   tggtaaataa tttacatagt tttgcattag ctcaatttgc aaaaattgag caaacagtgc 60
   aattttctgt aattatgcca 80
<210> 1160
   <211> 72
   <212> DNA
   <213> Homo sapiens
<400> 1160
   gcagttgtgc aacatgaaaa ctgcagtgac atgttaccat ttgactgtct ccgtagttcg 60
   tgatgcatct gt 72
<210> 1161
   <211> 87
   <212> DNA
   <213> Homo sapiens
<400> 1161
   ggactctacc attcttgatt tagttttgag agaaaagggt ttgcaaaaac tataataatc 60
   tcacagctgg agatggctgg aaagtcc 87
<210> 1162
   <211> 76
   <212> DNA
   <213> Homo sapiens
<400> 1162
   gatctcggac acatctgtgg tgctgtccat ggacaacagc cgctccctgg acatggacag 60
   catcattgct gaggtc 76
<210> 1166
   <211> 74
   <212> DNA
   <213> Homo sapiens
<400> 1166
   cgcagaaacc ttctggaggt aaatctgcag aatggcattt ttgtgagttc tctgatagac 60
   ctcgaggagc tgtg 74
<210> 1173
   <211> 76
   <212> DNA
   <213> Homo sapiens
<400> 1173
   cttaggggtg ggggagccct gttagccctg taaataaagt ttaacgaggt gaacaatggc 60
   tggctctgtc cctgag 76
<210> 1174
   <211> 81
   <212> DNA
   <213> Homo sapiens
<400> 1174
   atcacctttc cccctcccat gtgctttcct tcatttgaga tcttttgacc tttggcttta 60
   tttgggaggg ggaagggtga t 81
<210> 1175
   <211> 85
   <212> DNA
   <213> Homo sapiens
<400> 1175
   gataataccg gcacctcttt tcactctaat ctcctacccg tctgagcaat tcagcaggag 60
   ataggggcat taaggccgta ttatt 85
<210> 1198
   <211> 82
<212> DNA
<213> Homo sapiens
<400> 1198
   ggggcgggga ggaattccgg ttctctggga ctttccaaaa aaggcgaaga tccggtgccg 60
   gcggctccgc ctccctagcc ct 82
<210> 1201
   <211> 86
   <212> DNA
   <213> Homo sapiens
<400> 1201
   catttcacat ccatgaagta ggaattgggg ctctgcacca ggcgtttctt cttgtgtttc 60
   ctcttctcct cttctggaga gggatg 86
<210> 1205
   <211> 88
   <212> DNA
   <213> Homo sapiens
<400> 1205
   agtgcttttc tgtaacagtc atttgacatt taatatacca taattgtatt attaaattta 60
   aatgcatatt tgcaattcta aaagcact 88
<210> 1207
   <211> 79
   <212> DNA
   <213> Homo sapiens
<400> 1207
   tatgcaggtg taggataggt ttatgtgaaa cttctggaat cactgcagga gtggtataga 60
   caatgaaaaa ccctgcatg 79
<210> 1212
   <211> 88
   <212> DNA
   <213> Homo sapiens
<400> 1212
   tgctatctcg cctcacacat caacacacgt gccagacaga ttctgactgc aaagtctgtt 60
   attggtgatg agagaggcag agagggca 88
<210> 1217
   <211> 79
   <212> DNA
   <213> Homo sapiens
<400> 1217
   agcctgttcc gtgctcgcta actataaact atctgattta tattcattaa ccagtactag 60
   acagcggcag gcacaggct 79
<210> 1233
   <211> 74
   <212> DNA
   <213> Homo sapiens
<400> 1233
   gagattagcg aaagggattc tggccaaatc cctgatcaag ttgcttggca cagaattatt 60
   gattggcaaa tctt 74
<210> 1238
   <211> 89
   <212> DNA
   <213> Homo sapiens
<400> 1238
   catcccatgg tgtgccccca aacatcttat ttgtcttgat atttctttca cagaaataca 60
   tatgacagat aaggagtcac tagaggatg 89
<210> 1251
   <211> 79
   <212> DNA
   <213> Homo sapiens
<400> 1251
   gcaggtgaca tcggagaaac atgagtgaat gtgatttcga acattctgaa tttcttcatg 60
   ttgatgttcc ttcacctgc 79
<210> 1267
   <211> 71
   <212> DNA
   <213> Homo sapiens
<400> 1267
   atttaatgta atttccctaa aatggaggta ataggtactc agggaataca cctccagggt 60
   ttacatcaaa t 71
<210> 1282
   <211> 84
   <212> DNA
   <213> Homo sapiens
<400> 1282
   gcttaaaaaa caatgaagtt aatttttaaa aacctttcat tgagttccat gagaccccta 60
   ttaattttat tgtttcaata aagc 84
<210> 1299
   <211> 76
   <212> DNA
   <213> Homo sapiens
<400> 1299
   gtcatgatgt tgcgtcacca cgtgatgacg ctgtgccatc acaacacatc atcgcatatt 60
   gacacaatgt catggc 76
<210> 1321
   <211> 73
   <212> DNA
   <213> Homo sapiens
<400> 1321
   cattgtctga attactgaag ctcaactcat ctgataaaat aggtgagctt tgtggtaatg 60
   aagtcagata atg 73
<210> 1323
   <211> 81
   <212> DNA
   <213> Homo sapiens
<400> 1323
   ccgcgaggac cttctggagg taaatctgca gaatggcatt ttgtttgtga attctcggat 60
   cgaccgcgag gagctgtgcg g 81
<210> 1341
   <211> 75
   <212> DNA
   <213> Homo sapiens
<400> 1341
   gatgccgggc gcccgccgca gccgctgccg ccggagcccg ggatggggcg agaggctgcg 60
   gcggacgcca gcatc 75
<210> 1343
   <211> 81
   <212> DNA
   <213> Homo sapiens
<400> 1343
   ttacaggcaa tccagtcact atggcaggaa aagtaattac caaactgcca cttcctgcaa 60
   acagcaagat tgtcgctgta a 81
<210> 1344
   <211> 86
   <212> DNA
   <213> Homo sapiens
<400> 1344
   gccacattct gaaagtgatc tttttgaaac gaggattgca aatagctgca gcttctcaaa 60
   actgcaattg cttttcatgg tgtggc 86
<210> 1346
   <211> 73
   <212> DNA
   <213> Homo sapiens
<400> 1346
   gtcaggctgc tgtattctct tacacagatg ccagtaagaa caaaggcatc acgtggggag 60
   aggataccct gat 73
<210> 1352
   <211> 85
   <212> DNA
   <213> Homo sapiens
<400> 1352
   gctatgctca gctgccaccc tgaaatttat ggatgccggt ggtgacctgg caacagtaaa 60
   tttcaaggct agttaagagg atggt 85
<210> 1370
   <211> 75
   <212> DNA
   <213> Homo sapiens
<400> 1370
   cggtggaggg aaaggggaaa ggagccattt tctgctgcac atcagtcagt gcctgcgccc 60
   tccctccctc cgccg 75
<210> 1371
   <211> 80
   <212> DNA
   <213> Homo sapiens
<400> 1371
   aggaggccct gagtgctagt agccacttat agaaagtcgc ttccagcaat tcttctagga 60
   gtggagcctc agggctgcct 80
<210> 1396
   <211> 80
   <212> DNA
   <213> Homo sapiens
<400> 1396
   tggcaggaag atggcactgg ccaggttgcc ggtgacacca ctcagcaggt agatgatggc 60
   tatgcggtgc cagcctgcca 80

<210> 1403
   <211> 76
   <212> DNA
   <213> Homo sapiens
<400> 1403
   ggtaattcaa aacaaatttc aagctgtgac aataaaatgg ttcttgtagt aggttggggt 60
   ttgtgctgaa attgcc 76
<210> 1425
   <211> 80
   <212> DNA
   <213> Homo sapiens
<400> 1425
   agagcgcagc tactggtgct ttgcaggagg caagtgaggc ctatctggtt ggcctttttg 60
   aagacaccaa cctgtgcgct 80
<210> 1429
   <211> 90
   <212> DNA
   <213> Homo sapiens
<400> 1429
   gtgaagaatg caggcaggtt gtgattagag gtgagccagt tcctgattgt gggttaataa 60
   gctctgctaa taaactgcct gtagctttac 90
<210> 1433
   <211> 75
   <212> DNA
   <213> Homo sapiens
<400> 1433
   taaggcagga tgattcggga aatggactat ttaaagcaag ctgctttgga cttgaatacc 60
   cccatcctgc gctta 75
<210> 1485
   <211> 86
   <212> DNA
   <213> Homo sapiens
<400> 1485
   gtctgcctct ttctctgcag taattgcttc ctgacatttg tttattttaa ttaggagagc 60
   agtcttgatc aagagggagg gcagac 86
<210> 1531
   <211> 74
   <212> DNA
   <213> Homo sapiens
<400> 1531
   agcctaaata cattagcgag ctggtaaagc ttttaaggcc ttcttgggag cgagtggctg 60
   gctaatgaga ggtt 74
<210> 1545
   <211> 81
   <212> DNA
   <213> Homo sapiens
<400> 1545
   tcagaatgca gggcctggcc tacaatactg gagttaagga aattcttaac ttcttccaag 60
   gttaccaggt cagtagcttg a 81
<210> 1547
   <211> 72
   <212> DNA
   <213> Homo sapiens
<400> 1547
   tctttgttaa aatgtaaaat gcatattggg caaatgctcc agggcaattt gcataaaaag 60
   tgatgacaaa ga 72
<210> 1559
   <211> 75
   <212> DNA
   <213> Homo sapiens
<400> 1559
   tctccatcct ctgtctccct tgatcctctg ttctccctga tggctttgag atgaaggcga 60
   cggcaaggat ggagg 75
<210> 1586
   <211> 80
   <212> DNA
   <213> Homo sapiens
<400> 1586
   ggaaaatgga cgtgtgtctc atggtagccg cagtttaagt aaacataaac tctgaaccag 60
   agatcatacc cgccatttcc 80
<210> 1592
   <211> 80
   <212> DNA
   <213> Homo sapiens
<400> 1592
   ggcgcccgcc gggctgtccg gagcggccga tggggcccgt gtgagcgcgc ccaggcccgg 60
   cccggtgccc ggcgggcggc 80
<210> 1601
   <211> 84
   <212> DNA
   <213> Homo sapiens
<400> 1601
   ttccagctat ttagtaactc ttccaaaaca ctgtcagcac ccatgctagg atgcagggag 60
   tgggaaggaa gtctaagtag ggaa 84
<210> 1610
   <211> 87
   <212> DNA
   <213> Homo sapiens
<400> 1610
   gctggaagtc agcctctttc aaagctgtcc actgggatgt acccagtgca ggagctaaat 60
   atagaaaggg ctccctcctc ccccagc 87
<210> 1611
   <211> 69
   <212> DNA
   <213> Homo sapiens
<400> 1611
   gcatcattag ctaacacagg gaaagtgaaa tgctgttaac ttttcttttt cttctgcaag 60
   ctgatgagc 69
<210> 1617
   <211> 77
   <212> DNA
   <213> Homo sapiens
<400> 1617
   ccttttgagt ggacctgaag ggttttgacc tccttcagga aaggcaaggc aaaaacctta 60
   aaacagttcc actgagg 77
<210> 1620
   <211> 85
   <212> DNA
   <213> Homo sapiens
<400> 1620
   gtcctttaag acagtggttc taaaaatgtg agccgaggat cttttggctt gcagcattaa 60
   tctccacagt atgtacttta agggc 85
<210> 1634
   <211> 89
   <212> DNA
   <213> Homo sapiens
<400> 1634
   tacttttagt ataatggatt tttaagggaa aatattccat gaaagcattg agtattgaat 60
   attttatttg aaagcaaatg ctaaaagta 89
<210> 1645
   <211> 88
   <212> DNA
   <213> Homo sapiens
<400> 1645
   gtgctgacaa acaagtgaat cactttatta atgtgctata aaaggataaa tgaggcttga 60
   gaaactttgt ttgccctaga ctcagcac 88
<210> 1648
   <211> 84
   <212> DNA
   <213> Homo sapiens
<400> 1648
   gagctgcttt gaattgctcg cagtttgccg gaggcggtgt gctgggttgg acgctccggg 60
   aaacaaagca acccaaaaca gctc 84
<210> 1649
   <211> 86
   <212> DNA
   <213> Homo sapiens
<400> 1649
   catttgcttg attaattact gctctgaaac ccttgaaaac agacatggtc tgtaagtggt 60
   atctgttgtt taatcgagga cagatg 86
<210> 1653
   <211> 87
   <212> DNA
   <213> Homo sapiens
<400> 1653
   ttattccagg gagactctgt aatagatacc agcattttag taatgataca agcaatctgt      60
   aagctattac agttttcttt agagtaa                                          87
<210> 1656
   <211> 87
   <212> DNA
   <213> Homo sapiens
<400> 1656
   gcttgtccta aaagatcttc cttctgtttc cctgggttta tccacttggt tggcctgatg      60
   ggagcaggag gcggtgaggg ggcgggc                                          87
<210> 1661
   <211> 85
   <212> DNA
   <213> Homo sapiens
<400> 1661
   gccttgctgt atttcttata ccatttatta aaaagctgct ttcacggtaa aattatgttg      60
   gtttgaaagg aggaaatagc aaggt                                            85
<210> 1667
   <211> 88
   <212> DNA
   <213> Homo sapiens
<400> 1667
   ctggcccatt ttcattctgc ataaaatttt aatggtctct ctggctgatc cgggacggca      60
   gcgcgcggag aggctcttaa agggccag                                         88
<210> 1681
   <211> 81
   <212> DNA
   <213> Homo sapiens
<400> 1681
   atagatcctg ttatgtggaa agagtcaatt aattcacact ttgatctaat aacataggct      60
   ccatccttgg acaggattta t                                                81

<210> 1686
   <211> 82
   <212> DNA
   <213> Homo sapiens
<400> 1686
   tgactgtggt aaatggtctt taaggtaagg tgctgcaaca agtaccttta aataaaatca 60
   aataaaaagt ttaccacaat ca 82
<210> 1688
   <211> 78
   <212> DNA
   <213> Homo sapiens
<400> 1688
   gagctgcaca gacagctgca attcatgggc gacgctgatt gccagaaacg ctatggattg 60
   gcatggggtc tgcagctc 78
<210> 1693
   <211> 75
   <212> DNA
   <213> Homo sapiens
<400> 1693
   caggcattgc ctggctcaga gacctttgct gctgagcaaa ctgagttaac aaagtgggct 60
   gaggggtgaa gcctg 75
<210> 1697
   <211> 33
   <212> DNA
   <213> Homo sapiens
<220>
   <223> mature of SEQ ID 3
<400> 1697
   ggggaaagtt tggagccggc gaggggcgcc ggg 33
<210> 1698
   <211> 31
   <212> DNA
   <213> Homo sapiens
<220>
   <223> mature of SEQ ID 3
<400> 1698
   ggagttgggg aaagtttgga gccggcgagg g 31
<210> 1700
   <211> 27
   <212> DNA
   <213> Homo sapiens
<220>
   <223> mature of SEQ ID 5
<400> 1700
   gcacctcctc caggggatga atctcat 27
<210> 1705
   <211> 19
   <212> DNA
   <213> Homo sapiens
<220>
   <223> mature of SEQ ID 8
<400> 1705
   ggcggaaggc agctgagtt 19
<210> 1719
   <211> 24
   <212> DNA
   <213> Homo sapiens
<220>
   <223> mature of SEQ ID 16
<400> 1719
   cctcaaatct gcaattcatg tatg 24
<210> 1720
   <211> 35
   <212> DNA
   <213> Homo sapiens
<220>
   <223> mature of SEQ ID 16
<400> 1720
   caaggatgac atagatttga caaaaaatta atgat 35
<210> 1721
   <211> 33
   <212> DNA
   <213> Homo sapiens
<220>
   <223> mature of SEQ ID 16
<400> 1721
   ggcatacaca aggatgacat agatttgaca aaa 33
<210> 1730
   <211> 31
   <212> DNA
   <213> Homo sapiens
<220>
   <223> mature of SEQ ID 22
<400> 1730
   agaaaaacct tgggttagac ctacaggggg t 31
<210> 1731
   <211> 34
   <212> DNA
   <213> Homo sapiens
<220>
   <223> mature of SEQ ID 22
<400> 1731
   accttgggtt agacctacag ggggtctggc tggt 34
<210> 1732
   <211> 28
   <212> DNA
   <213> Homo sapiens
<220>
   <223> mature of SEQ ID 22
<400> 1732
   tgtttccagc attcccaggt aggccaag 28
<210> 1737
   <211> 19
   <212> DNA
   <213> Homo sapiens
<220>
   <223> mature of SEQ ID 27
<400> 1737
   ggtctcctgc tctgctgat 19
<210> 1744
   <211> 31
   <212> DNA
   <213> Homo sapiens
<220>
   <223> mature of SEQ ID 32
<400> 1744
   gcaggagcct cctctggctt gagccctttc t 31
<210> 1745
   <211> 33
   <212> DNA
   <213> Homo sapiens
<220>
   <223> mature of SEQ ID 32
<400> 1745
   gcctcctctg gcttgagccc tttcttggta agt 33
<210> 1746
   <211> 31
   <212> DNA
   <213> Homo sapiens
<220>
   <223> mature of SEQ ID 34
<400> 1746
   caaaagtggc tgttcatttt gttatctgac c 31
<210> 1747
   <211> 19
   <212> DNA
   <213> Homo sapiens
<220>
   <223> mature of SEQ ID 35
<400> 1747
   atgggtcaga aggactcct 19
<210> 1748
   <211> 20
   <212> DNA
   <213> Homo sapiens
<220>
   <223> mature of SEQ ID 35
<400> 1748
   gcccagagca agagaggcat 20
<210> 1749
   <211> 22
   <212> DNA
   <213> Homo sapiens
<220>
   <223> mature of SEQ ID 36
<400> 1749
   tctgacactt gtctaaatcc tg 22
<210> 1750
   <211> 20
   <212> DNA
   <213> Homo sapiens
<220>
   <223> mature of SEQ ID 36
<400> 1750
   aagggcaaag ggcacagatc 20
<210> 1784
   <211> 29
   <212> DNA
   <213> Homo sapiens
<220>
   <223> mature of SEQ ID 60
<400> 1784
   tgagacctgg aagccacagc actggtgga 29
<210> 1785
   <211> 31
   <212> DNA
   <213> Homo sapiens
<220>
   <223> mature of SEQ ID 60
<400> 1785
   tctactgcct gctgctccag atccaagggg a 31

<210> 1804
   <211> 25
   <212> DNA
   <213> Homo sapiens
<220>
   <223> mature of SEQ ID 69
<400> 1804
   cagagtcaat tactcactgt gtgaa 25
<210> 1805
   <211> 33
   <212> DNA
   <213> Homo sapiens
<220>
   <223> mature of SEQ ID 70
<400> 1805
   gaacatctat cttccatgac ctcttgatta .ctg 33
<210> 1806
   <211> 31
   <212> DNA
   <213> Homo sapiens
<220>
   <223> mature of SEQ ID 70
<400> 1806
   agtgaagaac atctatcttc catgacctct t 31
<210> 1807
   <211> 20
   <212> DNA
   <213> Homo sapiens
<220>
   <223> mature of SEQ ID 70
<400> 1807
   gccagagtgg tctggacaca 20
<210> 1815
   <211> 33
   <212> DNA
   <213> Homo sapiens
<220>
   <223> mature of SEQ ID 77
<400> 1815
   tttgttgaca aaacagtgaa atgtctagac cat 33
<210> 1816
   <211> 30
   <212> DNA
   <213> Homo sapiens
<220>
   <223> mature of SEQ ID 77
<400> 1816
   agggctttgt tgacaaaaca gtgaaatgtc 30
<210> 1817
   <211> 31
   <212> DNA
   <213> Homo sapiens
<220>
   <223> mature of SEQ ID 77
<400> 1817
   cagagatatt gacatagtga gaaaacattg c 31
<210> 1818
   <211> 29
   <212> DNA
   <213> Homo sapiens
<220>
   <223> mature of SEQ ID 77
<400> 1818
   atggcagaga tattgacata gtgagaaaa 29
<210> 1824
   <211> 33
   <212> DNA
   <213> Homo sapiens
<220>
   <223> mature of SEQ ID 81
<400> 1824
   acataaagag gcagggatgc tgattttaga cac 33
<210> 1825
   <211> 30
   <212> DNA
   <213> Homo sapiens
<220>
   <223> mature of SEQ ID 81
<400> 1825
   ggagtacata aagaggcagg gatgctgatt 30
<210> 1826
   <211> 31
   <212> DNA
   <213> Homo sapiens
<220>
   <223> mature of SEQ ID 81
<400> 1826
   attgtgagtg tcccttgcca tatttctgct a 31
<210> 1827
   <211> 34
   <212> DNA
   <213> Homo sapiens
<220>
   <223> mature of SEQ ID 81
<400> 1827
   agtgtccctt gccatatttc tgctaatgtg tttc 34
<210> 1828
   <211> 31
   <212> DNA
   <213> Homo sapiens
<220>
   <223> mature of SEQ ID 82
<400> 1828
   ttgacaatca aatggagctt aaagggaatt a 31
<210> 1837
   <211> 32
   <212> DNA
   <213> Homo sapiens
<220>
   <223> mature of SEQ ID 90
<400> 1837
   tttaattaac ttcgagatgc tccggcggcg gg 32
<210> 1838
   <211> 29
   <212> DNA
   <213> Homo sapiens
<220>
   <223> mature of SEQ ID 90
<400> 1838
   ccgctttaat taacttcgag atgctccgg 29
<210> 1839
   <211> 30
   <212> DNA
   <213> Homo sapiens
<220>
   <223> mature of SEQ ID 90
<400> 1839
   cccacgcacg gagggtcgcc aggaaagtgg 30
<210> 1842
   <211> 29
   <212> DNA
   <213> Homo sapiens
<220>
   <223> mature of SEQ ID 93
<400> 1842
   aaacggatcg catggctcat ttatcttgc 29
<210> 1843
   <211> 32
   <212> DNA
   <213> Homo sapiens
<220>
   <223> mature of SEQ ID 93
<400> 1843
   ggatcgcatg gctcatttat cttgcagcca tt 32
<210> 1849
   <211> 19
   <212> DNA
   <213> Homo sapiens
<220>
   <223> mature of SEQ ID 97
<400> 1849
   caaatcacat gtgggtctt 19
<210> 1896
   <211> 22
   <212> DNA
   <213> Homo sapiens
<220>
   <223> mature of SEQ ID 139
<400> 1896 22
   cccactggag catggagcca gc 22
<210> 1897
   <211> 23
   <212> DNA
   <213> Homo sapiens
<220>
   <223> mature of SEQ ID 139
<400> 1897
   gctggctcct gctcagagtg aca 23
<210> 1898
   <211> 32
   <212> DNA
   <213> Homo sapiens
<220>
   <223> mature of SEQ ID 141
<400> 1898 32
   tgatcaggcg cttgatatga caggctgtgg ga 32
<210> 1899
   <211> 35
   <212> DNA
   <213> Homo sapiens
<220>
   <223> mature of SEQ ID 141
<400> 1899
   gcgcttgata tgacaggctg tgggagtcag gaccc 35

<210> 1900
   <211> 25
   <212> DNA
   <213> Homo sapiens
<220>
   <223> mature of SEQ ID 141
<400> 1900
   acagagtggg ccttgtctgt cacag 25
<210> 1911
   <211> 32
   <212> DNA
   <213> Homo sapiens
<220>
   <223> mature of SEQ ID 151
<400> 1911
   agcattttaa gtaggtgtta taaatctctg cc 32
<210> 1912
   <211> 30
   <212> DNA
   <213> Homo sapiens
<220>
   <223> mature of SEQ ID 151
<400> 1912
   agtgtagcat tttaagtagg tgttataaat 30
<210> 1913
   <211> 21
   <212> DNA
   <213> Homo sapiens
<220>
   <223> mature of SEQ ID 151
<400> 1913
   ggcacattca tagccctgct t 21
<210> 1914
   <211> 31
   <212> DNA
   <213> Homo sapiens
<220>
   <223> mature of SEQ ID 152
<400> 1914
   agagggtctc ctatgccttt gaggagggtg a 31
<210> 1915
   <211> 29
   <212> DNA
   <213> Homo sapiens
<220>
   <223> mature of SEQ ID 152
<400> 1915
   aaacagaggg tctcctatgc ctttgagga 29
<210> 1916
   <211> 32
   <212> DNA
   <213> Homo sapiens
<220>
   <223> mature of SEQ ID 152
<400> 1916
   attttccttt aattaaggct ctgtggaggt gt 32
<210> 1917
   <211> 29
   <212> DNA
   <213> Homo sapiens
<220>
   <223> mature of SEQ ID 152
<400> 1917
   ttccattttc ctttaattaa ggctctgtg 29
<210> 1925
   <211> 32
   <212> DNA
   <213> Homo sapiens
<220>
   <223> mature of SEQ ID 158
<400> 1925
   tgaactcgag ttggaagagg cgagtccggt ct 32
<210> 1926
   <211> 27
   <212> DNA
   <213> Homo sapiens
<220>
   <223> mature of SEQ ID 158
<400> 1926
   gacgactggc cccgcctctt cctctcg 27
<210> 1933
   <211> 32
   <212> DNA
   <213> Homo sapiens
<220>
   <223> mature of SEQ ID 163
<400> 1933
   gccgccttgc ccagcccctg agggccccag cc 32
<210> 1934
   <211> 24
   <212> DNA
   <213> Homo sapiens
<220>
   <223> mature of SEQ ID 163
<400> 1934
   tgtgagtggg tttggggtga ggct 24
<210> 1944
   <211> 34
   <212> DNA
   <213> Homo sapiens
<220>
   <223> mature of SEQ ID 170
<400> 1944
   ccattaaggc aatctggctg ccagatgaag tctt 34
<210> 1945
   <211> 30
   <212> DNA
   <213> Homo sapiens
<220>
   <223> mature of SEQ ID 170
<400> 1945
   tgtgtgtctg gctatgtcag gccagggtgg 30
<210> 1949
   <211> 29
   <212> DNA
   <213> Homo sapiens
<220>
   <223> mature of SEQ ID 175
<400> 1949
   cggggacctt ctggaggtaa atctgcaga 29
<210> 1950
   <211> 31
   <212> DNA
   <213> Homo sapiens
<220>
   <223> mature of SEQ ID 175
<400> 1950
   gaccttctgg aggtaaatct gcagaatggc a 31
<210> 1951
   <211> 21
   <212> DNA
   <213> Homo sapiens
<220>
   <223> mature of SEQ ID 1323
<400> 1951
   tgtgaattct cggatcgacc g 21
<210> 1952
   <211> 33
   <212> DNA
   <213> Homo sapiens
<220>
   <223> mature of SEQ ID 176
<400> 1952
   tactcaagat gatttaaatt gtgaaaatgt cag 33
<210> 1953
   <211> 30
   <212> DNA
   <213> Homo sapiens
<220>
   <223> mature of SEQ ID 176
<400> 1953
   tgcaggaaaa tcagtaacat tagtcatctt 30
<210> 1954
   <211> 32
   <212> DNA
   <213> Homo sapiens
<220>
   <223> mature of SEQ ID 176
<400> 1954
   gaaaatcagt aacattagtc atcttaaaag gg 32
<210> 1959
   <211> 33
   <212> DNA
   <213> Homo sapiens
<220>
   <223> mature of SEQ ID 183
<400> 1959
   tgatttgtaa cactttaaaa aacattttct agg 33
<210> 1960
   <211> 30
   <212> DNA
   <213> Homo sapiens
<220>
   <223> mature of SEQ ID 183
<400> 1960
   ttgattgatt tgtaacactt taaaaaacat 30
<210> 1961
   <211> 33
   <212> DNA
   <213> Homo sapiens
<220>
   <223> mature of SEQ ID 183
<400> 1961
   gagagtgttt tataatgtat tcagtttaat taa 33
<210> 1962
   <211> 30
   <212> DNA
   <213> Homo sapiens
<220>
   <223> mature of SEQ ID 183
<400> 1962
   attaggagag tgttttataa tgtattcagt 30
<210> 1963
   <211> 31
   <212> DNA
   <213> Homo sapiens
<220>
   <223> mature of SEQ ID 1321
<400> 1963
   gtgagctttg tggtaatgaa gtcagataat g 31
<210> 1964
   <211> 29
   <212> DNA
   <213> Homo sapiens
<220>
   <223> mature of SEQ ID 1321
<400> 1964
   ataggtgagc tttgtggtaa tgaagtcag 29
<210> 1968
   <211> 20
   <212> DNA
   <213> Homo sapiens
<220>
   <223> mature of SEQ ID 187
<400> 1968
   ggccggcggg gggcctctgg 20
<210> 1973
   <211> 29
   <212> DNA
   <213> Homo sapiens
<220>
   <223> mature of SEQ ID 189
<400> 1973
   gctggacagc aaacatggag agaatcatg 29
<210> 1975
   <211> 22
   <212> DNA
   <213> Homo sapiens
<220>
   <223> mature of SEQ ID 191
<400> 1975
   gtaattgctg gccagctgca gg 22
<210> 1976
   <211> 23
   <212> DNA
   <213> Homo sapiens
<220>
   <223> mature of SEQ ID 191
<400> 1976
   gccctgtgag ctgtgttcag ggc 23
<210> 1977
   <211> 31
   <212> DNA
   <213> Homo sapiens
<220>
   <223> mature of SEQ ID 192
<400> 1977
   tgtcacatga ctttttggtg gcctgattag c 31
<210> 1978
   <211> 29
   <212> DNA
   <213> Homo sapiens
<220>
   <223> mature of SEQ ID 192
<400> 1978
   cctttgtcac atgacttttt ggtggcctg 29
<210> 1979
   <211> 25
   <212> DNA
   <213> Homo sapiens
<220>
   <223> mature of SEQ ID 192
<400> 1979
   gttggaaggc acagttaaag ggtca 25
<210> 1990
   <211> 35
   <212> DNA
   <213> Homo sapiens
<220>
   <223> mature of SEQ ID 200
<400> 1990
   taaaattaga caaagcaata attagtttgg cacca 35
<210> 1991
   <211> 32
   <212> DNA
   <213> Homo sapiens
<220>
   <223> mature of SEQ ID 200
<400> 1991
   ctggatttaa aattagacaa agcaataatt ag 32
<210> 1992
   <211> 32
   <212> DNA
   <213> Homo sapiens
<220>
   <223> mature of SEQ ID 200
<400> 1992
   gctttgaatt aatgtgcaag ctgttaatct cc 32
<210> 1993
   <211> 29
   <212> DNA
   <213> Homo sapiens
<220>
   <223> mature of SEQ ID 200
<400> 1993
   tggtgctttg aattaatgtg caagctgtt 29
<210> 2000
   <211> 22
   <212> DNA
   <213> Homo sapiens
<220>
   <223> mature of SEQ ID 205
<400> 2000
   ttagatcttc atctcttatc ac 22
<210> 2001
   <211> 31
   <212> DNA
   <213> Homo sapiens
<220>
   <223> mature of SEQ ID 205
<400> 2001
   ataagggaag aagacaagaa aatgatattg t 31
<210> 2002
   <211> 29
   <212> DNA
   <213> Homo sapiens
<220>
   <223> mature of SEQ ID 205
<400> 2002
   gtgcataagg gaagaagaca agaaaatga 29
<210> 2005
   <211> 19
   <212> DNA
   <213> Homo sapiens
<220>
   <223> mature of SEQ ID 207
<400> 2005
   ttattaaagt gggagagag 19
<210> 2022
   <211> 28
   <212> DNA
   <213> Homo sapiens
<220>
   <223> mature of SEQ ID 221
<400> 2022
   ggagagggat gaaggagatc ctttgtga 28
<210> 2025
   <211> 32
   <212> DNA
   <213> Homo sapiens
<220>
   <223> mature of SEQ ID 223
<400> 2025
   ctttaagcct cattagattt cttgatagca aa 32
<210> 2026
   <211> 34
   <212> DNA
   <213> Homo sapiens
<220>
   <223> mature of SEQ ID 223
<400> 2026
   cctcattaga tttcttgata gcaaaacctc ccat 34
<210> 2027
   <211> 33
   <212> DNA
   <213> Homo sapiens
<220>
   <223> mature of SEQ ID 224
<400> 2027
   gtctgtcacc cttgattgga aagtaataat tca 33
<210> 2033
   <211> 29
   <212> DNA
   <213> Homo sapiens
<220>
   <223> mature of SEQ ID 229
<400> 2033
   ccctccccct gctgggatcc ccccagccc 29
<210> 2068
   <211> 33
   <212> DNA
   <213> Homo sapiens
<220>
   <223> mature of SEQ ID 248
<400> 2068
   cgtgaggagg agggactttt cgaagtttta tag 33
<210> 2072
   <211> 22
   <212> DNA
   <213> Homo sapiens
<220>
   <223> mature of SEQ ID 251
<400> 2072
   agcacccgga gctggcagac aa 22
<210> 2078
   <211> 21
   <212> DNA
   <213> Homo sapiens
<220>
   <223> mature of SEQ ID 256
<400> 2078
   aggggggctt tctctctcgc c 21
<210> 2082
   <211> 32
   <212> DNA
   <213> Homo sapiens
<220>
   <223> mature of SEQ ID 259
<400> 2082
   agataaatta cagtacatgc cagggaacag gc 32
<210> 2083
   <211> 29
   <212> DNA
   <213> Homo sapiens
<220>
   <223> mature of SEQ ID 259
<400> 2083
   gactagataa attacagtac atgccaggg 29
<210> 2086
   <211> 24
   <212> DNA
   <213> Homo sapiens
<220>
   <223> mature of SEQ ID 261
<400> 2086
   ggcccctccc ccaccattgt tctg 24
<210> 2108
   <211> 27
   <212> DNA
   <213> Homo sapiens
<220>
   <223> mature of SEQ ID 275
<400> 2108
   caaaattggc attttagcta atactgt 27
<210> 2111
   <211> 34
   <212> DNA
   <213> Homo sapiens
<220>
   <223> mature of SEQ ID 276
<400> 2111
   gcagtgtgat tgctgcttta gtggcaccag ggag 34
<210> 2112
   <211> 26
   <212> DNA
   <213> Homo sapiens
<220>
   <223> mature of SEQ ID 276
<400> 2112
   gcatctcttg ccaatcaaat cagtgc 26
<210> 2164
   <211> 34
   <212> DNA
   <213> Homo sapiens
<220>
   <223> mature of SEQ ID 316
<400> 2164
   gtgaaatcta tcaaggaagg agggttattt ttac 34
<210> 2173
   <211> 33
   <212> DNA
   <213> Homo sapiens
<220>
   <223> mature of SEQ ID 323
<400> 2173
   attttaattt tatcccagtt atggatatct ggc 33
<210> 2174
   <211> 31
   <212> DNA
   <213> Homo sapiens
<220>
   <223> mature of SEQ ID 323
<400> 2174
   gcacagattt taattttatc ccagttatgg a 31
<210> 2175
   <211> 26
   <212> DNA
   <213> Homo sapiens
<220>
   <223> mature of SEQ ID 323
<400> 2175
   tactctgggc agactggtgt gaaatt 26
<210> 2180
   <211> 31
   <212> DNA
   <213> Homo sapiens
<220>
   <223> mature of SEQ ID 328
<400> 2180
   ggtgcgcagg gaggtcggag gagcgggcac t 31
<210> 2181
   <211> 33
   <212> DNA
   <213> Homo sapiens
<220>
   <223> mature of SEQ ID 328
<400> 2181
   cagggaggtc ggaggagcgg gcactgccca ccc 33
<210> 2186
   <211> 19
   <212> DNA
   <213> Homo sapiens
<220>
   <223> mature of SEQ ID 333
<400> 2186
   agactggtta atgataaca 19
<210> 2196
   <211> 19
   <212> DNA
   <213> Homo sapiens
<220>
   <223> mature of SEQ ID 340
<400> 2196
   gcatgcttgg aaataggag 19
<210> 2201
   <211> 31
   <212> DNA
   <213> Homo sapiens
<220>
   <223> mature of SEQ ID 344
<400> 2201
   aaaaggtatg attgctcagg aatgaaaaca t 31
<210> 2202
   <211> 29
   <212> DNA
   <213> Homo sapiens
<220>
   <223> mature of SEQ ID 344
<400> 2202
   agataaaagg tatgattgct caggaatga 29
<210> 2203
   <211> 20
   <212> DNA
   <213> Homo sapiens
<220>
   <223> mature of SEQ ID 344
<400> 2203
   gtatgttttc atgtttatct 20
<210> 2219
   <211> 34
   <212> DNA
   <213> Homo sapiens
<220>
   <223> mature of SEQ ID 357
<400> 2219
   acttgttaat ttatttctct gtttgttaat gggc 34
<210> 2220
   <211> 30
   <212> DNA
   <213> Homo sapiens
<220>
   <223> mature of SEQ ID 357
<400> 2220
   gctctattat acagaacaga aggaacattt 30
<210> 2221
   <211> 32
   <212> DNA
   <213> Homo sapiens
<220>
   <223> mature of SEQ ID 357
<400> 2221
   attatacaga acagaaggaa catttggtgg gt 32
<210> 2228
   <211> 21
   <212> DNA
   <213> Homo sapiens
<220>
   <223> mature of SEQ ID 363
<400> 2228
   acagggaact ttttgatgag c 21
<210> 2229
   <211> 33
   <212> DNA
   <213> Homo sapiens
<220>
   <223> mature of SEQ ID 363
<400> 2229
   ggggttcatc ggagaaactc cctgcgatga gcc 33
<210> 2231
   <211> 31
   <212> DNA
   <213> Homo sapiens
<220>
   <223> mature of SEQ ID 366

<400> 2231
   gtacttggtt tttctaattt gacagaggat a 31
<210> 2232
   <211> 34
   <212> DNA
   <213> Homo sapiens
<220>
   <223> mature of SEQ ID 366
<400> 2232
   ggtttttcta atttgacaga ggataaaaga aggc 34
<210> 2233
   <211> 32
   <212> DNA
   <213> Homo sapiens
<220>
   <223> mature of SEQ ID 366
<400> 2233
   tgttttattt ctaaactcat ttagcaccaa gt 32
<210> 2234
   <211> 30
   <212> DNA
   <213> Homo sapiens
<220>
   <223> mature of SEQ ID 366
<400> 2234
   ggctttgttt tatttctaaa ctcatttagc 30
<210> 2235
   <211> 25
   <212> DNA
   <213> Homo sapiens
<220>
   <223> mature of SEQ ID 367
<400> 2235
   attgaaactg tatagatgat tgata 25
<210> 2236
   <211> 25
   <212> DNA
   <213> Homo sapiens
<220>
   <223> mature of SEQ ID 367
<400> 2236
   tatcaaattc tctataaatc tttca 25
<210> 2237
   <211> 28
   <212> DNA
   <213> Homo sapiens
<220>
   <223> mature of SEQ ID 368
<400> 2237
   gcgggtaaac ggcgggagta actatgac 28
<210> 2238
   <211> 34
   <212> DNA
   <213> Homo sapiens
<220>
   <223> mature of SEQ ID 368
<400> 2238
   gatgtgattt ctgcccagtg ctctgaatgt caaa 34
<210> 2259
   <211> 23
   <212> DNA
   <213> Homo sapiens
<220>
   <223> mature of SEQ ID 384
<400> 2259
   aatttccggg cagggggctg caa 23
<210> 2260
   <211> 27
   <212> DNA
   <213> Homo sapiens
<220>
   <223> mature of SEQ ID 385
<400> 2260
   aacactatat agaagttgtg cttacca 27
<210> 2269
   <211> 34
   <212> DNA
   <213> Homo sapiens
<220>
   <223> mature of SEQ ID 390
<400> 2269
   caggcagcag caggcgcacg gccgtctgga tctc 34
<210> 2278
   <211> 25
   <212> DNA
   <213> Homo sapiens
<220>
   <223> mature of SEQ ID 400
<400> 2278
   cttcagccaa tctgtgataa aaggg 25
<210> 2279
   <211> 31
   <212> DNA
   <213> Homo sapiens
<220>
   <223> mature of SEQ ID 400
<400> 2279
   ttatatcgac agttggtagc acaagccttt t 31
<210> 2280
   <211> 29
   <212> DNA
   <213> Homo sapiens
<220>
   <223> mature of SEQ ID 400
<400> 2280
   ccttttatat cgacagttgg tagcacaag 29
<210> 2313
   <211> 29
   <212> DNA
   <213> Homo sapiens
<220>
   <223> mature of SEQ ID 425
<400> 2313
   catttgatta caagcctcat tcagtacag 29
<210> 2314
   <211> 28
   <212> DNA
   <213> Homo sapiens
<220>
   <223> mature of SEQ ID 425
<400> 2314
   gctactgttg tgaatgataa tcaagtag 28
<210> 2360
   <211> 33
   <212> DNA
   <213> Homo sapiens
<220>
   <223> mature of SEQ ID 453
<400> 2360
   ccaggcacag aaagctgaag gtgctggaga tgc 33
<210> 2361
   <211> 30
   <212> DNA
   <213> Homo sapiens
<220>
   <223> mature of SEQ ID 453
<400> 2361
   acagaccagg cacagaaagc tgaaggtgct 30
<210> 2368
   <211> 26
   <212> DNA
   <213> Homo sapiens
<220>
   <223> mature of SEQ ID 458
<400> 2368
   agaaactaag agaaagtgtc aggagc 26
<210> 2369
   <211> 29
   <212> DNA
   <213> Homo sapiens
<220>
   <223> mature of SEQ ID 459
<400> 2369
   tgcatttttc tgtgtttctt tctaaccct 29
<210> 2389
   <211> 35
   <212> DNA
   <213> Homo sapiens
<220>
   <223> mature of SEQ ID 472
<400> 2389
   aaggcactcg ggcagcacaa agggagcaga tgccc 35
<210> 2390
   <211> 32
   <212> DNA
   <213> Homo sapiens
<220>
   <223> mature of SEQ ID 472
<400> 2390
   cagggggaag gcactcgggc agcacaaagg ga 32
<210> 2408
   <211> 30
   <212> DNA
   <213> Homo sapiens
<220>
   <223> mature of SEQ ID 489
<400> 2408
   gctattaatc tctgtggtca ggcctgtaaa 30
<210> 2409
   <211> 32
   <212> DNA
   <213> Homo sapiens
<220>
   <223> mature of SEQ ID 489
<400> 2409
   taatctctgt ggtcaggcct gtaaagctgg tc 32
<210> 2410
   <211> 34
   <212> DNA
   <213> Homo sapiens
<220>
   <223> mature of SEQ ID 489
<400> 2410
   agcccaggtt cacatggtta ccacattatt tggc 34
<210> 2419
   <211> 25
   <212> DNA
   <213> Homo sapiens
<220>
   <223> mature of SEQ ID 494
<400> 2419
   cttgttagac aatcacagct ggggg 25
<210> 2427
   <211> 31
   <212> DNA
   <213> Homo sapiens
<220>
   <223> mature of SEQ ID 500
<400> 2427
   gaaagcaagc gtgagatcat ttcatcagcg t 31
<210> 2428
   <211> 29
   <212> DNA
   <213> Homo sapiens
<220>
   <223> mature of SEQ ID 500
<400> 2428
   cttagaaagc aagcgtgaga tcatttcat 29
<210> 2429
   <211> 33
   <212> DNA
   <213> Homo sapiens
<220>
   <223> mature of SEQ ID 500
<400> 2429
   tttgaagggt ctctgttttc tctcttaatt tgg 33
<210> 2430
   <211> 31
   <212> DNA
   <213> Homo sapiens
<220>
   <223> mature of SEQ ID 500
<400> 2430
   atgctgtttg aagggtctct gttttctctc t 31
<210> 2431
   <211> 30
   <212> DNA
   <213> Homo sapiens
<220>
   <223> mature of SEQ ID 501
<400> 2431
   ggctaattag aataccaatt agaactggca 30
<210> 2432
   <211> 33
   <212> DNA
   <213> Homo sapiens
<220>
   <223> mature of SEQ ID 501
<400> 2432
   attagaatac caattagaac tggcagggct atc 33
<210> 2433
   <211> 35
   <212> DNA
   <213> Homo sapiens
<220>
   <223> mature of SEQ ID 501
<400> 2433
   tttgttaaaa agtttaatgt tcttaattag ttgaa 35
<210> 2434
   <211> 32
   <212> DNA
   <213> Homo sapiens
<220>
   <223> mature of SEQ ID 501
<400> 2434
   gatagccttt gttaaaaagt ttaatgttct ta 32
<210> 2437
   <211> 30
   <212> DNA
   <213> Homo sapiens
<220>
   <223> mature of SEQ ID 503
<400> 2437
   agaagcaata agaactgggt attggcttaa 30
<210> 2438
   <211> 32
   <212> DNA
   <213> Homo sapiens
<220>
   <223> mature of SEQ ID 503
<400> 2438
   caataagaac tgggtattgg cttaatttgg gg 32
<210> 2450
   <211> 31
   <212> DNA
   <213> Homo sapiens
<220>
   <223> mature of SEQ ID 512
<400> 2450
   acttgttcaa ggttacttta cccaccctcc t 31
<210> 2451
   <211> 29
   <212> DNA
   <213> Homo sapiens
<220>
   <223> mature of SEQ ID 512
<400> 2451
   gaccacttgt tcaaggttac tttacccac 29
<210> 2459
   <211> 21
   <212> DNA
   <213> Homo sapiens
<220>
   <223> mature of SEQ ID 517
<400> 2459
   ggagccagca gagggagctg t 21
<210> 2469
   <211> 28
   <212> DNA
   <213> Homo sapiens
<220>
   <223> mature of SEQ ID 527
<400> 2469
   gctcatacat atgcattgtt tttagatt 28
<210> 2470
   <211> 30
   <212> DNA
   <213> Homo sapiens
<220>
   <223> mature of SEQ ID 527
<400> 2470
   aatctgtagt cataagcagt gtgggtgaaa 30
<210> 2471
   <211> 33
   <212> DNA
   <213> Homo sapiens
<220>
   <223> mature of SEQ ID 527
<400> 2471
   gtagtcataa gcagtgtggg tgaaatacaa agt 33
<210> 2472
   <211> 23
   <212> DNA
   <213> Homo sapiens
<220>
   <223> mature of SEQ ID 528
<400> 2472
   agcctggaaa atggtaaatg atc 23
<210> 2476
   <211> 27
   <212> DNA
   <213> Homo sapiens
<220>
   <223> mature of SEQ ID 530
<400> 2476
   ggtgacaaaa tttacatctt tgtattc 27
<210> 2477
   <211> 27
   <212> DNA
   <213> Homo sapiens
<220>
   <223> mature of SEQ ID 531
<400> 2477
   ttcctgctcc cagagccata aagtggg 27
<210> 2482
   <211> 19
   <212> DNA
   <213> Homo sapiens
<220>
   <223> mature of SEQ ID 534
<400> 2482
   tggagaggga tgaaggaga 19
<210> 2489
   <211> 22
   <212> DNA
   <213> Homo sapiens
<220>
   <223> mature of SEQ ID 541
<400> 2489
   gggggtcaaa ggggagttct tt 22
<210> 2493
   <211> 25
   <212> DNA
   <213> Homo sapiens
<220>
   <223> mature of SEQ ID 542
<400> 2493
   gaagtgcggg gttattgtga gtgac 25
<210> 2522
   <211> 22
   <212> DNA
   <213> Homo sapiens
<220>
   <223> mature of SEQ ID 562
<400> 2522
   agaggaagga aggggaaaaa tg 22
<210> 2532
   <211> 21
   <212> DNA
   <213> Homo sapiens
<220>
   <223> mature of SEQ ID 407
<400> 2532
   ggaacccact taggtggcac c 21
<210> 2543
   <211> 28
   <212> DNA
   <213> Homo sapiens
<220>
   <223> mature of SEQ ID 579
<400> 2543
   ggggctttgg ttttgtgacc tactcttg 28
<210> 2613
   <211> 32
   <212> DNA
   <213> Homo sapiens
<220>
   <223> mature of SEQ ID 633
<400> 2613
   aagatagagg tcatttctat gcagagaaaa ac 32
<210> 2617
   <211> 30
   <212> DNA
   <213> Homo sapiens
<220>
   <223> mature of SEQ ID 638
<400> 2617
   tgccaccatg gcctgatgag tgatctggtg 30
<210> 2618
   <211> 32
   <212> DNA
   <213> Homo sapiens
<220>
   <223> mature of SEQ ID 638
<400> 2618
   ccatggcctg atgagtgatc tggtgggcga cg 32
<210> 2620
   <211> 22
   <212> DNA
   <213> Homo sapiens
<220>
   <223> mature of SEQ ID 640
<400> 2620
   gttgccataa caataagata tc 22
<210> 2621
   <211> 34
   <212> DNA
   <213> Homo sapiens
<220>
   <223> mature of SEQ ID 640
<400> 2621
   ataacaactt tgatatttct ttattatttt tagc 34
<210> 2622
   <211> 32
   <212> DNA
   <213> Homo sapiens
<220>
   <223> mature of SEQ ID 640
<400> 2622
   agttgctata acaactttga tatttcttta tt 32
<210> 2637
   <211> 31
   <212> DNA
   <213> Homo sapiens
<220>
   <223> mature of SEQ ID 651
<400> 2637
   ttccagccag gccacctctc ctttatctgc a 31
<210> 2648
   <211> 23
   <212> DNA
   <213> Homo sapiens
<220>
   <223> mature of SEQ ID 664
<400> 2648
   tggttctgca cagttaaaaa tag 23
<210> 2656
   <211> 26
   <212> DNA
   <213> Homo sapiens
<220>
   <223> mature of SEQ ID 669
<400> 2656
   gaaagctgaa ggtgctggag atgcca 26
<210> 2660
   <211> 25
   <212> DNA
   <213> Homo sapiens
<220>
   <223> mature of SEQ ID 671
<400> 2660
   tccattaggc cagcaacgct tgtag 25
<210> 2679
   <211> 20
   <212> DNA
   <213> Homo sapiens
<220>
   <223> mature of SEQ ID 683
<400> 2679
   aatcacacca ttcatgcatg 20
<210> 2712
   <211> 19
   <212> DNA
   <213> Homo sapiens
<220>
   <223> mature of SEQ ID 712
<400> 2712
   aatgttaata aaggtttcg 19
<210> 2719
   <211> 27
   <212> DNA
   <213> Homo sapiens
<220>
   <223> mature of SEQ ID 716
<400> 2719
   cacggtaggt tcattagtca ataaagc 27
<210> 2730
   <211> 34
   <212> DNA
   <213> Homo sapiens
<220>
   <223> mature of SEQ ID 728
<400> 2730
   agtaattgcg ggtaatttat ttcttggagg gcag 34
<210> 2746
   <211> 22
   <212> DNA
   <213> Homo sapiens
<220>
   <223> mature of SEQ ID 738
<400> 2746
   catttctctt tcataacggg cc 22
<210> 2754
   <211> 33
   <212> DNA
   <213> Homo sapiens
<220>
   <223> mature of SEQ ID 744
<400> 2754
   aatagtgcat aaaatgtcac cacttcattt aac 33
<210> 2755
   <211> 30
   <212> DNA
   <213> Homo sapiens
<220>
   <223> mature of SEQ ID 744
<400> 2755
   ggcttaatag tgcataaaat gtcaccactt 30
<210> 2756
   <211> 21
   <212> DNA
   <213> Homo sapiens
<220>
   <223> mature of SEQ ID 744
<400> 2756
   tgtaatcatg cattatgagt c 21
<210> 2771
   <211> 34
   <212> DNA
   <213> Homo sapiens
<220>
   <223> mature of SEQ ID 753
<400> 2771
   gtttactgca gcttttgact tcttcaaaga gctg 34
<210> 2791
   <211> 34
   <212> DNA
   <213> Homo sapiens
<220>
   <223> mature of SEQ ID 770
<400> 2791
   ctatgcgtct tgacggattt ggggttggca gagc 34
<210> 2809
   <211> 29
   <212> DNA
   <213> Homo sapiens
<220>
   <223> mature of SEQ ID 784
<400> 2809
   cacccatcgg ttatctgtgt cgcctgaag 29
<210> 2810
   <211> 31
   <212> DNA
   <213> Homo sapiens
<220>
   <223> mature of SEQ ID 784
<400> 2810
   gtaaggtaca ggaaaaaggt gggggtattg t 31
<210> 2814
   <211> 25
   <212> DNA
   <213> Homo sapiens
<220>
   <223> mature of SEQ ID 786
<400> 2814
   ttggaccagt catctggctc tgcct 25
<210> 2815
   <211> 27
   <212> DNA
   <213> Homo sapiens
<220>
   <223> mature of SEQ ID 786
<400> 2815
   gctggctgct ggtgaggcca ggaggcc 27
<210> 2862
   <211> 24
   <212> DNA
   <213> Homo sapiens
<220>
   <223> mature of SEQ ID 826
<400> 2862
   atagcaatac tttgtgaggt taat 24
<210> 2863
   <211> 29
   <212> DNA
   <213> Homo sapiens
<220>
   <223> mature of SEQ ID 826
<400> 2863
   agtgcagatg caaataatcc taacaaatt 29
<210> 2865
   <211> 20
   <212> DNA
   <213> Homo sapiens
<220>
   <223> mature of SEQ ID 827
<400> 2865
   gtgctgggtg ggtggttttt 20
<210> 2870
   <211> 31
   <212> DNA
   <213> Homo sapiens
<220>
   <223> mature of SEQ ID 832
<400> 2870
   catgtgattt ctgcccagtg ctctgaatgt c 31
<210> 2871
   <211> 33
   <212> DNA
   <213> Homo sapiens
<220>
   <223> mature of SEQ ID 832
<400> 2871
   atttctgccc agtgctctga atgtcaaagt gaa 33
<210> 2872
   <211> 25
   <212> DNA
   <213> Homo sapiens
<220>
   <223> mature of SEQ ID 832
<400> 2872
   cacgggtaaa cggcgggagt aacta 25
<210> 2877
   <211> 30
   <212> DNA
   <213> Homo sapiens
<220>
   <223> mature of SEQ ID 837
<400> 2877
   gggagaagga taaagtggaa atctaatttt 30
<210> 2879
   <211> 28
   <212> DNA
   <213> Homo sapiens
<220>
   <223> mature of SEQ ID 838
<400> 2879
   aggacaatat attaaatgga tttttgga 28
<210> 2895
   <211> 24
   <212> DNA
   <213> Homo sapiens
<220>
   <223> mature of SEQ ID 852
<400> 2895
   tgctgaagct gctttttaaa gggg 24
<210> 2900
   <211> 28
   <212> DNA
   <213> Homo sapiens
<220>
   <223> mature of SEQ ID 858
<400> 2900
   tactgctttt gaaggttcca ggtctgtg 28
<210> 2924
   <211> 25
   <212> DNA
   <213> Homo sapiens
<220>
   <223> mature of SEQ ID 874
<400> 2924
   ggctttcctc ctctctgcag gggag 25
<210> 2931
   <211> 34
   <212> DNA
   <213> Homo sapiens
<220>
   <223> mature of SEQ ID 878
<400> 2931
   aaggcggcgc gtgcggttgg gaacgcggag cgga 34
<210> 2932
   <211> 32
   <212> DNA
   <213> Homo sapiens
<220>
   <223> mature of SEQ ID 878
<400> 2932
   gttccagaag gcggcgcgtg cggttgggaa cg 32
<210> 2958
   <211> 35
   <212> DNA
   <213> Homo sapiens
<220>
   <223> mature of SEQ ID 903
<400> 2958
   tctgcccttg gatacacaca acaaaacccc cattg 35
<210> 2959
   <211> 32
   <212> DNA
   <213> Homo sapiens
<220>
   <223> mature of SEQ ID 903
<400> 2959
   gctggattct gcccttggat acacacaaca aa 32
<210> 2960
   <211> 32
   <212> DNA
   <213> Homo sapiens
<220>
   <223> mature of SEQ ID 903
<400> 2960
   caatggaaat tgtgcatgca tatccagggg ca 32
<210> 2961
   <211> 34
   <212> DNA
   <213> Homo sapiens
<220>
   <223> mature of SEQ ID 903
<400> 2961
   aattgtgcat gcatatccag gggcagaatt tggc 34
<210> 2964
   <211> 29
   <212> DNA
   <213> Homo sapiens
<220>
   <223> mature of SEQ ID 906
<400> 2964
   taggtgtggt tgcagttgga gaggatccc 29
<210> 2966
   <211> 29
   <212> DNA
   <213> Homo sapiens
<220>
   <223> mature of SEQ ID 909
<400> 2966
   tagtgctaaa ctcatttgca caactacag 29
<210> 2967
   <211> 32
   <212> DNA
   <213> Homo sapiens
<220>
   <223> mature of SEQ ID 909
<400> 2967
   gctaaactca tttgcacaac tacagttaat tg 32
<210> 2968
   <211> 32
   <212> DNA
   <213> Homo sapiens
<220>
   <223> mature of SEQ ID 909
<400> 2968
   caattaatta atgtgttgga aatttggcac tg 32
<210> 2971
   <211> 31
   <212> DNA
   <213> Homo sapiens
<220>
   <223> mature of SEQ ID 911
<400> 2971
   taggacacat ggtctacttc ttctcaatat c 31
<210> 2972
   <211> 29
   <212> DNA
   <213> Homo sapiens
<220>
   <223> mature of SEQ ID 911
<400> 2972
   gacgtaggac acatggtcta cttcttctc 29
<210> 2973
   <211> 29
   <212> DNA
   <213> Homo sapiens
<220>
   <223> mature of SEQ ID 911
<400> 2973
   ggtatttgaa gatgcggttg accatggtg 29
<210> 2974
   <211> 32
   <212> DNA
   <213> Homo sapiens
<220>
   <223> mature of SEQ ID 911
<400> 2974
   tttgaagatg cggttgacca tggtgtgtac gc 32
<210> 2999
   <211> 21
   <212> DNA
   <213> Homo sapiens
<220>
   <223> mature of SEQ ID 926
<400> 2999
   aggaggagga ggcagggccg a 21
<210> 3000
   <211> 32
   <212> DNA
   <213> Homo sapiens
<220>
   <223> mature of SEQ ID 927
<400> 3000
   tctctaaaag gtttctaaat gatgttgatg tt 32
<210> 3001
   <211> 22
   <212> DNA
   <213> Homo sapiens
<220>
   <223> mature of SEQ ID 927
<400> 3001
   ccatttaaca gccttttaaa ga 22
<210> 3015
   <211> 34
   <212> DNA
   <213> Homo sapiens
<220>
   <223> mature of SEQ ID 940
<400> 3015
   taatgagggg aaaaattctc cagctggggc tgag 34
<210> 3017
   <211> 30
   <212> DNA
   <213> Homo sapiens
<220>
   <223> mature of SEQ ID 942
<400> 3017
   cataaataca ttgtaatgtc ccatgtaatt 30
<210> 3018
   <211> 32
   <212> DNA
   <213> Homo sapiens
<220>
   <223> mature of SEQ ID 942
<400> 3018
   atacattgta atgtcccatg taattacatg gt 32
<210> 3019
   <211> 33
   <212> DNA
   <213> Homo sapiens.
<220>
   <223> mature of SEQ ID 942
<400> 3019
   gtactctgta attactgtac attgtaacaa atg 33
<210> 3021
   <211> 27
   <212> DNA
   <213> Homo sapiens
<220>
   <223> mature of SEQ ID 944
<400> 3021
   gcacattata aactctaatt cattaac 27
<210> 3023
   <211> 20
   <212> DNA
   <213> Homo sapiens
<220>
   <223> mature of SEQ ID 946
<400> 3023
   aatacataaa tgaacgggta 20
<210> 3024
   <211> 24
   <212> DNA
   <213> Homo sapiens
<220>
   <223> mature of SEQ ID 946
<400> 3024
   aaagagccac ctcatattca tagg 24
<210> 3025
   <211> 33
   <212> DNA
   <213> Homo sapiens
<220>
   <223> mature of SEQ ID 947
<400> 3025
   gcagagagag aggggagaaa acagggagat aca 33
<210> 3026
   <211> 31
   <212> DNA
   <213> Homo sapiens
<220>
   <223> mature of SEQ ID 947
<400> 3026
   ggaggggcag agagagaggg gagaaaacag g 31
<210> 3027
   <211> 29
   <212> DNA
   <213> Homo sapiens
<220>
   <223> mature of SEQ ID 947
<400> 3027
   tcattgttat tccaaaagtc tctgtctag 29
<210> 3028
   <211> 23
   <212> DNA
   <213> Homo sapiens
<220>
   <223> mature of SEQ ID 949
<400> 3028
   cttgagcagg aagagtttat ttt 23
<210> 3029
   <211> 23
   <212> DNA
   <213> Homo sapiens
<220>
   <223> mature of SEQ ID 951
<400> 3029
   gagtgacaaa tttggtactt aaa 23
<210> 3030
   <211> 19
   <212> DNA
   <213> Homo sapiens
<220>
   <223> mature of SEQ ID 951
<400> 3030
   gttaaaaaca accctgtca 19
<210> 3038
   <211> 30
   <212> DNA
   <213> Homo sapiens
<220>
   <223> mature of SEQ ID 955
<400> 3038
   gggatttatt taattcagtc tcacagcctg 30
<210> 3039
   <211> 24
   <212> DNA
   <213> Homo sapiens
<220>
   <223> mature of SEQ ID 955
<400> 3039
   caggctgaca actgatatca caag 24

<210> 3048
   <211> 33
   <212> DNA
   <213> Homo sapiens
<220>
   <223> mature of SEQ ID 963
<400> 3048
   cagttcccat agcaactggg ctgtagcagc cag 33
<210> 3049
   <211> 31
   <212> DNA
   <213> Homo sapiens
<220>
   <223> mature of SEQ ID 963
<400> 3049
   ccctcccagt tcccatagca actgggctgt a 31
<210> 3050
   <211> 21
   <212> DNA
   <213> Homo sapiens
<220>
   <223> mature of SEQ ID 963
<400> 3050
   actgaggtgg ggaaaggagg g 21
<210> 3052
   <211> 19
   <212> DNA
   <213> Homo sapiens
<220>
   <223> mature of SEQ ID 967
<400> 3052
   aaattaaaat ccaatacac 19
<210> 3053
   <211> 31
   <212> DNA
   <213> Homo sapiens
<220>
   <223> mature of SEQ ID 967
<400> 3053
   agtgcaattt gtgtataaca ttttagtact t 31
<210> 3054
   <211> 34
   <212> DNA
   <213> Homo sapiens
<220>
   <223> mature of SEQ ID 967
<400> 3054
   atttgtgtat aacattttag tacttaaatc aact 34
<210> 3061
   <211> 36
   <212> DNA
   <213> Homo sapiens
<220>
   <223> mature of SEQ ID 975
<400> 3061
   gggaaaatga aaaccagaaa attaattaat ctctaa 36
<210> 3062
   <211> 34
   <212> DNA
   <213> Homo sapiens
<220>
   <223> mature of SEQ ID 975
<400> 3062
   tgattgccag ggaaaatgaa aaccagaaaa ttaa 34
<210> 3067
   <211> 32
   <212> DNA
   <213> Homo sapiens
<220>
   <223> mature of SEQ ID 978
<400> 3067
   atctttgggt gaggtccaac cagagaggga gc 32
<210> 3142
   <211> 23
   <212> DNA
   <213> Homo sapiens
<220>
   <223> mature of SEQ ID 1039
<400> 3142
   gggaatctat tctccccatt tcc 23
<210> 3149
   <211> 20
   <212> DNA
   <213> Homo sapiens
<220>
   <223> mature of SEQ ID 1045
<400> 3149
   gggaagggtg gggtttccat 20
<210> 3162
   <211> 29
   <212> DNA
   <213> Homo sapiens
<220>
   <223> mature of SEQ ID 1060
<400> 3162
   ttccaagaag atgagcagtt tctgggagc 29
<210> 3169
   <211> 32
   <212> DNA
   <213> Homo sapiens
<220>
   <223> mature of SEQ ID 1065
<400> 3169
   ggcgcgggcc ggagagcggg tgtgcaaagt gg 32
<210> 3170
   <211> 29
   <212> DNA
   <213> Homo sapiens
<220>
   <223> mature of SEQ ID 1065
<400> 3170
   caggggcgcg ggccggagag cgggtgtgc 29
<210> 3189
   <211> 33
   <212> DNA
   <213> Homo sapiens
<220>
   <223> mature of SEQ ID 1083
<400> 3189
   cccctctgcc acgcccccac cttcctggca ggt 33
<210> 3209
   <211> 33
   <212> DNA
   <213> Homo sapiens
<220>
   <223> mature of SEQ ID 1100
<400> 3209
   tgcatctgac tcccatccct ggataaatat tgt 33
<210> 3210
   <211> 31
   <212> DNA
   <213> Homo sapiens
<220>
   <223> mature of SEQ ID 1100
<400> 3210
   ggtcgctgca tctgactccc atccctggat a 31
<210> 3211
   <211> 26
   <212> DNA
   <213> Homo sapiens
<220>
   <223> mature of SEQ ID 1100
<400> 3211
   ttcagaagct atttaactgg gtgacc 26
<210> 3212
   <211> 35
   <212> DNA
   <213> Homo sapiens
<220>
   <223> mature of SEQ ID 1100
<400> 3212
   tgcagggagg ttcagaagct atttaactgg gtgac 35
<210> 3213
   <211> 35
   <212> DNA
   <213> Homo sapiens
<220>
   <223> mature of SEQ ID 1100
<400> 3213
   gcaattagaa tgcagggagg ttcagaagct attta 35
<210> 3230
   <211> 33
   <212> DNA
   <213> Homo sapiens
<220>
   <223> mature of SEQ ID 1117
<400> 3230
   tccaattaga gctgttagag gattctggca ggg 33
<210> 3231
   <211> 30
   <212> DNA
   <213> Homo sapiens
<220>
   <223> mature of SEQ ID 1117
<400> 3231
   gtttttccaa ttagagctgt tagaggattc 30
<210> 3232
   <211> 22
   <212> DNA
   <213> Homo sapiens
<220>
   <223> mature of SEQ ID 1118
<400> 3232
   ggagtaacgg gacgtcgccg cg 22
<210> 3233
   <211> 21
   <212> DNA
   <213> Homo sapiens
<220>
   <223> mature of SEQ ID 1118
<400> 3233
   gggggcgagg ctatgtcgcg g 21
<210> 3249
   <211> 33
   <212> DNA
   <213> Homo sapiens
<220>
   <223> mature of SEQ ID 1129
<400> 3249
   gtttgttagg cttcgcatgc tggtctccat atg 33
<210> 3250
   <211> 30
   <212> DNA
   <213> Homo sapiens
<220>
   <223> mature of SEQ ID 1129
<400> 3250
   gaagtgtttg ttaggcttcg catgctggtc 30
<210> 3260
   <211> 20
   <212> DNA
   <213> Homo sapiens
<220>
   <223> mature of SEQ ID 1136
<400> 3260
   ctcaaacctg gcatggtggt 20
<210> 3261
   <211> 30
   <212> DNA
   <213> Homo sapiens
<220>
   <223> mature of SEQ ID 1137
<400> 3261
   agatctctgg cagtggagga agtctcttta 30
<210> 3271
   <211> 33
   <212> DNA
   <213> Homo sapiens
<220>
   <223> mature of SEQ ID 1145
<400> 3271
   gagcaaacag tgcaattttc tgtaattatg cca 33
<210> 3272
   <211> 31
   <212> DNA
   <213> Homo sapiens
<220>
   <223> mature of SEQ ID 1145
<400> 3272
   aaaattgagc aaacagtgca attttctgta a 31
<210> 3273
   <211> 32
   <212> DNA
   <213> Homo sapiens
<220>
   <223> mature of SEQ ID 1145
<400> 3273
   aataatttac atagttttgc attagctcaa tt 32
<210> 3274
   <211> 30
   <212> DNA
   <213> Homo sapiens
<220>
   <223> mature of SEQ ID 1145
<400> 3274
   tggtaaataa tttacatagt tttgcattag 30
<210> 3300
   <211> 29
   <212> DNA
   <213> Homo sapiens
<220>
   <223> mature of SEQ ID 1160
<400> 3300
   gcagttgtgc aacatgaaaa ctgcagtga 29
<210> 3301
   <211> 31
   <212> DNA
   <213> Homo sapiens
<220>
   <223> mature of SEQ ID 1160
<400> 3301
   ttgtgcaaca tgaaaactgc agtgacatgt t 31
<210> 3302
   <211> 32
   <212> DNA
   <213> Homo sapiens
<220>
   <223> mature of SEQ ID 1161
<400> 3302
   attcttgatt tagttttgag agaaaagggt tt 32
<210> 3303
   <211> 30
   <212> DNA
   <213> Homo sapiens
<220>
   <223> mature of SEQ ID 1161
<400> 3303
   ctaccattct tgatttagtt ttgagagaaa 30
<210> 3304
   <211> 23
   <212> DNA
   <213> Homo sapiens
<220>
   <223> mature of SEQ ID 1161
<400> 3304
   cagctggaga tggctggaaa gtc 23
<210> 3305
   <211> 28
   <212> DNA
   <213> Homo sapiens
<220>
   <223> mature of SEQ ID 1162
<400> 3305
   gacacatctg tggtgctgtc catggaca 28
<210> 3320
   <211> 32
   <212> DNA
   <213> Homo sapiens
<220>
   <223> mature of SEQ ID 1173
<400> 3320
   cgaggtgaac aatggctggc tctgtccctg ag 32
<210> 3321
   <211> 29
   <212> DNA
   <213> Homo sapiens
<220>
   <223> mature of SEQ ID 1173
<400> 3321
   ttaacgaggt gaacaatggc tggctctgt 29
<210> 3322
   <211> 20
   <212> DNA
   <213> Homo sapiens
<220>
   <223> mature of SEQ ID 1173
<400> 3322
   cttaggggtg ggggagccct 20
<210> 3323
   <211> 32
   <212> DNA
   <213> Homo sapiens
<220>
   <223> mature of SEQ ID 1174
<400> 3323
   ctttccccct cccatgtgct ttccttcatt tg 32
<210> 3324
   <211> 30
   <212> DNA
   <213> Homo sapiens
<220>
   <223> mature of SEQ ID 1174
<400> 3324
   atcacctttc cccctcccat gtgctttcct 30
<210> 3325
   <211> 25
   <212> DNA
   <213> Homo sapiens
<220>
   <223> mature of SEQ ID 1174
<400> 3325
   tttatttggg agggggaagg gtgat 25
<210> 3326
   <211> 35
   <212> DNA
   <213> Homo sapiens
<220>
   <223> mature of SEQ ID 1175
<400> 3326
   gataataccg gcacctcttt tcactctaat ctcct 35
<210> 3327
   <211> 35
   <212> DNA
   <213> Homo sapiens
<220>
   <223> mature of SEQ ID 1175
<400> 3327
   gcacctcttt tcactctaat ctcctacccg tctga 35
<210> 3328
   <211> 26
   <212> DNA
   <213> Homo sapiens
<220>
   <223> mature of SEQ ID 1175
<400> 3328
   tcactctaat ctcctacccg tctgag 26
<210> 3329
   <211> 23
   <212> DNA
   <213> Homo sapiens
<220>
   <223> mature of SEQ ID 1175
<400> 3329
   ttcagcagga gataggggca tta 23
<210> 3363
   <211> 25
   <212> DNA
   <213> Homo sapiens
<220>
   <223> mature of SEQ ID 1198
<400> 3363
   ggggcgggga ggaattccgg ttctc 25
<210> 3367
   <211> 32
   <212> DNA
   <213> Homo sapiens
<220>
   <223> mature of SEQ ID 1201
<400> 3367
   tgtttcctct tctcctcttc tggagaggga tg 32
<210> 3372
   <211> 33
   <212> DNA
   <213> Homo sapiens
<220>
   <223> mature of SEQ ID 1205
<400> 3372
   ttttctgtaa cagtcatttg acatttaata tac 33
<210> 3373
   <211> 30
   <212> DNA
   <213> Homo sapiens
<220>
   <223> mature of SEQ ID 1205
<400> 3373
   agtgcttttc tgtaacagtc atttgacatt 30
<210> 3374
   <211> 32
   <212> DNA
   <213> Homo sapiens
<220>
   <223> mature of SEQ ID 1205
<400> 3374
   gtattattaa atttaaatgc atatttgcaa tt 32
<210> 3375
   <211> 35
   <212> DNA
   <213> Homo sapiens
<220>
   <223> mature of SEQ ID 1205
<400> 3375
   taaatttaaa tgcatatttg caattctaaa agcac 35
<210> 3378
   <211> 23
   <212> DNA
   <213> Homo sapiens
<220>
   <223> mature of SEQ ID 1207
<400> 3378
   tagacaatga aaaaccctgc atg 23
<210> 3379
   <211> 23
   <212> DNA
   <213> Homo sapiens
<220>
   <223> mature of SEQ ID 1207
<400> 3379
   taggtttatg tgaaacttct gga 23
<210> 3381
   <211> 23
   <212> DNA
   <213> Homo sapiens
<220>
   <223> mature of SEQ ID 1212
<400> 3381
   gcaaagtctg ttattggtga tga 23
<210> 3391
   <211> 20
   <212> DNA
   <213> Homo sapiens
<220>
   <223> mature of SEQ ID 1217
<400> 3391
   attaaccagt actagacagc 20
<210> 3392
   <211> 26
   <212> DNA
   <213> Homo sapiens
<220>
   <223> mature of SEQ ID 1217
<400> 3392
   tgctcgctaa ctataaacta tctgat 26
<210> 3416
   <211> 20
   <212> DNA
   <213> Homo sapiens
<220>
   <223> mature of SEQ ID 1233
<400> 3416
   gggattctgg ccaaatccct 20
<210> 3417
   <211> 29
   <212> DNA
   <213> Homo sapiens
<220>
   <223> mature of SEQ ID 1233
<400> 3417
   agttgcttgg cacagaatta ttgattggc 29
<210> 3422
   <211> 33
   <212> DNA
   <213> Homo sapiens
<220>
   <223> mature of SEQ ID 1238
<400> 3422
   gcccccaaac atcttatttg tcttgatatt tct 33
<210> 3423
   <211> 31
   <212> DNA
   <213> Homo sapiens
<220>
   <223> mature of SEQ ID 1238
<400> 3423
   tggtgtgccc ccaaacatct tatttgtctt g 31
<210> 3424
   <211> 25
   <212> DNA
   <213> Homo sapiens
<220>
   <223> mature of SEQ ID 1238
<400> 3424
   agaaatacat atgacagata aggag 25
<210> 3440
   <211> 28
   <212> DNA
   <213> Homo sapiens
<220>
   <223> mature of SEQ ID 1251
<400> 3440
   gcaggtgaca tcggagaaac atgagtga 28
<210> 3441
   <211> 32
   <212> DNA
   <213> Homo sapiens
<220>
   <223> mature of SEQ ID 1251
<400> 3441
   ctgaatttct tcatgttgat gttccttcac ct 32
<210> 3442
   <211> 30
   <212> DNA
   <213> Homo sapiens
<220>
   <223> mature of SEQ ID 1251
<400> 3442
   acattctgaa tttcttcatg ttgatgttcc 30
<210> 3461
   <211> 32
   <212> DNA
   <213> Homo sapiens
<220>
   <223> mature of SEQ ID 1267
<400> 3461
   atttaatgta atttccctaa aatggaggta at 32
<210> 3482
   <211> 29
   <212> DNA
   <213> Homo sapiens
<220>
   <223> mature of SEQ ID 1282
<400> 3482
   ccctattaat tttattgttt caataaagc 29
<210> 3497
   <211> 29
   <212> DNA
   <213> Homo sapiens
<220>
   <223> mature of SEQ ID 1299
<400> 3497
   gtcatgatgt tgcgtcacca cgtgatgac 29
<210> 3498
   <211> 31
   <212> DNA
   <213> Homo sapiens

<220>
   <223> mature of SEQ ID 1299
<400> 3498
   tgatgttgcg tcaccacgtg atgacgctgt g 31
<210> 3499
   <211> 32
   <212> DNA
   <213> Homo sapiens
<220>
   <223> mature of SEQ ID 1299
<400> 3499
   cacatcatcg catattgaca caatgtcatg gc 32
<210> 3528
   <211> 32
   <212> DNA
   <213> Homo sapiens
<220>
   <223> mature of SEQ ID 1323
<400> 3528
   ggaccttctg gaggtaaatc tgcagaatgg ca 32
<210> 3552
   <211> 23
   <212> DNA
   <213> Homo sapiens
<220>
   <223> mature of SEQ ID 1341
<400> 3552
   aggctgcggc ggacgccagc atc 23
<210> 3555
   <211> 23
   <212> DNA
   <213> Homo sapiens
<220>
   <223> mature of SEQ ID 1343
<400> 3555
   gtcactatgg caggaaaagt aat 23
<210> 3556
   <211> 29
   <212> DNA
   <213> Homo sapiens
<220>
   <223> mature of SEQ ID 1343
<400> 3556
   tgccacttcc tgcaaacagc aagattgtc 29
<210> 3557
   <211> 32
   <212> DNA
   <213> Homo sapiens
<220>
   <223> mature of SEQ ID 1343
<400> 3557
   acttcctgca aacagcaaga ttgtcgctgt aa 32
<210> 3559
   <211> 20
   <212> DNA
   <213> Homo sapiens
<220>
   <223> mature of SEQ ID 1346
<400> 3559
   tggggagagg ataccctgat 20
<210> 3567
   <211> 29
   <212> DNA
   <213> Homo sapiens
<220>
   <223> mature of SEQ ID 1352
<400> 3567
   gcaacagtaa atttcaaggc tagttaaga 29
<210> 3593
   <211> 30
   <212> DNA
   <213> Homo sapiens
<220>
   <223> mature of SEQ ID 1370
<400> 3593
   cggtggaggg aaaggggaaa ggagccattt 30
<210> 3594
   <211> 32
   <212> DNA
   <213> Homo sapiens
<220>
   <223> mature of SEQ ID 1370
<400> 3594
   gagggaaagg ggaaaggagc cattttctgc tg 32
<210> 3596
   <211> 24
   <212> DNA
   <213> Homo sapiens
<220>
   <223> mature of SEQ ID 1371
<400> 3596
   gcaattcttc taggagtgga gcct 24
<210> 3619
   <211> 31
   <212> DNA
   <213> Homo sapiens
<220>
   <223> mature of SEQ ID 1396
<400> 3619
   ctcagcaggt agatgatggc tatgcggtgc c 31
<210> 3620
   <211> 34
   <212> DNA
   <213> Homo sapiens
<220>
   <223> mature of SEQ ID 1396
<400> 3620
   aggtagatga tggctatgcg gtgccagcct gcca 34
<210> 3621
   <211> 21
   <212> DNA
   <213> Homo sapiens
<220>
   <223> mature of SEQ ID 1396
<400> 3621
   tggcaggaag atggcactgg c 21
<210> 3627
   <211> 22
   <212> DNA
   <213> Homo sapiens
<220>
   <223> mature of SEQ ID 1403
<400> 3627
   tggggtttgt gctgaaattg cc 22
<210> 3628
   <211> 33
   <212> DNA
   <213> Homo sapiens
<220>
   <223> mature of SEQ ID 1403
<400> 3628
   ggtaattcaa aacaaatttc aagctgtgac aat 33
<210> 3655
   <211> 25
   <212> DNA
   <213> Homo sapiens
<220>
   <223> mature of SEQ ID 1425
<400> 3655
   tggtgctttg caggaggcaa gtgag 25
<210> 3663
   <211> 34
   <212> DNA
   <213> Homo sapiens
<220>
   <223> mature of SEQ ID 1429
<400> 3663
   aatgcaggca ggttgtgatt agaggtgagc cagt 34
<210> 3670
   <211> 21
   <212> DNA
   <213> Homo sapiens
<220>
   <223> mature of SEQ ID 1433
<400> 3670
   gctttggact tgaatacccc c 21
<210> 3786
   <211> 23
   <212> DNA
   <213> Homo sapiens
<220>
   <223> mature of SEQ ID 1531
<400> 3786
   gagtggctgg ctaatgagag gtt 23
<210> 3804
   <211> 33
   <212> DNA
   <213> Homo sapiens
<220>
   <223> mature of SEQ ID 1545
<400> 3804
   tgcagggcct ggcctacaat actggagtta agg 33
<210> 3805
   <211> 31
   <212> DNA
   <213> Homo sapiens
<220>
   <223> mature of SEQ ID 1545
<400> 3805
   tcagaatgca gggcctggcc tacaatactg g 31
<210> 3806
   <211> 30
   <212> DNA
   <213> Homo sapiens
<220>
   <223> mature of SEQ ID 1545
<400> 3806
   tcttaacttc ttccaaggtt accaggtcag 30
<210> 3807
   <211> 33
   <212> DNA
   <213> Homo sapiens
<220>
   <223> mature of SEQ ID 1545
<400> 3807
   acttcttcca aggttaccag gtcagtagct tga 33
<210> 3808
   <211> 33
   <212> DNA
   <213> Homo sapiens
<220>
   <223> mature of SEQ ID 1547
<400> 3808
   cagggcaatt tgcataaaaa gtgatgacaa aga 33
<210> 3862
   <211> 25
   <212> DNA
   <213> Homo sapiens
<220>
   <223> mature of SEQ ID 1586
<400> 3862
   ggaaaatgga cgtgtgtctc atggt 25
<210> 3863
   <211> 28
   <212> DNA
   <213> Homo sapiens
<220>
   <223> mature of SEQ ID 1586
<400> 3863
   cataaactct gaaccagaga tcataccc 28
<210> 3870
   <211> 22
   <212> DNA
   <213> Homo sapiens
<220>
   <223> mature of SEQ ID 1592
<400> 3870
   ggcccggtgc ccggcgggcg gc 22
<210> 3871
   <211> 25
   <212> DNA
   <213> Homo sapiens
<220>
   <223> mature of SEQ ID 1592
<400> 3871
   gtccggagcg gccgatgggg cccgt 25
<210> 3882
   <211> 23
   <212> DNA
   <213> Homo sapiens
<220>
   <223> mature of SEQ ID 1601
<400> 3882
   gcagggagtg ggaaggaagt cta 23
<210> 3896
   <211> 30
   <212> DNA
   <213> Homo sapiens
<220>
   <223> mature of SEQ ID 1610
<400> 3896
   ggagctaaat atagaaaggg ctccctcctc 30
<210> 3897
   <211> 32
   <212> DNA
   <213> Homo sapiens
<220>
   <223> mature of SEQ ID 1610
<400> 3897
   taaatataga aagggctccc tcctccccca gc 32
<210> 3898
   <211> 20
   <212> DNA
   <213> Homo sapiens
<220>
   <223> mature of SEQ ID 1610
<400> 3898
   gctggaagtc agcctctttc 20
<210> 3912
   <211> 22
   <212> DNA
   <213> Homo sapiens
<220>
   <223> mature of SEQ ID 1617
<400> 3912
   agtggacctg aagggttttg ac 22
<210> 3914
   <211> 23
   <212> DNA
   <213> Homo sapiens
<220>
   <223> mature of SEQ ID 1620
<400> 3914
   taatctccac agtatgtact tta 23
<210> 3931
   <211> 33
   <212> DNA
   <213> Homo sapiens
<220>
   <223> mature of SEQ ID 1634
<400> 3931
   gaatatttta tttgaaagca aatgctaaaa gta 33
<210> 3943
   <211> 34
   <212> DNA
   <213> Homo sapiens
<220>
   <223> mature of SEQ ID 1645
<400> 3943
   gcttgagaaa ctttgtttgc cctagactca gcac 34
<210> 3948
   <211> 28
   <212> DNA
   <213> Homo sapiens
<220>
   <223> mature of SEQ ID 1648
<400> 3948
   gttggacgct ccgggaaaca aagcaacc 28
<210> 3950
   <211> 33
   <212> DNA
   <213> Homo sapiens
<220>
   <223> mature of SEQ ID 1649
<400> 3950
   aagtggtatc tgttgtttaa tcgaggacag atg 33
<210> 3951
   <211> 31
   <212> DNA
   <213> Homo sapiens
<220>
   <223> mature of SEQ ID 1649
<400> 3951
   gtctgtaagt ggtatctgtt gtttaatcga g 31
<210> 3956
   <211> 29
   <212> DNA
   <213> Homo sapiens
<220>
   <223> mature of SEQ ID 1653
<400> 3956
   ttattccagg gagactctgt aatagatac 29
<210> 3957
   <211> 24
   <212> DNA
   <213> Homo sapiens
<220>
   <223> mature of SEQ ID 1653
<400> 3957
   caatctgtaa gctattacag tttt 24
<210> 3959
   <211> 33
   <212> DNA
   <213> Homo sapiens
<220>
   <223> mature of SEQ ID 1656
<400> 3959
   gcctgatggg agcaggaggc ggtgaggggg cgg 33
<210> 3960
   <211> 30
   <212> DNA
   <213> Homo sapiens
<220>
   <223> mature of SEQ ID 1656
<400> 3960
   ggttggcctg atgggagcag gaggcggtga 30
<210> 3966
   <211> 34
   <212> DNA
   <213> Homo sapiens
<220>
   <223> mature of SEQ ID 1661
<400> 3966
   attatgttgg tttgaaagga ggaaatagca aggt 34
<210> 3967
   <211> 30
   <212> DNA
   <213> Homo sapiens
<220>
   <223> mature of SEQ ID 1661
<400> 3967
   gccttgctgt atttcttata ccatttatta 30
<210> 3968
   <211> 33
   <212> DNA
   <213> Homo sapiens
<220>
   <223> mature of SEQ ID 1661
<400> 3968
   gctgtatttc ttataccatt tattaaaaag ctg 33
<210> 3973
   <211> 27
   <212> DNA
   <213> Homo sapiens
<220>
   <223> mature of SEQ ID 1667
<400> 3973
   cgcgcggaga ggctcttaaa gggccag 27
<210> 3974
   <211> 32
   <212> DNA
   <213> Homo sapiens
<220>
   <223> mature of SEQ ID 1667
<400> 3974
   ataaaatttt aatggtctct ctggctgatc cg 32
<210> 3975
   <211> 35
   <212> DNA
   <213> Homo sapiens
<220>
   <223> mature of SEQ ID 1667
<400> 3975 ..
   ttcattctgc ataaaatttt aatggtctct ctggc 35
<210> 3976
   <211> 35
   <212> DNA
   <213> Homo sapiens
<220>
   <223> mature of SEQ ID 1667
<400> 3976
   ctggcccatt ttcattctgc ataaaatttt aatgg 35
<210> 3986
   <211> 19
   <212> DNA
   <213> Homo sapiens
<220>
   <223> mature of SEQ ID 1681
<400> 3986
   atccttggac aggatttat 19
<210> 3987
   <211> 24
   <212> DNA
   <213> Homo sapiens
<220>
   <223> mature of SEQ ID 1681
<400> 3987
   atagatcctg ttatgtggaa agag 24
<210> 3991
   <211> 21
   <212> DNA
   <213> Homo sapiens
<220>
   <223> mature of SEQ ID 1686
<400> 3991
   ataaaaagtt taccacaatc a 21
<210> 3992
   <211> 21
   <212> DNA
   <213> Homo sapiens
<220>
   <223> mature of SEQ ID 1686
<400> 3992
   aagtaccttt aaataaaatc a 21
<210> 3993
   <211> 29
   <212> DNA
   <213> Homo sapiens
<220>
   <223> mature of SEQ ID 1688
<400> 3993
   gctatggatt ggcatggggt ctgcagctc 29
<210> 3994
   <211> 26
   <212> DNA
   <213> Homo sapiens
<220>
   <223> mature of SEQ ID 1688
<400> 3994
   cacagacagc tgcaattcat gggcga 26
<210> 4001
   <211> 21
   <212> DNA
   <213> Homo sapiens
<220>
   <223> mature of SEQ ID 1693
<400> 4001
   caggcattgc ctggctcaga g 21
<210> 4002
   <211> 31
   <212> DNA
   <213> Homo sapiens
<220>
   <223> mature of SEQ ID 1693
<400> 4002
   gttaacaaag tgggctgagg ggtgaagcct g 31
<210> 4003
   <211> 29
   <212> DNA
   <213> Homo sapiens
<220>
   <223> mature of SEQ ID 1693
<400> 4003
   ctgagttaac aaagtgggct gaggggtga 29
<210> 4004
   <211> 29
   <212> DNA
   <213> Homo sapiens
<220>
   <223> mature of SEQ ID 1611
<400> 4004
   aagtgaaagg gacacaatcg attactacg 29
<210> 4181
   <211> 86
   <212> DNA
   <213> Homo sapiens
<400> 4181
   gttcctaacg agctgggtgg ggcaggcaga caatggagcc ctcccagcct gcctggctgc 60
   cccctgcctg gagctccccg aggaac 86
<210> 4293
   <211> 76
   <212> DNA
   <213> Homo sapiens
<400> 4293
   gggtggagag agggggtaga aaggaagagg gacatatggg agcctcttcc cccatgcccg 60
   aaagcttctc ccattt 76
<210> 4451
   <211> 60
   <212> DNA
   <213> Homo sapiens
<400> 4451
   gtcaccaggg taccatttgg taaattgctg ctgcaaagca gtgaatagta ccctggtggc 60
<210> 4492
   <211> 85
   <212> DNA
   <213> Homo sapiens
<400> 4492
   cctcgcctgc tcctcctgtc cccactcctc cttgccccac ccaatttggg ggctggagag 60
   gggacaggat gggccaagga caagg 85
<210> 4538
   <211> 75
   <212> DNA
   <213> Homo sapiens
<400> 4538
   ggctcaggaa cagcagcagc ccgggccgcg ccggcaggaa gcgaggccca tgttgctgct 60
   gttccctggc gcgcc 75
<210> 4567
   <211> 86
   <212> DNA
   <213> Homo sapiens
<400> 4567
   gggacagggg agaaagatgt gggtggagtc tgggaggcag atgcgagaat cagatcacaa 60
   tgacgagaag ccctttcctt tgtccc 86
<210> 4601
   <211> 85
   <212> DNA
   <213> Homo sapiens
<400> 4601
   gcacttgata ctaaccgagc tgtctatatc ctagccttgt gtcaaaggct tgtgatgtgt 60
   tgtgatagct gggtttaaga agtgc 85
<210> 4620
   <211> 66
   <212> DNA
   <213> Homo sapiens
<400> 4620
   ttctgagata tgatctgttg gattctctac taccaaagtg ggggaaaaac aggtggtact 60
   ccagaa 66
<210> 4713
   <211> 88
   <212> DNA
   <213> Homo sapiens
<400> 4713
   gccctggccc ctgtggggaa caggtcttgg ggtcatgaag ggctggaaac aagtgacccc 60
   agggcctgtc tcccttcttg ggtagggc 88
<210> 4724
   <211> 77
   <212> DNA
   <213> Homo sapiens
<400> 4724
   gccggctcca acccagaggc ccggaatagg cgcggagtta taaatagtgc cacccgcagg 60
   tgttgggggg agtcggc 77
<210> 4932
   <211> 89
   <212> DNA
   <213> Homo sapiens
<400> 4932 60
<210> 4936
   <211> 62
   <212> DNA
   <213> Homo sapiens
<400> 4936
   aggagaaaat aaatgtttgc tgaataaatt acgtaatgaa tatagataat taatgtgcta 60
   at 62
<210> 5044
   <211> 68
   <212> DNA
   <213> Homo sapiens
<400> 5044
   gatcaacagg cttattagaa gaatgaacta aggtgtctac catgattatt tttctaagct 60
   ggttggtt 68
<210> 5182
   <211> 83
   <212> DNA
   <213> Homo sapiens
<400> 5182
   gtgggatgat gtcacagaca ttaattgggt ggtgtctcaa gcagatatct atggtcatct 60
   ttctctgtgt ctgtctctcc cac 83
<210> 5388
   <211> 61
   <212> DNA
   <213> Homo sapiens
<400> 5388
   acacacacat atacatatgt atatatgtga gagatacaca accgtgtgtg tgtgtgtgtg 60
   t 61
<210> 5420
   <211> 84
   <212> DNA
   <213> Homo sapiens
<400> 5420
   tctccagata agaagtggct tggaactccc attgaggaga tgagaagaat gcctcagtgt 60
   gggatccggc tgcctctctt gaga 84
<210> 5480
   <211> 81
   <212> DNA
   <213> Homo sapiens
<400> 5480
   tttttgctac gctcattatc tttgtattta aatgttttag gcaacctaag aacaaatgta 60
   aaagtaaaga tgcagtaaaa g 81
<210> 5535
   <211> 71
   <212> DNA
   <213> Homo sapiens
<400> 5535
   aggaagggaa tggactggga gggtttcttt tcctgatgga aagcctattt ttcttattgt 60
   gttccttttc t 71
<210> 5745
   <211> 69
   <212> DNA
   <213> Homo sapiens
<400> 5745
   tcacaataac tgtgcatccc ttcagagtaa tgtcaacatt ttctggaatg tcgacagtct 60
   gattgctga 69
<210> 5789
   <211> 21
   <212> DNA
   <213> Homo sapiens
<220>
   <223> mature of SEQ ID 4293
<400> 5789
   agggggtaga aaggaagagg g 21
<210> 6008
   <211> 32
   <212> DNA
   <213> Homo sapiens
<220>
   <223> mature of SEQ ID 4451
<400> 6008
   gtcaccaggg taccatttgg taaattgctg ct 32
<210> 6065
   <211> 32
   <212> DNA
   <213> Homo sapiens
<220>
   <223> mature of SEQ ID 4601
<400> 6065
   gcacttgata ctaaccgagc tgtctatatc ct 32
<210> 6224
   <211> 32
   <212> DNA
   <213> Homo sapiens
<220>
   <223> mature of SEQ ID 5745
<400> 6224
   tcacaataac tgtgcatccc ttcagagtaa tg 32
<210> 6285
   <211> 17
   <212> DNA
   <213> Homo sapiens
<220>
   <223> mature of SEQ ID 4724
<400> 6285
   gtgccacccg caggtgt 17
<210> 6301
   <211> 35
   <212> DNA
   <213> Homo sapiens
<220>
   <223> mature of SEQ ID 4932
<400> 6301
   aaacaggacc atgaccattc acaacggcat gttct 35
<210> 6302
   <211> 32
   <212> DNA
   <213> Homo sapiens
<220>
   <223> mature of SEQ ID 4932
<400> 6302
   tgggagaaaa caggaccatg accattcaca ac 32
<210> 6463
   <211> 18
   <212> DNA
   <213> Homo sapiens
<220>
   <223> mature of SEQ ID 5420
<400> 6463
   atgcctcagt gtgggatc 18
<210> 6510
   <211> 30
   <212> DNA
   <213> Homo sapiens
<220>
   <223> mature of SEQ ID 5044
<400> 6510
   aacaggctta ttagaagaat gaactaaggt 30
<210> 6538
   <211> 29
   <212> DNA
   <213> Homo sapiens
<220>
   <223> mature of SEQ ID 5480
<400> 6538
   acctaagaac aaatgtaaaa gtaaagatg 29
<210> 6539
   <211> 31
   <212> DNA
   <213> Homo sapiens
<220>
   <223> mature of SEQ ID 5480
<400> 6539
   aagaacaaat gtaaaagtaa agatgcagta a 31
<210> 6606
   <211> 23
   <212> DNA
   <213> Homo sapiens
<220>
   <223> mature of SEQ ID 4492
<400> 6606
   cctcgcctgc tcctcctgtc ccc 23
<210> 7433
   <211> 19
   <212> DNA
   <213> Homo sapiens
<220>
   <223> mature of SEQ ID 4620
<400> 7433
   aaaacaggtg gtactccag 19
<210> 7480
   <211> 22
   <212> DNA
   <213> Homo sapiens
<220>
   <223> mature of SEQ ID 4713
<400> 7480
   ggggaacagg tcttggggtc at 22
<210> 7510
   <211> 21
   <212> DNA
   <213> Homo sapiens
<220>
   <223> mature of SEQ ID 5535
<400> 7510
   gactgggagg gtttcttttc c 21

## Description

### CROSS REFERENCE TO RELATED APPLICATIONS

This application claims the benefit of European Patent Application No. 05292359.6, filed November 8, 2005.

### REFERENCE TO A "SEQUENCE LISTING", A TABLE, OR A COMPUTER PROGRAM LISTING APPENDIX SUBMITTED ON A COMPACT DISK.

The Sequence Listing containing 7803 nucleic acid sequences accompanying the present patent application is provided on a CD and is available from a database maintained by the International Bureau of WIPO, and is hereby incorporated by reference in its entirety for all purposes as an integrated part of this patent application.

### FIELD OF THE INVENTION

This invention generally relates to bioinformatics, and more particularly to the identification of RNA sequences, particularly microRNA precursor candidates.

### BACKGROUND OF THE INVENTION

The development of multi-cellular and multi-tissue organisms requires specific and coordinated control involving complex regulatory mechanisms that target every aspect of gene expression. NpcRNA (non protein coding ribonucleic acids) such as tRNA and rRNA are known essential regulators of gene expression and protein synthesis. MicroRNA (miRNA) constitute a vast family of non-protein coding RNA that play critical regulatory functions in numerous biological processes and hold the key of many important aspects of human health. For example, microRNAs orchestrate many aspects of cell, tissue and organism development. They also play key roles in the regulation of fundamental cellular mechanisms (Brennecke J and al., 3003; Chen CZ, 2004; Esau C and al., 2004; Yekta S. and al., 2004). Most microRNAs are submitted to two types of regulations: a temporal regulation which is a function of different cell growth stages, differentiation and development, and a spatial regulation. Indeed, they show specific expression patterns in each cellular or tissue type (Reinhart BJ., 2000).

Besides the detection and profiling of miRNA genes, deciphering their function is also particularly challenging. With very few exceptions, their specific functions are unknown. MicroRNAs do not act alone, but act in combination to cooperatively regulate a definite biological process. This mode of action is the one generally referred to when talking about the so-called "microRNA code". The miRNAs that belong to a "functional family" need to be identified, and their "true" mRNA targets discerned from amongst thousands of possibilities. Being timely and spatially regulated, their discovery requires high-throughput methods, like microarrays, in order to find novel miRNAs, which are specifically expressed in particular cell-types, tissues and under different physiological conditions.

Additionally, the complete set of microRNA which are expressed in different organisms is unknown. Thus, one main challenge today consists in identifying the complete set of microRNAs. It appears that only the tip of the iceberg of the microRNA world is known. Contrary to what was believed for a long time, recent studies show that a great number of microRNA remain to be identified.

Today, it is a major challenge to identify novel microRNAs, and there is a growing interest of life science industries in microRNA. Indeed, the exhaustive knowledge of microRNA will make it possible to identify mechanisms of regulation, that will help to better understand the gene and protein regulation networks, and to discover new molecular biomarkers associated with cell development and many different diseases.

The principal method used until now to discover new microRNAs consists in their detection and their quantification by biological experiments using approaches and strategies of molecular biology, such as RNA extraction, amplification, cloning, gel electrophoresis, hybridization, and sequencing. However, the large majority of the microRNA are controlled in a space-time dependent manner, where they are mostly only slightly expressed. Thus, many escape detection by these conventional biochemical and genetic approaches.

Bioinformatic approaches have emerged as powerful alternatives. Predictive bioinformatics have provided many discoveries, obtained through combination of experimental and computational approaches. Nevertheless, these approaches require the development of new strategies. The identification of microRNA is indeed especially complex because of their very short size and their great variability in sequence. There is no evident specific sequence "signature" like for protein coding genes. The nature of their structural constraint is mainly defined by the complementarities with their mRNA targets, and not by any evident structural constraints within the mature miRNA iltself. Thus, most of the computational methods are based on a search for their precursors, which fold into a variable and irregular stem-loop structures (hairpin secondary structures).

A first work in 2003 (Lim and al., 2003) led the authors to estimate the number of microRNA in the human genome to be 255. Actually, current works reveal that the number of microRNAs was considerably underestimated. Indeed, to date, 474 microRNAs are already referenced in the public data base Rfam, while other scientists (Grad, 2003; Krichevsky, 2003; Bentwich and al., 2005) suggests that the number of microRNA within the human genome could be at least 800.

In the human genome, only a few of the known microRNA are fully characterized. Thus, for the largest part of them one cannot associate a function: which gene, which messenger RNA or which proteins they control. The development of a systematic approach, associating bioinformatics predictions with experimental validations makes it possible to increase the known set of microRNA molecules. Their exploitation opens new ways to discover new key regulatory and functional molecules directly implied in pathologies and to conceive a new classes of therapeutic tools (for example: biomarkers, diagnostic/prognostic molecules and drugs).

However, new computational methods need to be developed to overcome the limitations of today's standard bioinformatics and laboratory tools.

### BRIEF SUMMARY OF THE INVENTION

This invention provides methods for prediction and identification of microRNA precursors (pre-microRNA) and microRNA molecules using data processing programs and databases.

The method of the present invention brings new and effective solutions to identify new microRNA, thus offering an effective and relevant solution to allow the discovery of new microRNA, undetectable with current methods. The method of the present invention was validated by an approach using microarrays, which enabled the confirmation of the expression of predicted microRNA in different tissues. Embodiments of the method of the present invention makes it possible to eliminate most of the false positive occurrences observed with other methods, and to increase the sensitivity in the search of new microRNA.

Accordingly, in a first aspect, the invention provides a computer-implemented method of identifying microRNA precursor candidates in non-coding and coding regions of a genome. In some embodiments, the methods comprise:
receiving at least a first and a second genome from two different species, each containing a plurality of sequences including highly repetitive DNA;
masking a first set of highly repetitive DNA sequences in the first genome, wherein the first set includes SINE and LINE interspersed repeat sequences, but does not include at least 75% of other interspersed repeat sequences;
masking a second set of highly repetitive DNA sequences in the second genome, wherein the second set includes SINE and LINE interspersed repeat sequences, but does not include at least 75% of the other interspersed repeat sequences;
comparing the masked first genome to the masked second genome to determine pairs of sequences that are homologous between the two genomes;
creating a list of microRNA precursor candidates from the homologous pairs; and
analyzing the list of candidates to:
   eliminate sequences having less than 60 nucleotides; and
   identify sequences that have a stem-loop secondary structure with a 5' strand stem having from 20 to 45 nucleotides and with a 3' strand stem having from 20 to 45 nucleotides; and
   remove, from the list, sequences not having said stem-loop secondary structure.

In one embodiment, the other interspersed repeat sequences include processed pseudogenes, retrotranscripts, DNA transposons, and retrovirus retrotransposons.

In one embodiment, the methods further comprise the steps of:
creating a plurality of assemblies, each created from at least one pair of homologous sequences, wherein at least one assembly is created from a first group of at least two pairs of homologous sequences, wherein each pair of the first group has one sequence in common;
clustering the assemblies into a plurality of clusters, wherein each assembly of a cluster shares at least one sequence with at least one other assembly of that cluster, wherein a cluster includes one or more assemblies;
analyzing the properties of each cluster to eliminate sequences from the list of candidates.

In one embodiment, the methods further comprise the steps of:
analyzing the properties of each cluster to determine new sequences from the clusters to be added to the first set and second set of sequences that are masked, further comprising:
   adding the new sequences to the first set and to the second set; and
   repeating masking with the first set and to the second set, comparing the masked genomes, creating a plurality of assemblies, and clustering the assemblies.

In some embodiments, analyzing the properties of a cluster to determine new sequences comprises:
determining the number of sequences of a cluster, and
if the number of sequences of a cluster is greater than a predetermined number, selecting the sequences of that cluster to be added to the first set and to the second set.

. In some embodiments, at least one assembly is created from a second group of at least two pairs of homologous sequences, wherein the two sequences from the first genome of the second group overlap to form a first sequence, wherein the two sequences from the first genome of the second group overlap to form a second sequence, wherein the size variation between the first sequence and the second sequence is less than 10%, and wherein the alignment between the first sequence and the second sequence shows more than 85% sequence similarity.

In some embodiments, at least one assembly is created from a second group of at least two pairs of homologous sequences, wherein the two sequences from the first genome of the second group are consecutive and form a first sequence containing an intervening sequence, wherein the two sequences from the first genome of the second group are consecutive and form a second sequence containing an intervening sequence, wherein the size variation between the first sequence and the second sequence is less than 10%, and wherein the alignment between the first sequence and the second sequence shows more than 90% sequence similarity.

In some embodiment, analyzing the properties of a cluster includes:
if a cluster has more than a predetermined number of sequences, eliminating the sequences of that cluster from the list of candidates.

In some embodiments, the predetermined number is four. In some embodiments, the predetermined number is three.

In some embodiments, analyzing the properties of the cluster further includes:
flagging sequences that correspond to a coding gene as 'coding';
eliminating all of the sequences of the cluster if one of the sequences is flagged as 'coding'; and
eliminating the sequences of an assembly if the assembly is not identical by at least 85% within a minimal window of 60 nucleotides.

In some embodiments, analyzing comprises the steps of:
eliminating a pair of homologous sequences if a secondary structure resulting from a folding of the sequences does not satisfy each of a first set of criteria including:
   a number of nucleotides in a pre-miRNA stem-loop;
   a terminal hairpin being of a certain length;
   a percentage similarity of the sequences between the two genomes; and
   a Z score less than a specified amount; and
eliminating a pair of homologous sequences if a secondary structure resulting from a folding of the sequences does not satisfy a predetermined number of a second set of criteria including:
   a minimal free energy (MFE) of less than a specified amount;
   a GC content being within a certain percent range;
   a base-paring number being within a certain percent range; and
   perfect conservation of a sequence of a specified length along arms of the stem-loop.

In some embodiments, the first set and the second set of criteria have the following values:
the pre-miRNA stem-loop is between 60 to 120 nucleotides;
the percentage similarity of the sequences between the two genomes is at least 85 %;
at least one 17nt-long sequence is perfectly conserved along the arms of the stemloop;
the terminal hairpin-loop is between 4 and 15 nucleotides;
the GC content is from 30 % to 51 %;
the base-pairing number is between 30 and 40 %;
the MFE is lower than -25 kcal/mol; and
the Z score is less than 0.06.

In some embodiments, analyzing further comprises:
when a sequence has a secondary structure satisfying the first set of criteria and a predetermined number of the second set of criteria, extracting exact positions of the 5' strand start and exact positions of the 3' strand end to make a new sequence;
folding the new sequence to from a new secondary structure; and
parsing the secondary structure of the new structure to determine if the secondary structure satisfies the first set of criteria and a predetermined number of the second set of criteria.

In another aspect, the invention provides a computer-implemented method of identifying non coding RNA candidates. In some embodiments, the methods comprise:
receiving at least a first and a second genome from two different species, each containing a plurality of sequences including highly repetitive DNA;
masking a first set of highly repetitive DNA sequences in the first genome, wherein the first set includes SINE and LINE interspersed repeat sequences, but does not include at least 75% of other interspersed repeat sequences;
masking a second set of highly repetitive DNA sequences in the second genome, wherein the second set includes SINE and LINE interspersed repeat sequences, but does not include at least 75% of the other interspersed repeat sequences;
comparing the masked first genome to the masked second genome to determine pairs of sequences that are homologous between the two genomes;
creating a list of non coding RNA candidates from the homologous pairs; creating a plurality of assemblies, each created from at least one pair of homologous sequences, wherein at least one assembly is created from a first group of at least two pairs of homologous sequences, wherein cach pair of the first group has one sequence in common.
clustering the assemblies into a plurality of clusters, wherein each assembly ofa cluster shares at least one sequence with at least one other assembly of that cluster, wherein a cluster includes one or more assemblies;
analyzing the properties of each cluster to eliminate sequences from the list of candidates;
analyzing the list of candidates to:
eliminate sequences having less than 60 nucleotides; and
identify sequences that have a stem-loop secondary structure with a 5' strand stem having from 20 to 45 nucleotides and with a 3' strand stem having from 20 to 45 nucleotides;
remove, from the list, sequences not having said stem-loop secondary structure;
eliminating a pair of homologous sequences if the secondary structure does not satisfy each of a first set of criteria; and
eliminating a pair of homologous sequences if the secondary structure does not satisfy a predetermined number of a second set of criteria;

### DEFINITIONS

The term processed "pseudogene", as used herein refers to non-functional DNA sequences created by the reverse transcription of mRNA into cDNA with subsequent reintegration into the genome. Typically, processed pseudogenes fall into three categories. 1) those that are a complete copy of the mRNA transcribed from the functional gene; 2) those that arc only a partial copy of the mRNA transcribed from the functional gene; and 3) those that contain sequences in addition to sequences expected to be present in the mRNA transcribed from the functional gene.

General structural characteristics of processed pseudogenes include: 1) the complete lack of intervening sequences found in the functional gene (e.g. introns sequences); 2) a poly A tract located at the 3'-end of the sequence; 3) direct repeats flanking the pseudogene sequence: and 4) in most cases the pseudogene is located on a different chromosome from that of the functional gene. In identifying the processed pseudogenes, the computer compares the query sequence against a database comprised of genomic sequences which encode for the functional gene.

The term "DNA transposon" or "Class II transposons", as used herein refers to DNA sequences belonging to a class of mobile elements that are capable of transposing from one site in the genome to a new site in the genome. DNA transposons transpose directly through a DNA intermediate, rather than through an RNA intermediate. A defining feature of a DNA transposon is a sequence encoding for a protein required for transposition, having at least 95% sequence identity, or sequence identity based on the default setting, whichever is lower, to the transposase of bacterial insertional sequence elements. The sequence encoding the transposase-like protein is flanked on each end by a short (*i.e*., 4-15 bp) inverted repeat (IR) sequence, which are required for transposition. Each inverted repeat is flanked on its outside by a short (4-15 bp) direct repeat (DR) sequence (i.e. the 5'DR is 5' to the 5'IR): Another feature of DNA transposons is that they may contain introns as part of the intervening sequence between the inverted repeats, unlike retrotransposons.

The computer identifies DNA transposons by identifying sequences that contain the following elements in the 5' to 3' direction. 5'-DR-IR-(coding sequence for protein having at least 95% sequence identity to consensus sequence for known transposases)-IR-DR-3'. As mentioned above introns may or may not be present in the coding sequence and these will be identified by comparing the coding sequence with the genomic sequence of the functional gene.

The term "retrotransposons" or "Class I transposons", as used herein refer to mobile DNA elements that transpose through an RNA intermediate utilizing reverse transcriptase activity. Retrotransposons are subdivided into two classes. The first class are retrovirus retrotransposons (synonymous with LTR transposons) because they exhibit many similarities to the genomes of retroviruses. For example, they contain long terminal repeats (LTR) which are typically about 100bp to about 1kb in length that flank the 5' and 3' ends of the protein coding sequence of the LTR transposon. The computer identifies retrovirus retrotransposons by comparing the query sequence against a consensus LTR sequence derived from a database of known LTR sequences.

The second class of retrotransposons are non-retrovirus retrotransposons (synonymous with non-LTR retrotransposons) because they lack the characteristic LTRs. Non-LTR retrotransposons can be further subdivided into at least two subclasses, the most abundant being long interspersed elements (LINE) and short interspersed elements (SINE).

The term "LINE" as used herein refers to long (typically greater than 5kb) DNA sequences that represent reverse transcribed RNA molecules that were originally transcribed by RNA polymerase II. A consensus LINE element is characterized as having the following structural features. First, LINE elements are typically greater than 5kb in size and have at least two open reading frames. Typically, ORF1 is located near the 5' end of the top strand following a region containing multiple stop codons in all possible reading frames. ORF2 is located in the top strand 3' to ORF1, and encodes a protein having at least 95% sequence identity, or sequence identity of the default settings, whichever is lower, to the reverse transcriptases of retroviruses and viral retrotransposons. Flanking the 3'-end of ORF2 is an AT rich region. The entire LINE element is flanked by direct repeats at the 3' and 5' ends.

The computer searches for LINE elements by identifying regions greater than 5kb flanked by direct repeats. The intervening sequence, as discussed above will contain a region having multiple stop codons in all frames, followed by one to four open reading frames, one of which will share 95% sequence identity, or sequence identity of the default settings, whichever is lower, with proteins similar to reverse transcriptases of retroviruses and viral retrotransposons. The 3' most ORF is flanked on its 3'-end by an AT rich region.

The tem "SINE" as used herein refers to short DNA sequences (about 500bp or less) that represent reverse transcribed RNA molecules that were originally transcribed by RNA polymerase III into tRNA, rRNA and other small nuclear RNAs. SINES do not encode a functional protein having reverse transcriptase activity. The most common SINES are Alu elements which are typically about 300 bp and do not contain any coding sequences and can be recognized by the restriction enzyme AluL

Most SINE elements identified to date share the following common elements. A 5' tRNA, rRNA, or other small nuclear RNA related region (including A and B boxes for internal RNA polymerase III promoters), a tRNA, rRNA, or other small nuclear RNA unrelated region, and a 3' AT rich region, flanked on both the 3' and 5' ends by direct repeats.

The computer identifies SINE sequences by identifying sequences about 500 bp or less flanked by direct repeats. These sequences will be compared to a consensus sequences for tRNA, rRNA or other small nuclear RNAs compiled from a database of known RNA sequences. Sequences meeting this criteria will then be screened for an AT rich region 5' to the direct repeat located at the 3' end.

The term "retrotranscript" as used herein refers to the RNA intermediate of the retrotransposon.

The term "homologous pair" as used herein refers to any pair of sequences that have been identified as sharing ancestry. Any method and/or criteria may be used. For example, a percentage of similarity or identity may be used. In such an embodiment, no specific percentage is required, but simply that a percentage has been specified. In some embodiments, a homologous pair shares at least 80%, 85%, 90%, 93%, 95%, 97%, 99% or 100% sequence identity, In another embodiment, a homologous pair may be identified though an associative property between two other pairs, such as is done in clustering emboidments of the present invention.

As used interchangeably herein, the terms "nucleic acid", "oligonucleotides", and "polynucleotides" include RNA, DNA, or RNA/DNA hybrid sequences of more than one nucleotide in either single-stranded or double-stranded form. The term "nucleotide" as used herein as an adjective to describe molecules comprising RNA, DNA, or RNA/DNA hybrid sequences of any length. The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. Although the term "nucleotide" is also used herein to encompass "modified nucleotides" which comprise at least one modifications (a) an alternative linking group, (b) an analogous form of purine, (c) an analogous form of pyrimidine, or (d) an analogous sugar- However, the polynucleotides of the invention are preferably comprised of greater than 50 % conventional deoxyribose nucleotides, and most preferably greater than 90 % conventional deoxyribose nucleotides. The nucleic acids of the invention may be prepared by any known method, including synthetic, recombinant, ex vivo generation, or a combination thereof, as well as utilizing any purification methods known in the art.

As used herein, the term "isolated" requires that the material be removed from its original environment (e.g., the natural environment if it is naturally occurring). For example, a naturally-occurring nucleic acid in a living animal is not isolated, but the same nucleic acid, separated from some or all of the coexisting materials in the natural system, is isolated. Such nucleic acid could be part of a vector and/or such nucleic acid could be part of a composition, and still be isolated in that the vector or composition is not part of its natural environment.

The phrases "selectively (or specifically) hybridizes to" or "selectively (or specifically) capture" interchangeably refer to the binding, duplexing, or hybridizing of a molecule only to a particular nucleotide sequence under stringent hybridization conditions when that sequence is present in a complex mixture (e.g., total cellular or library DNA or RNA). Typically, specific or selective binding will be at least twice background signal or noise and more typically more than 10 to 100 times background.

The terms "complementary" or "complement thereof" are used herein to refer to the sequences of a nucleic acid which are capable of forming Watson & Crick base pairing with another specified nucleic acid throughout the entirety of the complementary region. This term is applied to pairs of nucleic acid based solely upon their sequences and not any particular set of conditions under which the two polynucleotides would actually bind.

As used herein, the terms "complementary" or "complement" interchangeably refer to 100 % antisense sequence identity.

The terms "sufficiently complementary" or "substantially complementary" interchangeably refer to antisense sequence identity between two nucleic acid sequences sufficient for hybridization under stringent hybridization conditions. Two nucleic acid sequences that are sufficiently complementary share at least 93 % antisense sequence identity (i.e., 1 mismatched nucleotide over a length of 15 nucleotides). In some embodiments, sufficiently complementary nucleic acid sequences share at least 94 %, 95 %, 96 %, 97 %, 98 % or 99 % antisense sequence identity.

As used herein, the term "hybridizes to" is intended to describe conditions for moderate stringency or high stringency hybridization, preferably where the hybridization and washing conditions permit nucleotide sequences at least 60 % identity to each other to remain hybridized to each other. Preferably, the conditions are such that sequences at least about 80 %, 85 %, 90 %, 93 %, 95 %, 97 %, 98 %, 99 % sequence identity to each other typically remain hybridized to each other. Stringent conditions are known to those skilled in the art and can be found in Ausubel, Current Protocols in Molecular Biology, 2006, *supra.* A preferred, non-limiting example of stringent hybridization conditions are as follows: the hybridization step is realized at 65°C in the presence of 6 x SSC buffer, 5 x Denhardt's solution, 0.5 % SDS and 100 µg/ml of salmon sperm DNA. The hybridization step is followed by four washing steps:
- two washings during 5 min, preferably at 65°C in a 2 x SSC and 0.1 %SDS buffer,
- one washing during 30 min, preferably at 65°C in a 2 x SSC and 0.1 % SDS butter;
- one washing during 10 min, preferably at 65°C in a 0.1 x SSC and 0.1 %SDS buffer,
these hybridization conditions being suitable for a nucleic acid molecule of about 20 nucleotides in length. It will be appreciated that the hybridization conditions described above are to be adapted according to the length of the desired nucleic acid, following techniques well known to the one skilled in the art, for example be adapted according to the teachings disclosed in Hames B.D. and Higgins S.J. (1985) Nucleic Acid Hybridization: A Practical Approach. Hames and Higgins Ed., IRL Press, Oxford; and Current Protocols in Molecular Biolog (supra).. Preferably, an isolated nucleic acid molecule of the invention that hybridizes under stringent conditions to a sequence of SEQ ID No 1 or 2 or 3 corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

As used herein, "percent sequence identity" between two nucleic acid sequences is intended to indicate a percentage of nucleotides between the two sequences to be compared, obtained after the best alignment (optimum alignment), this percentage being purely statistical and the differences between the two sequences being distributed randomly and over their entire length. The comparisons of sequences between two nucleic acid are traditionally carried out by comparing these sequences after having aligned them in an optimum manner, said comparison can be carried out, in addition to manually, by means of the local homology algorithm of Smith and Waterman (1981) [Ad. App. Math. 2:482], by means of the local homology algorithm of Neddleman and Wunsch (1970) [J. Mol. Biol. 48: 443], by means of the similarity search method of Pearson and Lipman (1988) [Proc. Natl. Acad. Sci. USA 85:2444), by means of computer software using these algorithms (GAP, BESTFIT, FASTA and TFASTA in the Wisconsin Genetics Software Package, Genetics Computer Group, 575 Science Dr., Madison, WI, or else by BLAST N or BLAST P comparison software).

The percentage of identity between two nucleic acid sequences is determined by comparing these two sequences aligned in an optimum manner and in which the nucleic acid to be compared can comprise additions or deletions with respect to the reference sequence for an optimum alignment between these two sequences. The percentage of identity is calculated by determining the number of identical positions for which the nucleotide is identical between the two sequences, by dividing this number of identical positions by the total number of positions in the comparison window and by multiplying the result obtained by 100 in order to obtain the percentage of identity between these two sequences.

For example, one can use the BLAST program, "BLAST 2 sequences" (Tatusova et al, "Blast 2 sequences - a new tool for comparing protein and nucleotide sequences", FEMS Microbiol Lett. 174:247-250) available on the site http://ww.ncbi.nlm.nih-gov/ gorf/bl2.html, the parameters used being those given by default (in particular for the parameters "open gap penalty": 5, and "extension gap penalty": 2; the matrix chosen being, for example, the matrix "BLOSUM 62" proposed by the program), the percentage of identity between the two sequences to be compared being calculated directly by the program.

By a nucleic sequence having at least 80 %, 85 %, 90 %, 91 %, 92 %, 93 %, 94 %, 95 %, 96 %, 97 %, 98 %, 99 % sequence identity with a reference nucleic acid sequence, those having, with respect to the reference sequence, certain modifications, for example, a deletion, addition or substitution of at least one nucleotide, a truncation or an elongation. In the case of a substitution, it may be one or more consecutive or nonconsecutive nucleotide(s).

General or current methods/protocols in molecular biology can be particularly found in the following references:
a) Sambrook and Russell, Molecular Cloning: A Laboratory Manual, 2001, Cold Spring Harbor Laboratory Press.
b) Bowtell and Sambrook, DNA Microarrays: A Molecular Cloning Manual, 2003, Cold Spring Harbor Laboratory Press.
c) Ausubel, et al., Current Protocols in Molecular Biology, 1987-2006, John Wiley Interscience.
d) Stirling and Bartlett, PCR Protocols, 2003, Humana Press.

### BRIEF DESCRIPTION OF THE DRAWINGS

Figure 1 illustrates a method of identifying microRNA precursor candidates in non-coding and coding regions of a genome according to an embodiment of the present invention.

Figure 2A illustrates an assembling process of creating an "assembly" by filtering according to one embodiment of the present invention.

Figure 2B illustrates another assembling process of creating an "assembly" by filtering according to one embodiment of the present invention.

Figure 2C illustrates an assembling process of creating an "assembly" by optimization according to one embodiment of the present invention.

Figure 3 illustrates a cluster created according to an embodiment of the present invention.

Figure 4 illustrates a method of clustering assemblies according to an embodiment of the present invention.

Figure 5 illustrates a cluster created according to an embodiment of the present invention.

Figure 6 illustrates a method of annotating the sequences of assemblies as containing coding or non-coding DNA according to an embodiment of the present invention.

Figure 7 illustrates a method of eliminating sequences of assemblies and clusters according to an embodiment of the present invention.

Figure 8 illustrates a secondary structure of an exemplary sequence according to an embodiment of the present invention.

Figure 9 illustrates a table showing the criteria and the decision of whether a sequence is a microRNA precursor candidates according to an embodiment of the present invention.

Figure 10 illustrates secondary structures of select sequences according to one embodiment of the present invention.

### DETAILED DESCRIPTION

This invention provides methods of identifying potential non-coding RNA, particularly microRNA precursor (pre-miRNA) candidates. In one embodiment, pre-miRNA are searched in homologous sequence pairs obtained from comparative genomics. In one aspect, to find more pre-miRNA, a different set of sequences are masked from the genomes than which is normally done. In some embodiments, the sequence pairs may be combined into assemblies and clustered to find new homologous relationships, which through analysis provide new techniques for discovering pre-miRNA. In another aspect, specific combinations of criteria and folding mechanism may be further used to determine pre-miRNA. Although, embodiments are directed to methods of finding microRNA precursor candidates, one skilled in the art will appreciate that embodiments may be used to find other non-coding RNA candidates.

### I. INTRODUCTION

NpcRNA (non protein coding ribonucleic acids) such as tRNA and rRNA are known essentials regulators of gene expression and protein synthesis. MicroRNA constitute a vast family of non-protein coding RNA. These microRNA are very short in length. In the human genome their size generally varies between 17 nucleotides (for example: miR-138 and miR-496) and 25 nucleotides (for example miR-519a-1, miR519a-2). With very few exceptions, their functions are unknown.

The microRNA (or miRNA) can be processed in two different ways. In the first way of biosynthesis, the microRNA is encoded by its endogenous gene, and is transcribed as a long primary precursor called pri-microRNA (or pri-miRNA) (review Nelson et al 2003, Bartel 2004). In mammals, this pri-microRNA is cleaved by the nuclease Drosha (Lee and al., 2003) to give a precursor of approximately 60-120 nucleotides length, which is called the pre-microRNA (or pre-miRNA). This precursor folds into a short and irregular stem-loop secondary structure. The pre-microRNA is then exported by the enzyme exportin-5 to the cytoplasm of the cells (Yi and al., 2003; Lundet and al. 2004; Bohnsack and al., 2004). The Dicer nuclease then cuts out the mature microRNA from the pre-microRNA (Hutvagner and al., 2001; Ketting and al., 2001; Knight and Bass 2001; Grishok and al., 2001). The mature microRNA are bound to a set of proteins, to the family of Argonaute proteins and the proteins Gemin3 and Gemin4 (most frequently in human cells), to form micro-Ribonucleoprotein complexes, called microRNP (or miRNP) (Mourelatos and al., 2002; Nelson and al., 2004).

In the second way of biosynthesis, the microRNA can be produced from introns of protein-coding genes. In this case, there is direct production of the pre-microRNA. Following maturation, steps remain common.

An essential characteristic of microRNAs are their antisense capability. The microRNAs function through more or less extended base-pairings with the 3'UTR region of specific messenger RNAs (Target Sequences of Recognition or TSR). According to the localization and the extent of complementarity (partial or complete), a microRNA will exert, either a repression of translation of this mRNA, or destruction of the messenger RNA target. In the latter case, an endonucleolytic cleavage occurs, which requires the intervention of an enzymatic complex called RISC (RNA-induced silencing complex).

MicroRNAs are involved in the cascades of events leading to cell differentiation. They control various metabolic pathways and physiological processes such as cell proliferation or cell apoptosis (Houbaviy HB., and al. 2003; He L and Hannon GJ., 2004; Kasashima K, and al., 2004; Xu P., and al., 2004; Bartel P., 2004). Thus, microRNAs orchestrate many aspects of cell, tissue and organism development. They play key roles in the regulation of fundamental cellular mechanisms (Brennecke J and al., 3003; Chen CZ, 2004; Esau C and al., 2004; Yekta S. and al., 2004).

Specifically, there is now an accumulation of evidence demonstrating the direct role of microRNA in the development of pathologies, such as cancers. In the adenocarcinoma of the lung, a reduction of the expression of the microRNA let-7 is associated with a significant shortening of post-operational survival, while an over-expression of this same microRNA in a cellular line (A549) of adenocarcinoma of the lung inhibits the growth of the cancerous cells in vitro (Takamizawa J and al., 2004).

A strong expression of the precursor of the microRNA-155/BIC in the lymphoma of Burkitt was observed (Metzler Mr. and al., 2003). Two microRNAs (miR-143 and miR-145), which are conserved between human and mouse genomes, show substantial reduction of accumulation in definite stages of colorectal cancers (Michael MZ., 2003).

Deletion and decrease of expression of the microRNA miR15 and miR16 occur in the majority (approximately 68 %) of leukemias of the "B cell chronic lymphocytic leukemia" type (Calin GA. and al., 2002; Calin GA. and al., 2004). These two microRNAs are localised on the chromosome 13q14, an area of 30-KB which is deleted in more than half of these leukemias. Also, it has been demonstrated that the microRNA mir-17-92 family is over-expressed in human lymphomas (He L, 2005). Moreover, an over-expression of the very same microRNAs results in a cancer in mice.

Recently, a direct link was established for the first time between microRNA and c-Myc, a proto-oncogene which encodes for a transcription factor and regulates cellular proliferation, cell growth and apoptosis (O' Donnel KA, 2005). The c-Myc simultaneously activates the transcription of the transcription factor E2F1 and the expression of 6 microRNAs, 2 of which (miR-17-5p and miR-20a) repress the expression of E2FI, thus allowing a fine control of the signals controlling cell proliferation.

MicroRNA are also involved in neurological and neurodegenerative diseases (Dostie, Z and al., 2003) and the brain expresses a great number of specific microRNAs (Kim J and al., 2004; Krichevsky AMNDT and al., 2004; Kubawara T and al., 2004); Miska EA, and aL, 2004; Sempere LF. and al., 2004).

The metabolic diseases also involve microRNAs. Recently, it was shown that a microRNA (miR-375), specific to the pancreatic small islet-specific cells, controls insulin secretion. Thus, miR-375 is a regulator of the secretion of insulin and could constitute a new pharmaceutical target for the treatment of diabetes (Poy MN, and al., 2004).

MicroRNAs act through networks of interactions. Higher organism requires two levels of programming: on the one hand specification of the functional component (primarily the proteome) and on the other hand the orchestration of the expression and the assembly of these components during the stages of differentiation and development. MicroRNA, which interact with mRNA and proteins, determine how, when and where genes must be expressed. These regulations take place in a coordinated network with one microRNA regulating the expression of several different genes post-transcriptionally and one gene being regulated by several different microRNA. These interactions are imbricated, function on several levels and allow a precise adjustment, in real time, of expression/regulation of a specific set of proteins in a cell. Playing a central role in mechanisms of regulation essential for the development and the integrity of the cellular and tissue organization of organism, microRNAs multiply the possibilities of regulation and challenge our vision of the networks of interactions between gene products (Mattick JS., 2003).

Biological experiments show that changes in this network architecture are as significant as variations in the proteome components for the determination of cells differences, but also certainly determinant for the differences between species and individuals. MicroRNA expression is sensitive to environmental parameters, and has a differential susceptibility for pathologies.

The implication of the microRNA in the mechanisms responsible for the gene activation/inactivation results in changes of the protein expression profiles and therefore allows the change of the cell status, that is development and differentiation within a physiological context. However, due to this capacity, aberrant microRNA expression can lead to major changes in protein expression profiles resulting in the development of specific pathologies.

It is now acquired that the microRNA, similar to transcription factors, act in a co-operative dose-dependent manner upon their mRNA targets. Experimental results show that specific gene expression profiles of different tissues can be explained by the "microRNA code" (Hobert O., 2004; Doench JG and Sharp Pa., 2004) which relates to the specific set of microRNA expressed within different tissues.

In a general way, a set of microRNA is necessary to control the same RNA. At the same time, a microRNA can have several different messenger RNA targets. This multiplicity of the targets and the co-operative aspect of the signals are essential features of the control of translation by microRNAs (Enright AJ and al., 2003; John B and aL, 2004).

MicroRNAs inhibit expression of protein-coding genes either without affecting the concentration of the targeted mRNA, or by decreasing its concentration (Lim LP and al., 2005). In vertebrates, 20 to 30 % of the protein coding genes are supposed to be controlled by microRNAs (Lewis LP and al., 2005; Krek A and al., 2005; Xie X et al., 2005).

### II. Methods of identifying MICRORNA precursor candidates

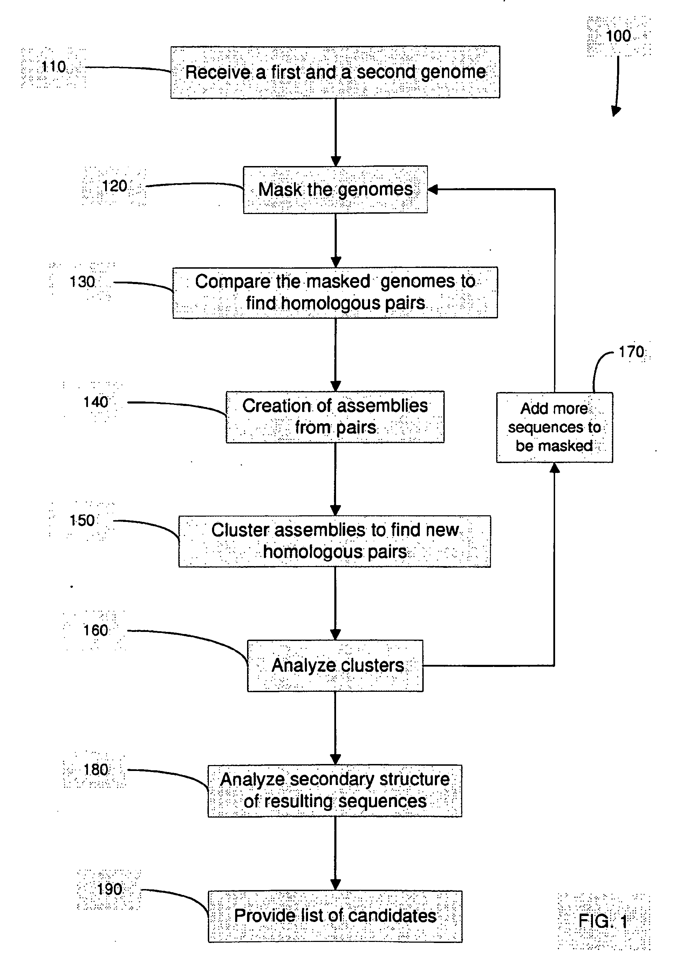

In a first aspect, the present invention is directed to a computer-implemented method for the identification of candidate microRNA precursor molecules (pre-microRNAs). Figure 1 illustrates a method 100 of identifying microRNA precursor candidates in non-coding and coding regions of a genome according to an embodiment of the present invention. These steps may be rearranged to be performed in a different order, and some steps may be removed. A general description of the method and these steps is initially provided. Subsequent sections provide greater detail of these specific steps and of some embodiments.

In step 110, at least two genomes are received. Any two genomes may be used and more than two genomes may be received. In one embodiment, the genomes are the *Homo Sapien* (human) genome and the *Mus Musculus* (mouse) genome. In other embodiments, the genomes may be from plants or any other living organism. In step 120, the received genomes are masked using a library of sequences. This masking reduces the amount of the genome that has to be analyzed, but does not reduce the amount of genome to be analyzed as to exclude significant portions of the genome that might include microRNAs.

In step 130, the genomes are compared, e.g., using BLAST, to determine pairs of sequences that are homologous between the two genomes. Any percentage of sequence similarity or identity may be used to define homology. In some embodiments, homologous pairs share at least 80 %, 85 %, 90 %, 93 %, 95 %, 97 %, 99 % or 100 % sequence identity. These homologues pairs may be used to create a list of microRNAs precursor candidates. In step 140, certain homologous pairs are combined into assemblies, for example, through filtering of coding sequences and optimisation of the data. In step 150, the assemblies are clustered, thereby, finding new homologous pairs of sequences.

In step 160, the clusters are analyzed. In one aspect, the sequences of certain clusters are removed from a list of microRNAs precursor candidates. In another aspect, the sequences of certain clusters are identified for being added to the library of sequences to be masked. Thus, in one embodiment, in step 170, these sequences are added to the library. At this point, steps 120-160 may be repeated as needed with the results from using the new library for masking in step 120. In step 180, the sequences remaining in the list of candidates are analyzed to determine if their secondary structure satisfies certain combination of criteria. In step 190, list of microRNAs precursor candidates is provided.

### A. Masking The Genomes

A wide part of vertebrate genomes consists of stretches of highly repetitive DNA sequences classifed into five categories: simple repeats, tandem repeats, segmental duplications and interspersed repeats. To avoid a tremendous number of spurious matches in whole genome comparisons or analyses, these low-complexity regions are "masked" by replacing corresponding nucleotides by another character, such "N" or "X" or a lowercase character of the same type. Typically, not only are highly repetitive sequences masked, but also pseudogenes, non-functional copies of RNA genes, small RNA pseudogenes and all active small RNAs.

Currently, almost 50% of the human genome is annotated as belonging to these classes, and is masked accordingly by the standard RepBase library. This masking of the genome has been designed for a more accurate and sensitive analysis of genomic regions directly related to the expression of coding genes. Accordingly, research has been focused on the coding part of the genomes, applying strong masking criteria and filtering out what they called « junk DNA ». Thus, most of these genomes lack non-coding RNA genes, mainly because the masking applied to the comparison has cleaned them out. With regards to finding microRNA, the result is that too much of the genome is masked out and removed from further analysis. When masking involves a comparison of an unknown sequence to a list of known sequences such as a list of transposons, retrotransposons or pseudogenes, the decision to mask or not to mask is made at the default settings of the software or at a 95% sequence identity level, which ever is lower, with a word size of 8, e-value of 1 and a penalty of -1 for one mismatch, unless otherwise stated.

Accordingly, in one embodiment, to find more microRNAs, non-processed pseudogenes, unitary pseudogenes, non-functional copies of RNA genes, non-protein coding RNAs, small RNA pseudogenes, snRNAs, snoRNA, and all active small RNAs are selectively or in combination not masked in appreciable numbers. This results in at least 75 % of this group of sequences not being masked.

In another embodiment, only certain interspersed repeat sequences are masked, and an appreciable number of other interspersed repeat sequences are not masked. For example, the masking is restricted to an appreciable number of SINE and LINE interspersed repeated sequences, but not other interspersed repeat sequences, thus allowing this previously inaccessible part of the genome to be analyzed. In one aspect, the appreciable number of SINE and LINE sequences that are masked is from 75 % to 100 % of commonly attributed SINE and LINE sequences. In another aspect, an appreciable number of the other interspersed repeat sequences *not* being masked results in at least 75 % of these other interspersed repeat sequences *not* being masked. In another aspect, the other interspersed repeat sequences, which are not masked, include one or more of processed pseudogenes, retrotranscripts, DNA transposons, and retrovirus retrotransposons.

In one embodiment, the sequences which are not included in the masking library are interspersed repeated sequences other than SINE and LINE, but also other non-coding RNA. For example, all non-functional genes (such as pseudogenes and small RNAs), which are normally included in the "available masking files", are not included in the masking file of an embodiment of the present invention.

Therefore, embodiments of the present invention have investigated about 20 % of mostly unexplored human and mouse genome sequences (about 500 millions nucleotides located in non-coding regions never analyzed by others). Thus, subsequent steps of comparing two masked genomes, analyzing secondary structure, and identifying highly conserved areas in the secondary structure can find previously hidden microRNA precursor molecules.

In one embodiment, BLAST is used with a set (library) of repeat sequences as defined for one of the embodiments being used as the database and the genome defined as query. Other sequence alignment tools and software may be used. The result from BLAST provides where the sequences of the library appear in the genome. These found sequences can then be removed or replaced with 'X'. For example, xblast.pl may parse the results and remove sequence repeats, replace the sequences with 'X', replace the sequences with lower case letters, or replace the sequences with any other character.

In one aspect, this methodology of using a library of sequences to be masked allows for much more precision as only the sequences included in the library are masked. In contrast, RepeatMasker extends in 5' and 3' the sequences contained in their library. This results in a large masking around each sequence of the library.

### B. Comparing The Two Masked Genomes

The masked genomes are compared to determine pairs of sequences that are homologous between the two genomes. In one embodiment, an alignment algorithm is used to identify between the two genomes a pair of sequences that have a sufficient percentage of similarity (identity).

In one embodiment, each genomic comparison was performed using mpiBLAST (Darling et al., The Design, Implementation, and Evaluation of mpiBLAST), that is an implementation of the ncbi Blast (Karlin, Altschul PNAS 87:2264-2268, 1990) allowing the use of a cluster of computer in a Massive Parallel Interface (MPI) language. In one aspect, four dual processor computers were used with another computer running as master controller for all the jobs.

BLAST calculates all segment-pairs between the query and the database sequences that present sequence similarity above a scoring threshold. The result is a list of high-scoring pairs (HSPs). Each HSP is a pair of sub-sequences of the same length that form an ungapped alignment. The number of HSPs found depends of the parameters set for running BLAST. In particular, BLAST will report only those sequences whose scores are over some cutoff score. In one embodiment, the BLAST parameters used were: e-value: 0.01; and word size: 11. However, any parameters and any percentage of sequence similarity or identity may be used to define homology. In some embodiments, homologous pairs share at least 80 %, 85 %, 90 %, 93 %, 95 %, 97 %, 99 % or 100 % sequence identity.

One genome may be used for making queries to the other genome, which is used as the database. In one embodiment, the masked *Homo sapiens* genome was used as the query and the masked *Mus musculus* genome was used as the database. In one aspect, MySQL was used as the database environment. In this embodiment, computing took about 6.5 days to compare the *Homo sapiens* genome (as query) and the *Mus musculus* genome (defined as database). The sequence used as query (*Homo sapiens*) was split in chunks of 100000 base pairs (bp) with an overlap of 3000 bp- This was done to increase the speed of the comparison and to avoid overloaded computer memory.

In one aspect, the homology coordinates between the query and the hit are stored in a database. The coordinates are defined as a High Scoring Pair (HSP as defined by BLAST). Each HSP is defined by a start, an end and a strand on each genome. In one embodiment, a total of over 1 million HSPs were obtained at the end of this step. These homologues pairs may be used to create a list of microRNA precursor candidates.

Since BLAST relies on heuristic procedures, it is not guaranteed to find the best matches. Also, BLAST produces only binary relationships between one sequence of genome I and one sequence of genome 2. Thus, some of them can be missed, in particular when long sequences are compared (such as two mammalian genomes). In our case, both the Query sequence (human genome) and the Database (mouse genome) are very long. Thus, in one embodiment, the parameters are set in order to increase the predictiveness to avoid false positive. A direct consequence is a decrease in sensitivity, i.e. the loss of true positives.

### C. Creation Of Assemblies from Homologous Pairs

The creation of "assemblies" can generally be achieved by two different processes. In filtering, two HSPs that overlap or are successive are combined. In optimization, HSPs that share the same sequence are collapsed into the same entry.

Filtering reduces the number of HSP (potentially over one million) by assembling the HSPs into large sequences, which are one form of an "assembly". In view to (1) avoid potential overlaps between HSP and (2) have a more realistic view of sequence homologies between the genomes compared, a unique record integrates those HSP which sequences are either overlapping or consecutive in the two genomes. These assembled HSPs are included within the term "assemblies".

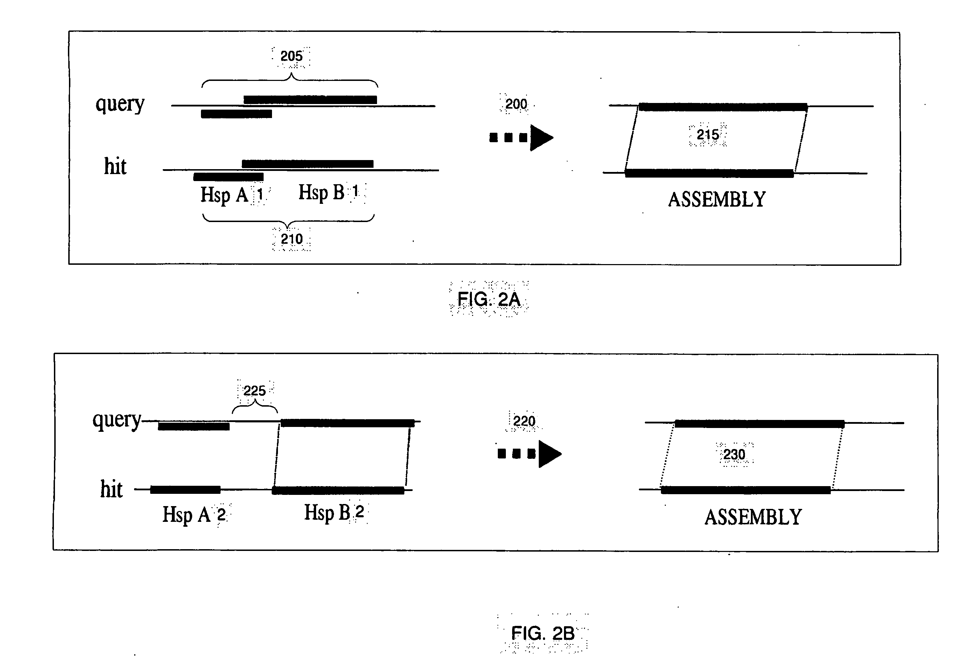

Figure 2A illustrates an assembling process 200 of creating an "assembly" by filtering according to one embodiment of the present invention. In process 200, if two or more HSPs share the same query and hit genomic sequence in terms of genomic position, then a new 'HSP' is built and an "assembly" is created. For example, HASP A1 and HSP B1 share the same query sequence 205 and hit sequence 210. Thus, they are combined into assembly 215.

Figure 2B illustrates another assembling process 220 of creating an "assembly" by filtering according to one embodiment of the present invention. In process 220, if two queries and two hits coming from two HSPs share the same chromosome and have a distance which is similar, then a new 'HSP' is built and an "assembly" is created. For example, HSP A2 and HSP B2 share the same chromosome and have a distance 225 which is similar. Thus, they are combined into assembly 230. So, an assembly may be defined by a start, an end, a chromosome, a strand on the query and on the hit, and an internal identifier in the database.

Therefore, an assembly created by filtering is a set of HSPs that follow certain rules. In one embodiment, tor the two genomes (species) included in the comparison, the size variation between two sequences of two consecutive HSP is less than 10 %, and the resulting alignment including the two HSPs plus the intervening sequence must show more than 90 % sequence similarity.

An "assembly" may also be created by optimization. Optimization avoids redundancies in the database, as "query" sequences having many "hits" are grouped in only one entry. When making a BLAST run, if a sequence defined as a query is present many times in the database, the results will give a report with the number of HSPs being equal to the number of times the sequence is present in the database HSPs with the above characteristic are grouped into an "assembly". This means all the "hits" will then refer to one "query" in the database, which are then marked with an integer. This step makes it possible to reflect the situation in the genome and to make a data compression of at least 29 %.

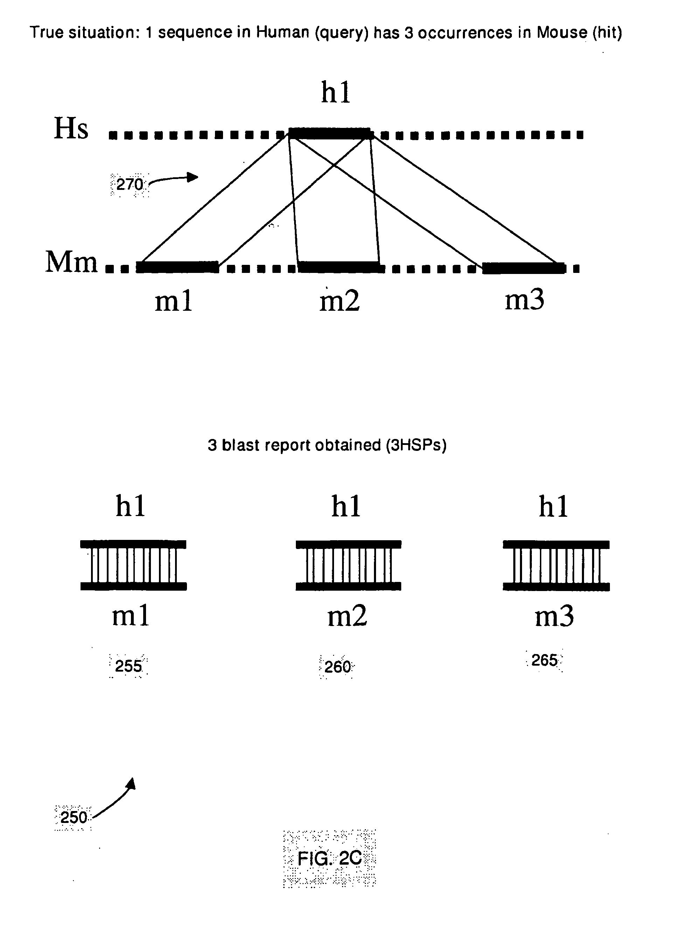

Figure 2C illustrates an assembling process 250 of creating an "assembly" by optimization according to one embodiment of the present invention. Three BLAST reports obtained three HSPs 255-265. Each HSP 255-265 has sequence "hl" as the query. Thus, process 250 combines HSPs 255-265 into "assembly" 270.

Thus, after this step, the list of homologous sequences is recorded either in HSPs or assemblies. Each homologous pair of sequences (HSP or assembly) is recorded with exactly the same information. An assembly may be defined by the resulting file containing the mix of HSPs and "assembled HSP" from the filtering process. An "assembly" also includes HSPs that have not been combined with other HSPs. For example, in instances, where filtering and optimization does not apply to an HSP, then that HSP would be considered an assembly.

Thus, in one embodiment, an assembly is defined by: an identifier, for both human and mouse genomes a chromosome, a start, an end, a strand. One query sequence (with a chromosome, a start, an end, a strand) may belong to many "assembles". One hit sequence (with a chromosome, a start, an end, a strand) may also belong to many "assemblies".

### D. Clustering Of Homologous Sequences

In order to reduce the background noise due to the genomic comparison and to filter sequences that are over represented in the two genomes, a clustering method is applied to cluster assemblies with homologous sequence but different genomic positions. In one aspect, the background noise comes from the fact that the BLAST parameters are chosen for increasing predicteness. Thus, very few false positive homologous sequences are obtained, but some true positive ones are lost. A subsequent clustering of assemblies allows finding undetected true positives.

Each assembly record contains an alignment of the two sequences identified as "homologous" by BLAST. This homology is, in particular, dependent on the degree of similarity between the two sequences. Each sequence within an assembly is defined by its coordinates (5'end, 3'end) along the genome, the genomic strand (plus or minus) and the chromosome number. The coordinates (5'end and 3'end, strand and chromosome-identification) of the sequences for all assemblies are scanned. In one embodiment, assemblies are clustered together if each assembly of a cluster contains one sequence (either query or database) which has exactly the same coordinate (5'end and 3'end, strand and chromosome identification) as at least one other assembly of the cluster. The underlying assumption is: if A is homologous to B and A is homologous to C, then B and C are homologous, which potentially has been missed by BLAST. This is term an associative property of homologous pairs.

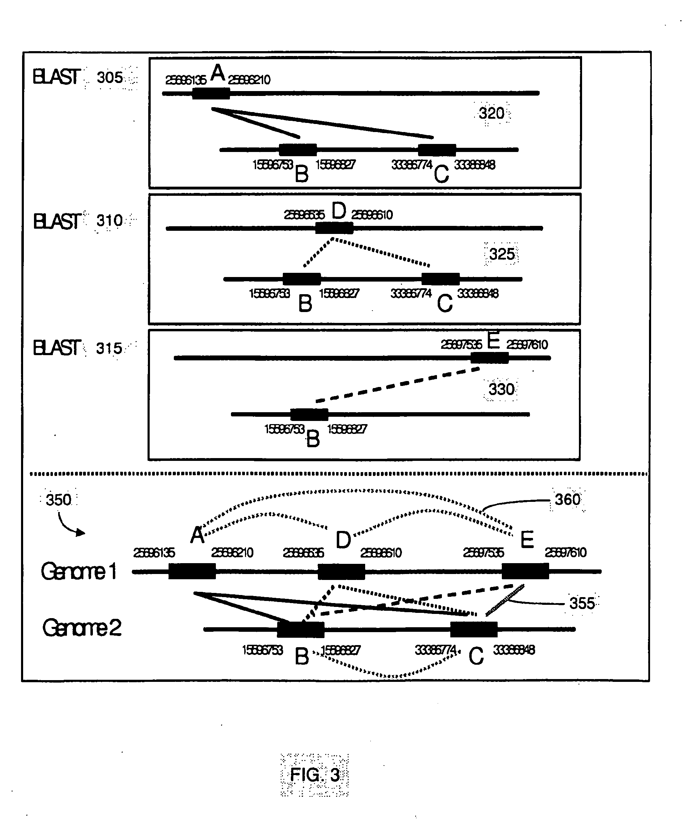

Figure 3 illustrates a cluster 350 created according to an embodiment of the present invention. Three BLAST reports 305-315 provide three different assemblies 320-335. These assemblies 320-335 are combined into cluster 350 by a clustering process that tests if at least one sequence of an assembly is shared with at least one other assembly of a cluster. In this example, there is even more sharing as each assembly shares sequence B. In this manner, new homologous relationships are found.

For example, sequences E and C, which are not seen as homologous by BLAST report 315 are actually related as shown by their very high sequence similarity with the other sequences of this cluster. This homology of an orthologous type is signified by the new relationship 355. Also, sequences A, D, and E are homologous to each other. This homology of a paralogous type is signified by relationships 360. Each line in Figure 3 signifies a homologous relationship, and each sequence in a cluster has a homologous relationship with every other sequence of that cluster.

Despite working on coordinates of homologous sequences detected by BLAST, an embodiment clusters together only sequences which have evident homologous relationships (high similarity rate). This is one of advantages of such a clustering method according an embodiment of the present invention. In one aspect, appropriate SQL syntax and Perl scripts are used to cluster "assemblies" that share at least one sequence (sequence hit or sequence query) with at least one other "assembly" of the cluster.

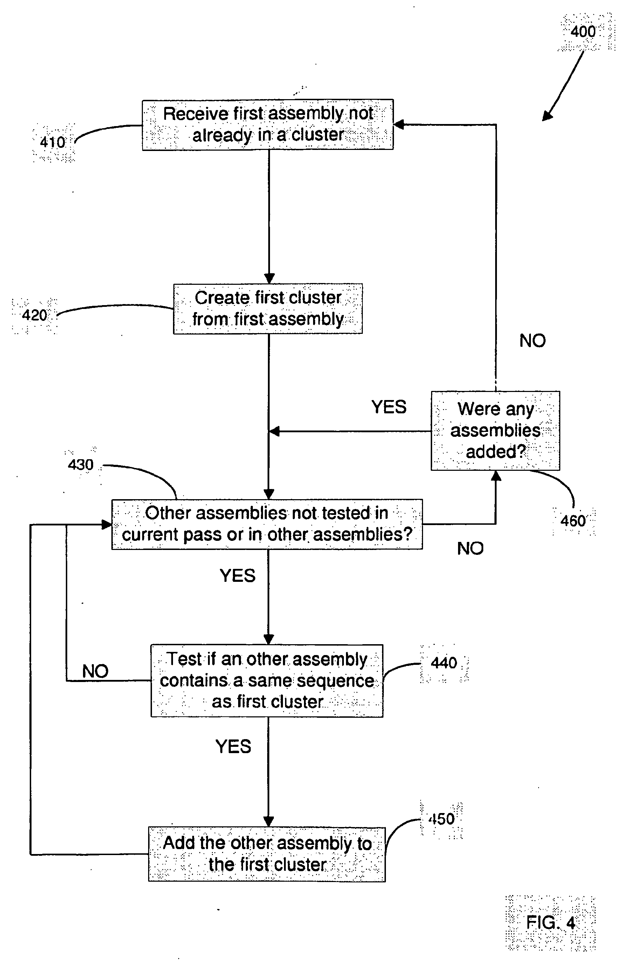

Figure 4 illustrates a method 400 of clustering assemblies according to an embodiment of the present invention. In step 410, a first assembly is received. In step 420, a cluster is created from the first assembly- At this point, the cluster and assembly are identical as no other assemblies have yet been added. In step 430, it is determined if other assemblies have not been tested. In one aspect, it is also determined if other assemblies do not already belong to a cluster.

If there is another assembly, in step 440, the other assembly is tested whether the assembly contains a sequence in the first cluster. If the other assembly does not contain a sequence that is within the first cluster, then the process reverts back to step 430 to determine if there are any other assemblies to be tested. If the other assembly does contain a sequence that is within the first cluster, then the sequences of that assembly are added to the first cluster. Once the assemblies are exhausted, the method moves form step 430 to step 460, which determines if any assemblies have been added to the first cluster in the current pass through the assemblies. If an assembly has been added in the current pass, then another pass of testing the assemblies is made. If no assemblies have been added, then method 400 repeats for the remaining assemblies not already in a cluster by receiving one of them and starting a new cluster. One skilled in the art will appreciate other methods for performing the clustering according to the rule that at least one sequence of an assembly is shared with at least one other assembly of a cluster.

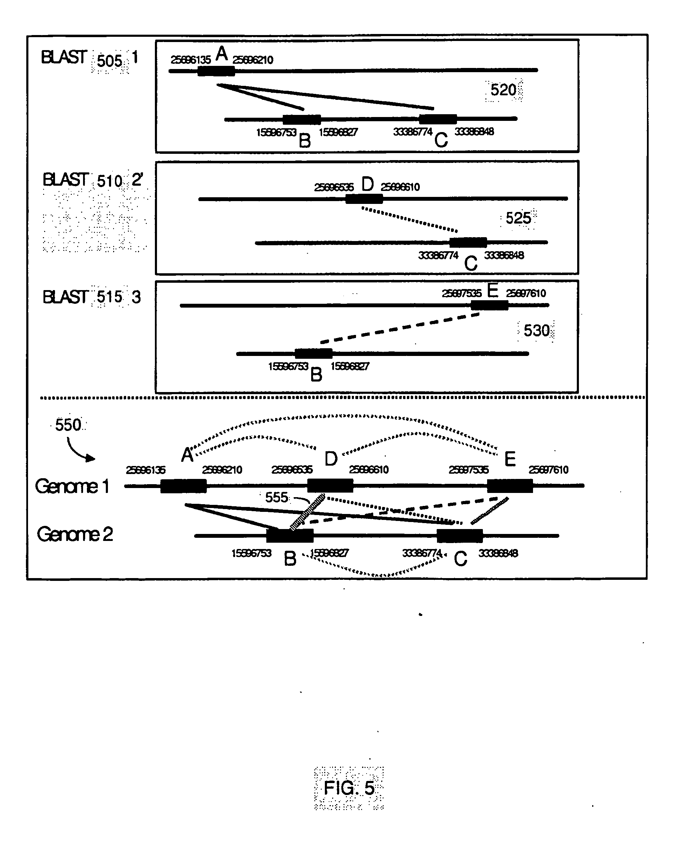

Figure 5 illustrates a cluster 550 created according to an embodiment of the present invention. Three BLAST reports 505-515 provide three different assemblies 520-535. These assemblies 520-535 are combined into cluster 550 by a clustering process, which tests if at least one sequence of an assembly is shared with at least one other assembly of a cluster, e.g. method 400. In this example, BLAST report 510 has not shown a homologous relationship between sequence D and B. However, since assembly 520 and assembly 525 both share sequence C, cluster 550 contains both D and B, thus deeming them to have a homologous relationship 555, which is of the orthologous type to be precise.

In one embodiment of using method 400, the first assembly received (410) is the assembly 520 of sequences A, B, and C. In step 420, cluster 550 is formed from assembly 520. In step 430, it is determined that assemblies 525 and 530 have not been tested. Control then moves to step 440, where assembly 525 is tested if assembly 525 contains a sequence in common with the sequences of cluster 550, which at this point are sequences A, B, and C. Since assembly 525 shares sequence C with cluster 550, sequence D is added to cluster 550, in step 450. Then upon returning to step 430, it is determined that assembly 530 has not been tested. At step 440, sequence B is determined to be shared between assembly 530 and cluster 550. Thus, at step 450, sequence E is added to cluster 550.

If there were other assemblies that were not added to cluster 550, then these remaining assemblies would be clustered in a similar procedure. In one aspect, another pass at the remaining assemblies is done to determine if any of them share sequences with assemblies (effectively then entire cluster) that were added to the cluster after that assembly was tested. This may be needed to ensure that a cluster has all assemblies of the genome that are homologous within one cluster. Continued passes over the remaining assemblies may be performed until no more assemblies are added. At that point, a new cluster may be created from one of the remaining assemblies, and method 400 may be repeated for that cluster.

Thus, in one embodiment, a cluster is created as a set of "assemblies" that are homologous in sequence. In one aspect, a cluster is referenced by an identifier which refers to the set of identifiers for each of the assembly of the cluster. In another aspect, a cluster is also referenced by the number of homologous sequences (links) that a cluster contains from the organisms that have been used for the comparative genomics. The number of links for each cluster is then stored in the database, along with the corresponding assemblies.

In one aspect, this clustering method allows grouping of paralogous sequences and orthologous sequences together. Paralogous sequences are sequences that have diverged from a common ancestral sequence. Orthologous sequences are sequences that have evolved directly from an ancestral sequence. According to this clustering method, the number of times a sequence is present in the genomes is known, and specifically the number of times a sequence is present in the query genome and how many times the same sequence is present in the database sequence is known.

The initial BLAST comparison is based on the similarities between the sequences to identify homology relationships. In, one aspect, our clustering method allows to group together all the sequences of both genomes which are homologous, between the two genomes. In addition, this clustering step allows identifying all the paralogous sequences within each genome. The underlying tenet is a systematic reference to homology relationships between sequences of the two genomes compared by BLAST.

In another aspect, this clustering method allows increasing the sensitivity of the BLAST result without increasing the number of false positive. The clustering method can identify homology relationships not detected by BLAST. The predictiveness of BLAST results is consequently also considerably increased.

### E. Analyzing Clusters

### 1. Selecting Sequences to Add to Masking Library

In one embodiment, the clustering of assemblies was also used to identify new highly repeated sequences. These new repeat sequences may be added to the masking library, as described in step 170 of Figure 1. These new repeat sequences may be determined from the clusters of assemblies. In one aspect, these new repeat sequences are taken from clusters that have a large number of sequences within that cluster. For example, clusters having more than 1000 sequences may be identified as being highly repetitive and are added to the masking library. In other embodiments, the required number of sequences may be as low as 500.

In one embodiment, 1200 different clusters having more than 1000 sequences were identified. In one aspect, for each cluster, one sequence is sufficient to define a cluster. Thus, one new repeat sequence may be added to the masking library for each cluster having a sufficient number of sequences. Then, as was previously done in step 120 of Figure 1, BLAST is used to mask each genome with these new sequences.

### 2. Annotation of the Conserved Non-Coding DNA

In one embodiment, to predict non-coding genes and in particular microRNA genes, all the coding regions as defined by an official genome annotation are flagged in order to exclude them for future predictions.

Using a database of the "assemblies" and knowing the exact position of the sequence corresponding to the query (e.g., human genome) and using human annotation provided by Ensembl on the same genome, the exact position of the query sequence which does not code for a protein with a 'non-coding' flag is stored in the database. Thus, in one aspect, the coordinates of each query sequence, which is conserved, are used to define whether sequence is part of a coding gene or not. All the conserved regions are then flagged 'coding' when they correspond to a coding gene. Then, the coordinates for the non-coding part of the conserved query genome are stored in a database.

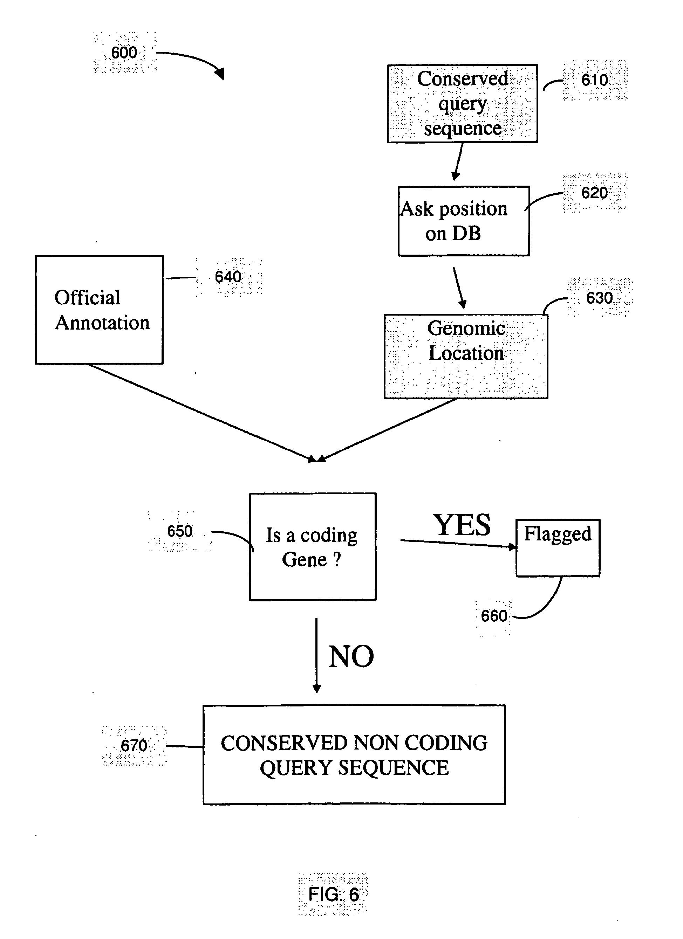

Figure 6 illustrates a method 600 of annotating the sequences of assemblies as containing coding or non-coding DNA. In step 610, conserved query sequences are taken from the sequences of the assemblies belonging to one of the genomes, e.g., the query genome. In step 620, the positions of the sequences are obtained from the database. In step 630, the genomic location of the sequence is determined from the positions. In step 650, these sequences are compared to the official annotation 640 to determine if a gene is coding or not. In step 660, if the gene is coding then the sequence is flagged as coding. In step 670, if the sequence does not contain a coding gene then it is determined to be a conserved non-coding query sequence.

### 3. Elimination of Assemblies and Clusters

In one embodiment, the sequences of the clusters are analyzed in order to eliminate one or more of the sequences as microRNA precursor candidates. In one aspect, one ore more of the following characteristics are used to eliminate sequences. MicroRNA are: (1) present as only a relatively low number or less of copies in one genome, e.g., about 6 per genome, which results in about 12 per cluster when two genomes are used; (2) flagged as non-coding; (3) at least 60 nucleotides (nt) in length under the form of their precursor; and (4) conserved with at least 85 % identity between two genomes, such as Homo sapiens and Mus musculus. In one embodiment, only the length characteristic is used.

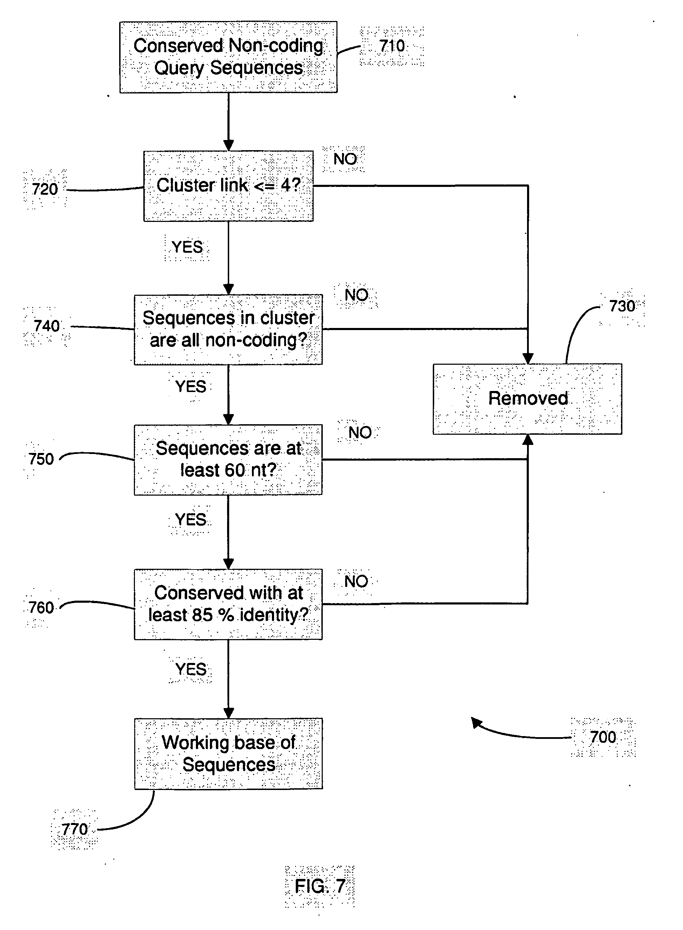

Figure 7 illustrates a method 700 of eliminating sequences of assemblies and clusters according to an embodiment of the present invention. In step 720, non-coding sequences 710 based on the query annotations are checked to see if they are in a cluster with less than or equal to a certain number of homologous sequences (links). For example, if four links is used, then a sequence is checked to determine if the sequence is in a cluster with no more than 2 hits and no more than 2 queries. If a cluster satisfies this characteristic, one or more of the sequences of a cluster remain in the list of candidates. If a cluster does not satisfy this characteristic, then the sequences of the cluster are removed from the list of candidates at step 730. The total number of hits and queries may also be used, in addition to the number specifically in each gnome.

In step 740, all the sequences in a given cluster are checked to determine if they are all flagged as 'non- coding'. If a cluster satisfies this characteristic, the cluster remains in the list of candidates, and if not, then it is removed in step 730. In step 750, sequences are checked if they have at least 60 nucleotides. In step 760, an assembly is checked to determine if the sequences of the assembly are at least 85 % identical. In step 770, the remaining sequences are put into a working database of sequences. -

This method helps to filter down the number of sequences to scan afterwards. The use of the cluster allows building a working database with sequences that have any of the characteristics of not coding for a gene, not over represented in the two compared genomes, sufficient length, or have at least 85 % identity.

### F. Analyzing Secondary Structure of Resulting Sequences

In one embodiment, the secondary structures of sequences in the list of candidates (working database) are analyzed. A pre-microRNA transcript is a precursor RNA having a stem-loop like structure. Using the working base (WB) described before, the inventors use folding algorithms to predict secondary structures of all the sequences in the WB in the forward and in the reverse strand. The algorithm which has been used comes from the Vienna package (Hofacker lL. Vienna RNA secondary structure Nucleic Acids Res., 2003, Jul 1;31(13):3429-31).

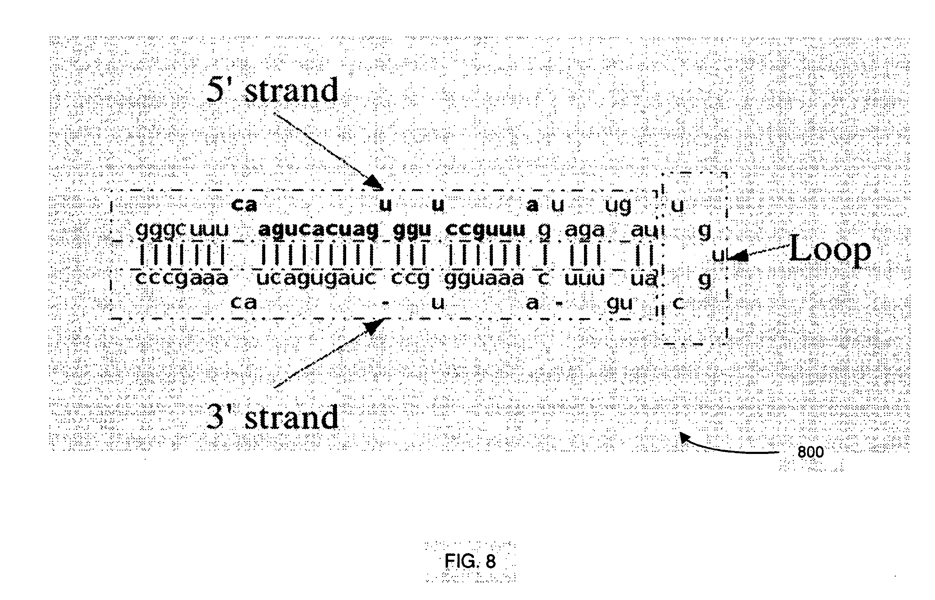

Figure 8 illustrates a secondary structure 800 ofan exemplary sequence. There is a 5' strand stem (arm), a 3' strand stem (arm), and a loop parts of secondary structure 800. In one aspect, the secondary structure is searched to locate a microRNA in the 5' strand stem or 3' strand end or both.

Knowing the secondary structure of a pre-microRNA, the results are parsed to find out if the sequences have a secondary structure which may correspond to the classical structure of pre-microRNA family members using an appropriate Perl script. Perl script evaluates a certain combination of criteria before accepting a sequence as a potential pre-microRNA candidate.

In one embodiment, sequences are identified that have a stem-loop secondary structure with a 5' strand (arm) stem having from 20 to 45 nucleotides and with a 3' strand stem having from 20 to 45 nucleotides. In one aspect, sequences are identified that also satisfy the following four criteria: (1) a number of nucleotides (nt) in the sequence, i.e. the pre-miRNA stem-loop; (2) a length of the loop; (3) a percent identity; and (4) a Zscore. The loop length is the length in nucleotides of the hairpin loop of the stem loop, i.e. not including arms. The percent identity is the percentage of conserved nucleotides between two sequences.

The Zscore is a statistical test. The principle of this method is to generate N random sequences having the same nucleotide composition of a given sequence. Each of these N random sequences are then folded and the Minimal Free Energy (MFE) is given. If R is the number of sequences with MFE < MFE of initial sequence, the Zscore equals R / (N+1). In one embodiment, the number of nucleotides (nt) required is 60-120 nt; the loop length is 4-15 nucleotides; and the percent identity is at least 85 %.

In one embodiment, sequences with a Score (using N = 1.000) less than 0.06 are kept. When a sequence and its reverse complement are less than 0.06, the one with the lower Zscore is kept. In one aspect, if the sequence comes from an orthologous region with less than 100 % identity, the same statistical test is applied to the orthologous sequence. In another aspect, the sequence is kept, if the orthologous one is also under 0.06.

In one embodiment, four more criteria are analyzed to determine if a predetermined number (subset) of them are satisfied: (1) energy (MFE); (2) %GC; (3) % of base pairing; and (4) minimum conserved nucleotide of one arm. The "%GC" is the percentage of nucleotides G + C in a given sequence. The "% base pairing" is the number of bases which are paired between the two arms. The minimum number of conserved nucleotides of one arm (Arm conserved) is the minimum number of nucleotides, which are perfectly conserved between the two species compared in at least one arm of the stem loop. In one embodiment, the following values for the criteria are used: % GC from 30 to 51 %; percentage base pairing between 30 and 40 %; a Minimum Free Energy less than 25 kcal/mol; and a minimum 17 nucleotides are perfectly conserved between the two species compared in at least one arm of the stem loop. In one aspect, three of the four criteria are required to be satisfied.

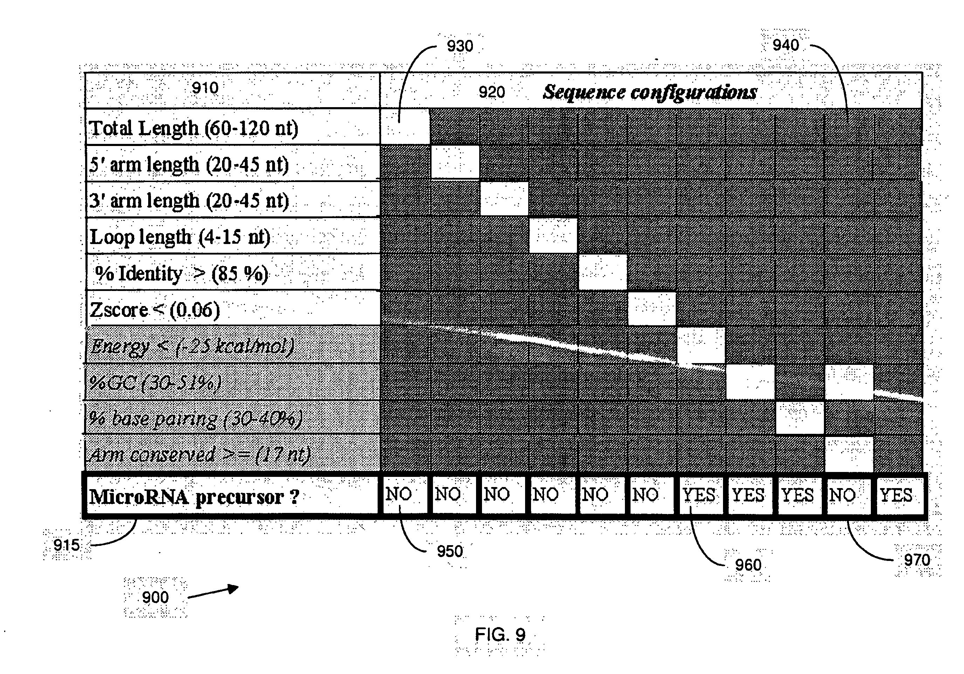

Figure 9 illustrates a table 900 showing the criteria and the decision of whether a sequence is a microRNA precursor candidate. Column 910 lists ten different criteria. In one embodiment, each of the first six criteria (with the light background) is deemed required. The last four criteria (with a shaded background) are deemed optional, and only a subset of them are required. In one aspect, three out of the last four criteria are required. Each of columns 920 signify a sequence whose secondary structure is analyzed. Row 915 shows the decision as to whether a sequence is a candidate.

A white box 930 signifies that the criterion is not satisfied. A dark box 940 signifies that the criterion is satisfied. In one embodiment, sequence 950 is deemed to not be a candidate as the first required criteria of total length is not satisfied. In one embodiment, sequence 960 is deemed to be a candidate since only one optional criterion (energy) is not satisfied. In one embodiment, sequence 970 is deemed not to be a candidate since two optional criteria (%GC and ARM conserved) are not satisfied.

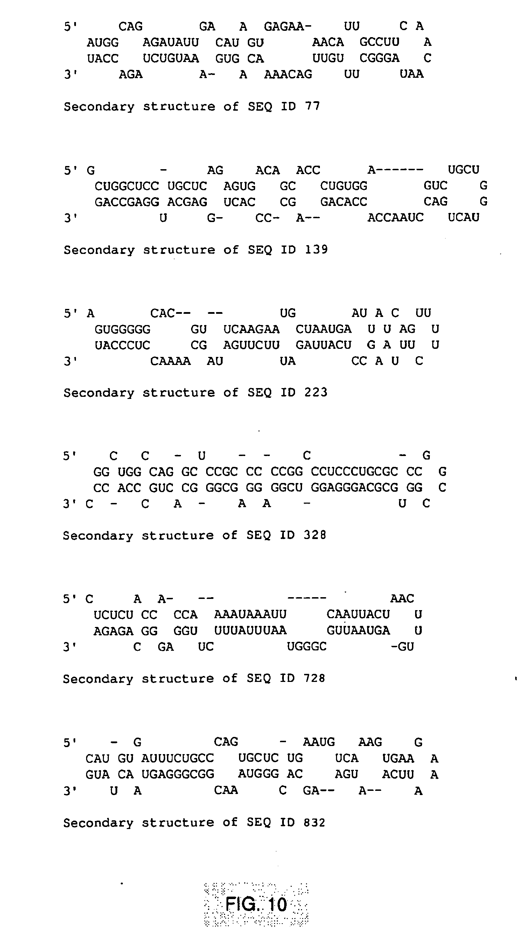

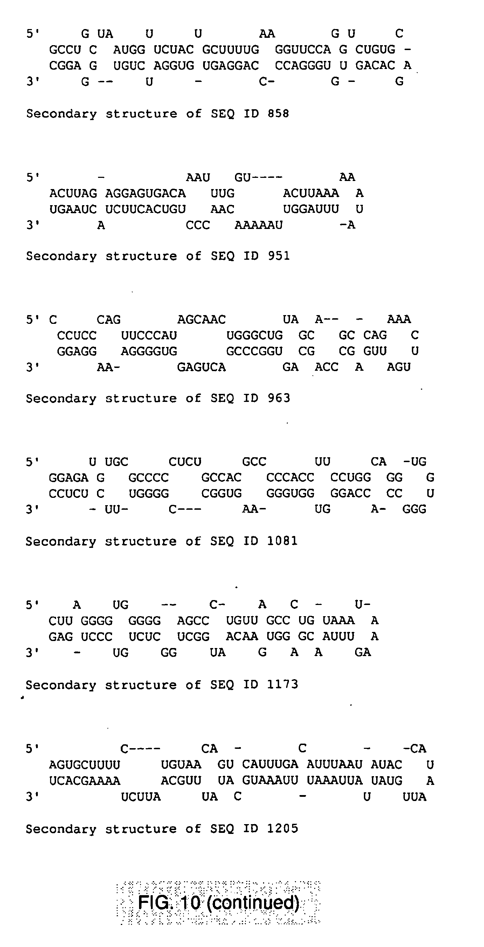

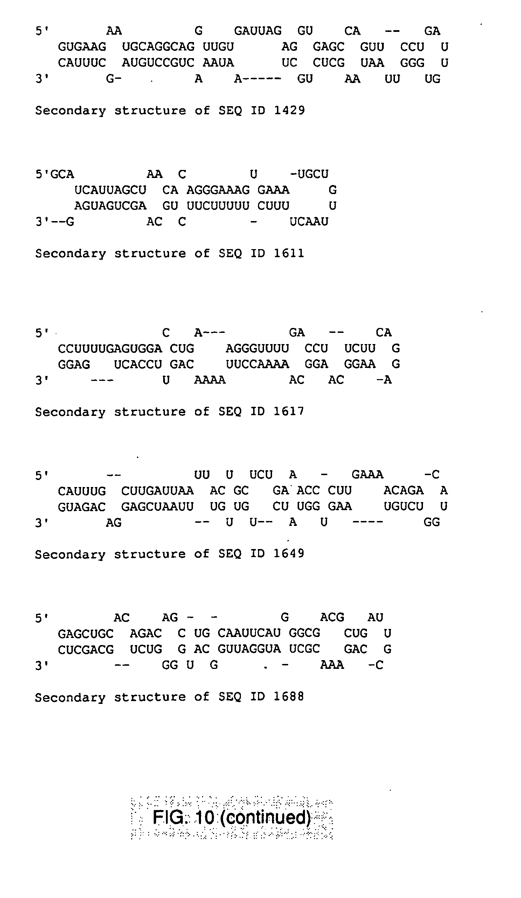

In one embodiment, when a stem-loop is found, the exact positions of the 5' strand start and the exact positions of the 3' strand end are extracted to make a new sequence. This new sequence is then folded again and the structure parsed again to see if it fits with a secondary structure corresponding to a pre-microRNA stem-loop. This is done because secondary structure may change according to the neighbouring nucleotide sequence. Genomic positions of sequences having a pre-microRNA secondary like structure are kept in the database. In one aspect, sequences are computed to find their tissue annotations by comparison with the dbEST database. Figure 10 illustrates secondary structures of select sequences.

### EXAMPLES

The following examples are offered to illustrate, but not to limit the claimed invention.

### Example 1: Identified pre-microRNA sequences using the validation protocol in Example 3, below.

See the pre-microRNA nucleic acids having the DNA sequence SEQ ID NOs. 3, 5, 16, 22, 32, 34, 35, 36, 60, 70, 77, 81, 82, 90, 93, 139, 141, 151, 152, 158, 163, 170, 175, 176, 183, 184, 191, 192, 200, 205, 219, 223, 224, 248, 275, 276, 323, 328, 344, 357, 363, 366, 385, 400, 406, 407, 425, 472, 481, 489, 500, 501, 503, 512, 527, 530, 531, 563, 608, 633, 640, 65t, 653, 664, 678, 728, 744, 753, 784, 785, 786, 826, 832, 858, 876; 903, 909, 911, 926, 927, 940, 942, 944, 946, 947, 951, 955, 963, 967, 975, 1055, 1060, 1083, 1118, 1129, 1137, 1145, 1160, 1161, 1166, 1173,1174,1175, 1198, 1.205, 1207, 1217, 1233, 1238, 1251, 1267, 1299, 1321, 1323, 1343, 1352, 1396, 1403, 1429, 1485, 1531, 1545, 1547, 1559, 1586, 1592, 1601, 1610, 1611, 1617, 1634, 1645, 1649, 1653, 1656, 1661, 1667, 1681, 1686, 1688, 1693 and 4773.

### Example 2: Identified microRNA sequences (mature microRNA) using the validation protocol in Example 3, below.

See the microRNAs having the DNA sequences SEQ ID NOs. 1697, 1698, 1700, 1719, 1720, 1730, 1731, 1732, 1744-1750, 1784, 1785, 1805-1807, 1815-1818, 1824-1828, 1837-1839, 1842, 1843, 1896-1900, 1911-1917, 1925, 1926, 1933, 1934, 1944, 1945, 1949-1954, 1959-1964, 1975-1979, 1990-1993, 2000-2002, 2025-2027, 2068, 2108, 2111, 2112, 2173-2175, 2180, 2181, 2201-2203, 2219-2221, 2228, 2229, 2231-2237, 2260, 2278-2280, 2313, 2314, 2408-2410, 2427-2434, 2437, 2438, 2532, 2450, 2451, 2469-2471, 2476, 2477, 2613, 2620-2622, 2637, 2648, 2679, 2730, 2754-2756, 2771, 2809, 2810, 2814, 2815, 2862, 2863, 2870-2872, 2900, 2958-2961, 2966-2968, 2971-2974, 2999-3001, 3015, 3017-3019, 3021, 3023-3027, 3029, 3030, 3038, 3039, 3048-3050, 3052-3054, 3061, 3062, 3162, 3189, 3232, 3233, 3249, 3250, 3271-3274, 3300-3304, 3320-3329, 3363, 3372-3375, 3378, 3379, 3391, 3392, 3416, 3417, 3422-3424, 3440-3442, 3461, 3497-3499, 3528, 3555-3557, 3567, 3619-3621, 3627, 3628, 3663, 3786, 3804-3808, 3862, 3863, 3870, 3871. 3896-3898, 3912, 3931, 3943, 3950, 3951, 3956, 3957, 3959, 3960, 3966-3968, 3973-3976, 3986, 3987, 3991-3994, 4001-4004.

### Example 3: Demonstration that identified microRNAs are transcripts present in cells

### A) Material and Methods

### Protocol for the microRNA expression profiling ("validation protocol")

### Sample: total RNA from mouse

Total RNA from three different mouse tissues listed below were tested in a first run of microRNA expression profiling based on microRNA identification performed with RNAgate^{™}.

### Mouse normal tissue total RNA

Total RNA utilized for the test experiments was purchased from Biochain Institute Inc., Hayward, CA, USA. The RNA quality was verified with the Agilent bioanalyser.

**Table 2**

| Tissue | cat # | Size |
|---|---|---|
| Liver | R1334149-50 | 50 µg |
| Skeletal muscle | R1334171-50 | 50 µg |
| Lung | R1334152-50 | 50 µg |
| Brain | R1334035-50 | 50 µg |
| Heart | R1334122-50 | 50 µg |

In general, RNA extraction is performed such that small RNAs are not lost. To this end commercially available kits for total RNA isolation can be utilized, such as offered by Ambion, Invitrogen and other motecular biology tool providers.

### MicroRNA enrichment

MicroRNA enrichment can be achieved by utilising the mirVANATM miRNA isolation kit, the mirVANATM PARJS^{™} kit from Ambion.

miRNA purification was performed using the below described gel purification protocol to mapp mature miRNA of about 20 nt in length. The decision not to use a commercially available kit was to diminish background noise, generated when purifying' total RNA with a size inferior to 200 nt.

### Protocol

1 Prepare a 15 % polyacrylamide denaturing gel.
2 Polymerise for 30 min.
3 Resuspend samples in sample buffer (1: 1).
4 Denature RNA samples at 95°C for 5 min.
5 Incubate on ice until loading.
6 Preheat the gel at 400 V for 20 min in 1 x TBE.
7 Clear wells from urea prior to loading.
8 Run the gel until the bromophenol blue dye front (the leading dye) migrates about 4-5 cm down the gel.
9 Cut out gel slice between both dyes (bromphenol Blue and Xylene Cyanol). Corresponds to size 60 - 10 nt.
10 Mix gel slice with elution buffer in an Eppendorf. The gel slice has to be entirely covered.
11 Vortex to mix.
12 Incubate at 37°C overnight.
13 Centrifuge at Vmax for 2 min.
14 Decant Supernatant 1 into a sterile tube.
15 2nd Elution: Add same amount of elution buffer as in step ten.
16 Incubate 4 min at 95°C.
17 Centrifuge at Vmax for 2 min.
18 Decant Supernatant 2 into a sterile tube.
19 Pool both supernatants.
20 Add 05 µl glycogen at 25 mg/µl.
21 Precipitate with 3 Volumes ethanol at 99 % (min. 2 hours).
22 Centrifuge for 15 min at Vmax and 4°C.
23 Wash with 70 % ethanol.
24 Vacuum dry for 5 min in speed-vac.
25 Disolve pellet in 3 µl sterile Rnase-free water.

### Buffers

| 10 x TBE buffer, pH 8.0 at 25°C | |
|---|---|
| Tris | 890 mM |
| Boric acid | 890 mM |
| EDTA | 20mM |

| Denaturing Polyacrylamide Gel | |
|---|---|
| Polyacrylamide | 15% |
| Urea | 7 M |
| Ammonium Persulfat | 0,1% |
| TEMED | 25 µl/100 ml |
| in 1 x TBE | |

| Sample buffer | |
|---|---|
| Formamide | 95 % |
| 500mM EDTA | 20 mM |
| Bromophenol Blue | 0.05 % |
| Xylene Cyanol FF | 0.05 % |

| Elution buffer | |
|---|---|
| Sodium Acetate | 500 mM |
| EDTA | 1 mM |

### Sample labeling

Samples were labeled Cy3 and/or Cy5 using the mirVanaTM miRNA labeling kit from Ambion

### Microarray

### Probe design

The oligonucleotides were designed according to SEQ ID 77, 139, 223, 328, 728, 832, 858, 951, 963, 1083, 1173, 1205, 1429, 1611, 1617, 1649, 1688 and to A. thaliana microRNA sequence named ath-MIR156a ath-MIR157 as negative control (the precursor sequence of both are not present in the human genome).

Probe was designed using the protocol below:
1) extracting both the 5' stem and the 3' stem of the precursor microRNA
   As used herein, the oligonucleotide coming from the 5' end stem is called SEQ ID-L1, and oligonucleotide coming from the 3' end is called SEQ ID -R1;
2) check the longest sequence for both which is fully conserved with mouse genome for the oligonucleotide R1 and L1;
3) if sequence (R or L) length <= 18 nt; remove it
   if length < 35; catch sequence
   if 45 > length >= 35; design 2 sequences (named -1/-2) with a minimum coverage of 24 nucleotides catch sequences else,
   make sequences with a walk of 10 nucleotides to cover all the sequence,
   catch sequences;
4) reverse complement sequence;
5) add a spacer (15 nt) on 5' of the sequence.

Reverse complement sequences from design without spacer are SEQ ID 1815, 1896, 2026, 2180, 2181, 2730, 2870, 2871, 2872, 2900, 3029, 3050, 3189, 3322, 3374, 3663, 3912, 3951, 3993, 4004.

Following is an example of oligonucleotide design for SEQ ID 77.
Example: oligonucleotide design for SEQ ID .77:
- **Sequence L1 = 5' stem**
>77-L1
ATGGCAGAGATATTGACATAGTGAGAAAACATT
conservation ofsequence L1 with mouse :
human ATGGCAGAGATATTGACATAGTGAGAAAACATTGCCTT
mouse ATGGCAGAGATATTGACATAGTGAGAAAACATTGCTTT
****************************************************** * *
sequence to use to design oligonucleotide
ATGGCAGAGATATTGACATAGTGAGAAAACATTGC
Length of this sequence is 35 nt, so 2 oligonucleotides for this 77-L1 with a minimum of coverage of 24 nucleotides:

| | |
|---|---|
| L1-2 | cagagatattgacatagtgagaaaacattgc |
| L1-1 | atggcagagatattgacatagtgagaaaa |

reverse complement the 2 oligo to obtain oligonucleotides:

| | |
|---|---|
| 77-L1-1 : | TTTTCTCACTATGTCAATATCTCTGCCAT |
| 77-L2-2 : | GCAATGTTTTCTCACTATGTCAATATCTCTG |

- **Sequence R1 = 3' stem**
>77-R1
AGGTTGACAAAACAGTGAAATGTCTAGACCAT
conservation of sequence R1 with mouse:

| | |
|---|---|
| human | AGGGCTTTGTTGACAAAACAGTGAAATGTCTAGACCAT |
| mouse | AGGGCTTTGTTGACAAAACAGTGAAATGTCTAGACCAT |
| *************************************************************** | |

sequence to use to designed oligo:
AGGGCTTTGTTGACAAAACAGTGAAATGTCTAGACCAT
length of this sequence is 35 nt, so 2 oligos for this 77-L1 with a minimum of coverage of 24 nucteotides:

| | |
|---|---|
| R1-2 | tttgttgacaaaacagtgaaatgtctagaccat |
| R1-1 | agggctttgttgacaaaacagtgaaatgtc |

reverse complement the 2 oligo to obtain oligo:

| | |
|---|---|
| 77-R1-1 : | AGACATTTCACTGTTTTGTCAACAAAGCCCT |
| 77-R1-2 : | ATGGTCTAGACATTTCACTGTTTTGTCAACAAA |

- **add spacer to all designed oligonucleotide** (TTGTAATACGACTCA) on 5':

| | |
|---|---|
| 77-L1-1 : | TTGTAATACGACTCATTTTCTCACTATGTCAATATCTCTGCCAT |
| 77-L1-2 : | TTGTAATACGACTCAGCAATGTTTTCTCACTATGTCAATATCTCTG |
| 77-R1-1 : | TTGTAATACGACTCAAGACATTTCACTGTTTTGTCAACAAAGCCCT |
| 77-R1-2 : | TTGTAATACGACTCAATGGTCTAGACATTTCACTGTTTTGTCAACAAA |

See Table 3 for the sequence of OLIGO design.

**Table 3: OLIGO design**

| | | |
|---|---|---|
| **OLIGO NAME** | | **SEQUENCE (SEQ ID Nos. 4005 to 4034)** |
| 77-R1-2 (10303-R1-2)* | 4005 | TTGTAATACGACTCAATGGTCTAGACATTTCACGTTTTGTCAACAAA |
| 139-R1-1(10553-R1-1) | 4006 | TTGTAATACGACTCACCTGGCTCCATGCTCCAGTGGG |
| 223-R1-2 (3926-R1-2) | 4007 | TTGTAATACGACTCAATGGGAGGTTTTGCTATCAAGAAGTCAATGAGG |
| 328.R1-1(4303-R1-1) | 4008 | TTGTAATACGACTCAAGTGCCCGCTCCTCCGACCTCCCTGCGCACC |
| 328-R1-2(4303R1-2) | 4009 | TTGTAATACGACTCAGGGTGGGCAGTGCCCCCTCCTCCGACCTCCCTG |
| 728-R1-1(5795-R1-1) | 4010 | TTGTAATACGACTCACTGCCCTCCAAGAAATAAATTACCCGCAATTACT |
| 832-L1-1(6216-L1-1) | 4011 | TTCTAATACGACTCACACATTCACACCACTCCCCAGAAATCACATG |
| 832-L1-2(6216-L1-2) | 4012 | TTGTAATACGACTCATTCACTTTGACATTCAGAGACACTGGGCAGAAAT |
| 832-R1-1(6216-R1-1) | 4013 | TTGTAATACGACTCATAGTTACTCCCGCCGWTACCCGTG |
| 838-L1-1(6328.L1-1) | 4014 | TTGTAATACGACTCACACAGACCTGGAACCTTCAAAAGCAGTA |
| 951-L1-1(6692-L1-1) | 4015 | TTGTAATACGACTCATTTAAGTACCAAATTTGTCACTC |
| 963-R1-1(6752-R1-1) | 4016 | TTGTAATACGACTCACCCTCCTTTCCCCACCTCAGT |
| 1083-L1-1(7211-L1-1) | 4017 | TTGTAATACGACTCAACCTGCCAGGAAGGTGGGGGCGTGGCAGACGGG |
| 1173-L1-1(7571-L1-1) | 4018 | TTGTAATACGACTCAAGGGCTCCCCTACCCCTAAG |
| 1205-R1-1(7747-R1-1) | 4019 | TTGTAATACGACTCAAATTGCAAATATGCATTTAAATTTAATAATAC |
| 1429-L1-1(8736-L1-1) | 4020 | TTCTAATACCACTCAACTGGCCTCACCTCTAATCACAACCTGCCTGCATT |
| 1611-L1-1(9627-L1-1) | 4021 | TTGTAATACGACTCATTCACTTTCCCTGTGTTAGCTAATGATGC |
| 1617-L1-1(9644-L1-1) | 4022 | TTGTAATACGACTCAGTCAAAACCCTTCAGGTCCACT . |
| 1649-R1-1(9753-R1-1) | 4023 | TTGTAATACGACTCACTCGATTAAACAACAGATACCACTTACAGAC |
| 1688-R1-1 (9955-R1-1) | 4024 | TTCTAATACCACTCAGACCTCCACACCCCATCCCAATCCATAGC |
| ath-MIRI56a-L-1 | 4025 | TTGTAATACGACTCATTGCCTTTGTGTGCTCACTCTCTTCTGTCAG |
| ath-MIR156a-L-2 | 4026 | TTGTAATACGACTCATGCACTTGCTCAGTGTGCTCACTCTTC |
| ath-MIRI56a-R-1 | 4027 | TTGTAATACGACTCAGACCACGAGCACGCGAGAGAAGCAAGTGCA |
| ath-MIR156a-R-2 | 4028 | TTGTAATACGACTCACTGACAGAAAGAGCAGTGAGCACGCAAGAGAAGCA |
| ath-MIRI57a-L-1 | 4029 | TTGTAATACGACTCATCTCATCATCTGTGCCTCTCTATCTTCTGTCAACAC |
| ath-MIRI57a-L-2 | 4030 | TTGTAATACGACTCACCGAATTATCCATCTCTTCTCTCTCTT |
| ath-MIRI57a-L-3 | 4031 | TTCTAATACGACTCACTCCGAATTGTATCTCATCATCTGTGC |
| ath-MIRI57a-R-1 | 4032 | TTGTAATACGACTCAAGGCTAGAGAGCACAAAGGAGTAAGATGCAAAGAA |
| ath-MIRI57a-R-2 | 4033 | TTGTAATACGACTCATGATGACAG4AGGCTAGAGAGCACAAAGGAGTAAG |
| MUSTRP2 | 4034 | TTGTAATACGACTCATCGCACCCAAAGCGAGAATCATACCCCTAGACCAACGAGC |

| | | |
|---|---|---|
| * In the wording "77-R1-2 (10303-R1-2)" for OLIGO NAME, the term in brackets 10303-R1-2 corresponds to the reference used for the name the oligonucleotide probe in Table 4. The number "77" in the term 77-R I-2 corresponds to the number of the corresponding pre-miRNA SEQ ID. | | |

Each specific mapping sequence is preceded by a spacer sequence of 15 nt. This spacer sequence serves to prevent non-specific interactions of the oligonucleotides with the array support. The oligonucleotides are amino-modified at their 5' end to allow their fixing onto the array support.

### Oligonucleotide synthesis

The probes to be spotted onto the array were synthesized according to standard protocols from Operon Biotechnologies, Cologne, Germany, specialised in the synthesis of modified oligonucleotide.

### Array support

As support for the microarray the Nexterion microarray glass slides from Schott were utilised.

### Spotting

Probe concentration is 50 µmol for each probe. The probes were spotted using the Nexterion spotting buffer + 1 % SDS (Sodium dodecyl sulfate) provided by Schott with the array glass support 1% SDS was added to allow larger spots (e.g. 100-150 micro compared to 70-100 micron without SDS).

The spotter utilized was the Q array mini from Gentix. After the deposit of 1 series of spots the spotting needle was washed 5x before spotting the next series of probes. Spotted glass slides were kept at 4°C until use.

### Hybridization

### Protocol

1 Prehybridize the slide with Prehybridization buffer for 30 min at 42°C.
2 Dip the slide into sterile water.
3 Dip the slide into isopropanol.
4 Dry the slide.
5 Heat the hybridization buffer up to 65°C for 5 min immediately before use.
6 Add 3X miRNA hybridization buffer to the labeled miRNA sample (final concentration: 1X).
7 Heat the miRNA hybridization mixture to 95°C for 3 min, then briefly centrifuge.
8 Transfer the miRNA hybridization mixture to a microarray. Be careful to avoid bubbles when placing the cover slip.
9 Place the array into a hybridization cassette (coming chamber). Add diluted salt to maintain humidity in the chamber.
10 Incubate 42°C for 12-16 hr.
11 Remove the array from the cassette and submerge it in low stringency wash solution at room temperature for 1min under agitation. The cover slip will disengage from the slide.
12 Transfer the array to a second low stringency wash solution for 1 mid. under agitation.
13 Transfer the array to a high stringency wash solution for 1min under agitation.
14 Dip the slide into water.
15 Dip the slide into isopropanol.
16 Dry the slide.

### Buffers

Prehybridization buffer
3X SSC
0.1% SDS
10 mg/ml Salmon sperm DNA

3x miRNA hybridization buffer
Solution delivered with mirVana Probe set kit Ambion
Washing solution
Solutions delivered with mirVana Probe set kit Ambion

| | |
|---|---|
| Low stringency wash solution: | 940 ml water |
| | 10 ml detergent concentrate from Ambion |
| | 50 ml Salt concentrate from Ambion |
| | |
| High stringency wash solution | 995 ml water |

**Image Acquisition**
Name of the Scanner Fluoroskan Ascent (Labsystem)

The digital images are acquired from the Axon scanner using the software Genepix. The image is formatted in tif format, defined by an image color depth of 16bits/pixel (1600*1600). Pixels can have intensities values ranging from 0 to 65,535.
Pixels exhibiting the maximum intensity value are "saturated" assigned the value 65,535.
Resolution: Scan array at 10 µm/px
Settings: For hybridization experiments using different fluorescent dyes (e.g Cy5 and Cy3) the photomultiplier tube(PMT) is adjusted to the higher intensity spot. Cy3 is scanned at lower PMT settings.

### Image analysis

A photo-multiplier of a laser scanner digitizes a captured fluorescence for a given "point" of a slide (or screen) and stores a numerical value in a pixel corresponding to that point. A picture composed of such pixels is analyzed during image analysis.

First task for image analysis is to detect the spot position and limits. This stage is often called segmentation. Usually spots are segmented by circles of adaptable or fixed radius. To be reliably segmented and quantified, the spot diameter should be more than 5-6 pixels. Before segmentation an indexing grid is provided giving approximate positions of spots. The segmentation itself detects the limits of spots near the grid nodes. The segmentation must be conducted in rather flexible way because of spotting imperfection or support deformation. Put in other way, the spots lye almost never on perfect rectangular grid.

The second task of image analysis is to quantify spots and export data in a result file. This is relatively easy and well defined task once the spots were determined on the image. Statistics most frequently used to quantify spot intensity are the mean or median of pixels belonging to a spot. Median is more robust than mean value in presence of outlier pixels. In practice, however, there are little differences in results obtained using mean or median.

### miRNA array prehybridization

Incubated the miRNA array in a solution: 3x SSC, SDS.0.1 %, 1 % (V/V) salmon sperm at 10 mg/ml for 30 min at 42°C, then wash the slide by dipping them in water, then isopropanol and dry the slide before the hybridization.

### miRNA array hybridization

Heat the 3X miRNA hybridization buffer to 65°C for 5min immediately before use to dissolve it completely.

Add 3X miRNA hybridization buffer to the labeled miRNA sample for a final. 1X concentration of miRNA hybridization buffer.

Heat the miRNA hybridization mixture to 95°C for 3 min, then briefly-centrifuge.

Transfer the miRNA hybridization mixture to a microarray. Becarefull to the bubble

Place the array into a hybridization cassette (coming chamber). Add diluted salt to maintain humidity in the chamber.

Incubate 42°C for 12-16 hrs.

### Washing

Remove the hybridized miRNA array from the cassette and submerge it in Low stringency wash at room temperature for 1min under agitation. The cover slip will disengage from the slide.

Transfer the hybridized miRNA array to a second low stringency wash clean slide holder for 1min under agitation.

Then transfer the hybridized miRNA array to a high stringency wash clean slide holder for 1min. under agitation.

Dip the slide into water, than isopropanol and dry the slide.

| | |
|---|---|
| Low stringency wash solution: | 940 ml water |
| | 10 ml detergent concentrate from Ambion |
| | 50 ml Salt concentrate from Ambion |
| High stringency wash solution: | 995 ml water |
| | 5 ml Salt concentrate from Ambion |

### Results

Tables 4, 5 and 6 demonstrate the presence of the microRNA transcripts identified by the present methods in cells of different tissues.

| Table 4 | | | | | | |
|---|---|---|---|---|---|---|
| TESTED TISSUES | Well | OLIGO NAME | Position Row / column | Intensity | SEQ ID precursor | SEQ ID mature |
| | 2B13 | MUSTRP2 | 195 | 0.61 | Positive control | |
| | 2B13 | MUSTRP2 | 20 | 0.55 | Positive control | |
| | 2A20 | ath-MIR156a-L-1 | 941 | -0.92 | Negative control | |
| | 2A21 | ath-MIR156a-L-2 | 11 5 | -0.88 | Negative control | |
| | 2B2 | ath-MIR157a-L-3 | 14 17 | -0.72 | Negative control | |
| | 2B4 | ath-MIR157a-R-2 | 1441 | -0.67 | Negative control | |
| | 2022 | 10553-R1-1 | 47 17 | -0.39 | 139 | 1896 |
| | 2D22 | 10S53-R1-1 | 48 17 | -0.35 | 139 | 1896 |
| | 2G4 | 4303-R1-1 | 25 42 | -0.06 | 328 | 2180 |
| | 2G4 | 4303-R1-1 | 2642 | -0.06 | 328 | 2180 |
| LUNG | 2G5 | 4303-R1-2 | 27 6 | -0.4 | 328 | 2181 |
| | 2G5 | 4303-R1-2 | 28 6 | -0.53 | 328 | 2181 |
| | 2K7 | 5195-R1-1 | 27 31 | -0.6 | 728 | 2730 |
| | 2K7 | 5795-R1-1 | 2831 | -0.57 | 728 | 2730 |
| | 2L4 | 6216-L1-1 | 37 43 | 1.52 | 832 | 2870 |
| | 2L4 | 6216-L1-1 | 3843 | 1.54 | 832 | 2870 |
| | 2L5 | 6216-L1-2 | 39 7 | 1.6 | 832 | 2871 |
| | 21.5 | 6216-L1-2 | 40 7 | 1.61 | 832 | 2871 |
| | 2L6 | 6216-R1-1 | 3919 | 1.91 | 832 | 2872 |
| | 2L6 | 6216-R1-1 | 40 19 | 1.88 | 832 | 2872 |
| | 2N9 | 6752-R1-1 | 178 | 0.73 | 963 | 3050 |
| | 2N9 | 6752-R1-1 | 188 | 0.7 | 963 | 3050 |
| | 2019 | 7571-L1-1 | 3332 | -0.14 | 1173 | 3322 |
| | 2019 | 7571-L1-1 | 3432 | -0.15 | 1173 | 3322 |
| | 3C16 | 9627-L1-1 | 3145 | -0.61 | 1611 | 4004 |
| | 3C16 | 9627-L1-1 | 32 45 | -0.54 | 1611 | 4004 |
| | 3D7 | 9955-R1-1 | 39 33 | -0.76 | 1688 | 3993 |
| | 3D7 | 9955-R1-1 | 40 33 | -0.6 | 1688 | 3993 |
| | 2B13 | MUSTRP2 | 195 | 0.16 | Positive control | |
| | 2813 | MUSTRP2 | 20 5 | 0.21 | Positive control | |
| | 2A21 | ath-MIR156a-L-2 | 125 | -0.11 | Negative control | |
| | 282 | ath-MIR157a-L-3 | 1317 | -0.42 | Negative control | |
| | 2B1 | ath-MIR157a-L-2 | 145 | -0.43 | Negative control | |
| | 283 | ath-MIR157a-R-1 | 13 29 | -0.58 | Negative control | |
| | 2A21 | ath-MIR158a-L-2 | 11 5 | -0.65 | Negative control | |
| | 2B1 | ath-MIR157a-L-2 | 135 | -1.01 | Negative control | |
| | 2D22 | 10553-R1-1 | 47 17 | 0.08 | 139 | 1896 |
| | 2022 | 10553-R1-1 | 48 17 | 0.03 | 139 | 1896 |
| | 2G4 | 4303-R1-1 | 25 42 | 0.48 | 328 | 2160 |
| | 2G4 | 4303-R1-1 | 26 42 | 0.47 | 328 | 2180 |
| | 2G5 | 4303-R1-2 | 27 6 | 0.02 | 328 | 2181 |
| | 2G5 | 4303-R1-2 | 28 6 | -0.03 | 328 | 2181 |
| MUSCLE | 2K7 | 5795-R1-1 | 27 31 | -0.05 | 728 | 2730 |
| | 2K7 | 5795-R1-1 | 28 31 | -0.01 | 728 | 2730 |
| | 2L4 | 6216-L1-1 | 37 43 | 1.99 | 832 | 2872 |
| | 2L4 | 6216-L1-1 | 3843 | 2.04 | 832 | 2872 |
| | 2L5 | 6216-L1-2 | 39 7 | 1.98 | 832 | 2872 |
| | 2L5 | 6216-L1-2 | 40 7 | 1.98 | 832 | 2872 |
| | 2L6 | 6216-R1-1 | 39 19 | 2.26 | 832 | 2872 |
| | 2L6 | 6216-R1.1 | 40 19 | 2.25 | 832 | 2872 |
| | 2N9 | 6752-R1-1 | 178 | 1.03 | 963 | 3050 |
| | 2N9 | 6752-R1-1 | 188 | 1.01 | 963 | 3050 |
| | 2019 | 7571-L1-1 | 3332 | 0.02 | 1173 | 3322 |
| | 2019 | 7571-L1-1 | 3432 | 0.08 | 1173 | 3322 |
| | 3C17 | 9644-L1-1 | 339 | -0.04 | 1617 | 3912 |
| | 3C17 | 9644-L1-1 | 349 | -0.04 | 1617 | 3912 |
| | 2B13 | MUSTRP2 | 195 | 0.21 | Positive control | |
| | 2B13 | MUSTRP2 | 20 5 | 0.21 | Positive control | |
| | 2B2 | ath-MIR157a-L-3 | 13 17 | -0.1 | Negative control | |
| | 2B1 | ath-MIR157a-L-2 | 145 | -0.24 | Negative control | |
| | 2A21 | ath-MIR156a-L-2 | 125 | -0.33 | Negative control | |
| | 2B3 | ath-MIR157a-R-1 | 1429 | -0.41 | Negative control | |
| | 2A23 | ath-MIR156a-R-2 | 11 29 | -0.57 | Negative control | |
| | 2A22 | ath-MIR156a-R-1 | 12 17 | -0.63 | Negative control | |
| | 2B1 | ath-MIR157a-L-2 | 135 | -0.65 | Negative control | |
| | 2D22 | 10553-R1-1 | 47 17 | 0.04 | 139 | 1896 |
| LIVER | 2D22 | 10553-R1-1 | 48 17 | 0.01 | 139 | 1896 |
| | 2G4 | 4303-R1-1 | 25 42 | 0.26 | 328 | 2180 |
| | 2G4 | 4303-R1-1 | 2642 | 0.21 | 328 | 2180 |
| | 2L4 | 6216-L1-1 | 3743 | 2.09 | 832 | 2872 |
| | 2L4 | 6216-L1-1 | 38 43 | 2.09 | 832 | 2872 |
| | 2L5 | 6216-L1-2 | 397 | 2.14 | 832 | 2872 |
| | 2L5 | 6216-L1-2 | 407 | 2.16 | 832 | 2872 |
| | 2L6 | 6216-R1-1 | 39 19 | 2.48 | 832 | 2872 |
| | 2L6 | 6216-R1-1 | 40 19 | 2.47 | 832 | 2872 |
| | 2N21 | 7221-L1-1 | 23 8 | 0.12 | 1083 | 3189 |
| | 2N21 | 7221-L1-1 | 24 8 | 0.08 | 1083 | 3189 |
| | 2N9 | 6752-R1-1 | 178 | 1.15 | 963 | 3050 |
| | 2N9 | 6752-R1-1 | 188 | 1.13 | 963 | 3050 |
| | 2019 | 7571-L1-1 | 3332 | 0.04 | 1173 | 3322 |
| | 2019 | 7571-L1-1 | 3432 | 0.03 | 1173 | 3322 |
| | 3B5 | 8738-L1-1 | 159 | -0.01 | 1429 | 3663 |
| | 3B5 | 8736-L1-1 | 169 | 0.04 | 1429 | 3663 |
| | 2B13 | MUSTRP2 | 195 | 0.93 | Positive control | |
| | 2B13 | MUSTRP2 | 20 5 | 0.81 | Positive control | |
| | 282 | ath-MIR157a-L-3 | 1417 | -0.01 | Negative control | |
| | 282 | ath-MIR157a-L-3 | 13 17 | -0.18 | Negative control | |
| | 2A21 | ath-MIR156a-L-2 | 11 5 | -0.22 | Negative control | |
| | 2B1 | ath-MIR157a-L-2 | 135 | -0.23 | Negative control | |
| | 2A24 | ath-MIR157a-L-1 | 11 41 | -0.26 | Negative control | |
| | 2A21 | ath-MIR156a-L-2 | 12 5 | -0.29 | Negative control | |
| | 284 | ath-MIR157a-R-2 | 1341 | -0.31 | Negative control | |
| | 2A23 | ath-MIR156a-R-2 | 1229 | -0.31 | Negative control | |
| | 283 | ath-MIR157a-R-1 | 1329 | -0.31 | Negative control | |
| | 2A23 | ath-MIR156a-R-2 | 1129 | -0.38 | Negative control | |
| | 2B1 | ath-MIR157a-L-2 | 145 | -0.38 | Negative control | |
| | 2A20 | ath-MIR156a-L-1 | 941 | -0.41 | Negative control | |
| HEART_BRAIN Cy5 | 2A20 | ath-MIR156a-L-1 | 1041 | -0.41 | Negative control | |
| | 284 | ath-MIR157a-R-2 | 1441 | -0.43 | Negative control | |
| | 2A22 | ath-MIR156a-R-1 | 1117 | -0.45 | Negative control | |
| | 2G4 | 4303-R1-1 | 25 42 | 0.26 | 328 | 2180 |
| | 2G4 | 4303-R1-1 | 26 42 | 0.3 | 328 | 2180 |
| | 2G5 | 4303-R1-2 | 276 | 0.02 | 328 | 2181 |
| | 2G5 | 4303-R1-2 | 28 6 | 0.02 | 328 | 2181 |
| | 2L4 | 6216-L1-1 | 37 43 | 2.08 | 832 | 2872 |
| | 2L4 | 6216-L1-1 | 38 43 | 2.09 | 832 | 2872 |
| | 2L5 | 6216-L1-2 | 397 | 2.04 | 832 | . 2872 |
| | 2L5 | 6216.Ll.2 | 407 | 2.06 | 832 | 2872 |
| | 2B13 | MUSTRP2 | 195 | 0.18 | Positive control | |
| | 2B13 | MUSTRP2 | 20 5 | 0.09 | Positive control | |
| | 283 | ath-MIR157a-R-1 | 1329 | -0.21 | Negative control | |
| | 2B4 | ath-MIR157a-R-2 | 13 41 | -0.25 | Negative control | |
| | 2B1 | ath-MIR157a-L-2 | 14 5 | -0.31 | Negative control | |
| | 2A24 | ath-MIR157a-L-1 | 12 41 | -0.32 | Negative control | |
| | 2A24 | ath-MIR157a-L-1 | 11 41 | -0.33 | Negative control | |
| | 2B2 | ath-MIR157a-L-3 | 14 17 | -0.34 | Negative control | |
| | 2B3 | ath-MIR157a-R-1 | 14 29 | -0.35 | Negative control | |
| | 2B1 | ath-MIR157a-L-2 | 13 5 | -0.39 | negative control | |
| | 2B2 | ath-MIR157a-L-3 | 13 17 | -0.41 | Negative control | |
| | 2A23 | ath-MIR156a-R-2 | 11 29 | -0.45 | Negative control | |
| | 2A23 | ath-MIR156a-R-2 | 1229 | -0.46 | Negative control | |
| | 2A21 | ath-MIR156a-L-2 | 125 | -0.67 | Negative control | |
| HEART_BRAIN Cy3 | 2D1 | 10303-R1-2 | 37 5 | -0.2 | 77 | 1815 |
| | 2D1 | 10303-R1-2 | 38 5 | -0.16 | 77 | 1815 |
| | 2F2 | 3926-R1-2 | 1318 | -0.17 | 223 | 2026 |
| | 2F2 | 3926-R1-2 | 14 18 | -0.19 | 223 | 2026 |
| | 2L6 | 6216-R1-1 | 39 19 | 2.4 | 832 | 2872 |
| | 2L6 | 6216-R1-1 | 40 19 | 2.42 | 832 | 2872 |
| | 2L8 | 6328-L1-1 | 3943 | 0.4 | 858 | 2900 |
| | 2L8 | 6328-L1-1 | 4043 | 0.07 | 858 | 2900 |
| | 2N3 | 6692-L1-1 | 13 32 | 0 | 951 | 3029 |
| | 2N3 | 6692-L1-1 | 14 32 | 0.03 | 951 | 3029 |
| | 2N9 | 6752-R1-1 | 178 | 1.09 | 963 | 3050 |
| | 2N9 | 6752-R1-1 | 188 | 1.04 | 963 | 3050 |
| | 2O19 | 7571-L1-1 | 3332 | 0.15 | 1173 | 3322 |
| | 2O19 | 7571-L1-1 | 3432 | 0.09 | 1173 | 3322 |
| | 2G4 | 4303-R1-1 | 25 42 | -0.04 | 328 | 2180 |
| | 2G4 | 4303-R1-1 | 2642 | 0.02 | 328 | 2180 |
| | 2L4 | 6216-L1-1 | 37 43 | 1.44 | 832 | 2872 |
| | 2L4 | 6216-L1-1 | 38 43 | 1.44 | 832 | 2872 |
| | 2L5 | 6216-L1-2 | 39 7 | 1.47 | 832 | 2872 |
| | 2L5 | 6216-L1-2 | 40 7 | 1.45 | 832 | 2872 |
| | 2L6 | 6216-R1-1 | 39 19 | 1.8 | 832 | 2872 |
| | 2L6 | 6216-R1-1 | 40 19 | 1.83 | 832 | 2872 |
| | 2N9 | 6752-R1-1 | 17 8 | 0.48 | 963 | 3050 |
| | 2N9 | 6752-R1-1 | 18 8 | 0.5 | 963 | 3050 |
| | 2019 | 7571-L1-1 | 33 32 | -0.16 | 1173 | 3322 |
| | 2019 | 7571-L1-1 | 34 32 | -0.15 | 1173 | 3322 |
| | 2P2 | 7747-R1-1 | 37 20 | -0.1 | 1205 | 3374 |
| | 2P2 | 7747-R1-1 | 38 20 | -0.14 | 1205 | 3374 |
| | 3D3 | 9753-R1-1 | 37 33 | -0.19 | 1649 | 3951 |
| | 3D3 | 9753-R1-1 | 38 33 | -0.12 | 1649 | 3951 |

**TABLE 5-Validated pre-microRNA Sequences**

| **SEQ ID NO:** | **OLIGO ID.** | **Tissue of Validation** |
|---|---|---|
| 3 | 8587-R2-1 | Commercial prostate cell line androgen independent(Cline2and3) |
| | 8587-R2-2 | Commercial prostate cell line immortalized(Cline 5) |
| | 10010-R2-1 | muscle after insulin injection(CTRav) |
| | 10010-R2-2 | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal(Cline7) |
| | | T cells control |
| | | T cells treated with synthetic androgen "R1881" |
| | | Total prostate RNA(Cline1) |
| | | Th1 |
| 5 | 10018-L1-1 | Th1eH |
| | | Th2eH |
| 16 | 10058-R1-1 | Th1eH |
| 22 | 10093-L2-1 | Commercial prostate cell line androgen dependent(Cline4) |
| | 10093-R2-1 | Commercial prostate cell line androgen independent(Cline2and3) |
| | 10093-R2-2 | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Clinel) |
| 32 | 10138-L2-1 | Commercial prostate cell line androgen dependent(Cline4) |
| | 10138-L2-2 | Commercial prostate cell line androgen independent(Cline2and3) |
| | | Commercial prostate cell line immortalized(cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 34 | 10145-L2-1 | T cells control |
| | | T cells treated with synthetic androgen "R 1881" |
| | | Total prostate RNA(Cline1) |
| 35 | 10149-R1-1 | bone_marrow |
| | | Th1eH |
| | | Th2eH |
| 36 | 10154-R1-1 | fat |
| | | testes |
| 60 | 10233-L2-2 | T cells treated with synthetic androgen "R1881" |
| 70 | 10273-L1-1 | Th1eH |
| | 10273-R1-2 | Th1lH |
| | | Th2eH |
| | | Th2lH |
| 77 | 10303-L1-1 | Th2eM |
| 81 | 10324-L1-1 | Th1eH |
| | 10324-L1-2 | Th2eM |
| 82 | 10325-R2-1 | T cells treated with synthetic androgen "R1881" |
| 90 | 10342-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostrate primary cell line cancer(Cline8) |
| | | T cells control |
| | | T cells treated with synthetic androgen "R1881" |
| | | Total prostate RNA(Cline1) |
| 93 | 10347-L1-2 | Th1eH Th2eH |
| 139 | 10553-L1-1 | Th1lH |
| | 10553-R1-1 | Th2lH |
| | | brain |
| | | Commercial prostate cell line androgen dependent(Cline4) |
| | | Commercial prostate cell line androgen independent(Cline2and3) |
| | | Commercial prostate cell line immortalized(Cline 5) |
| | | GFP.RV |
| | | kidney |
| | | muscle after insulin injection(CTRav) |
| | | muscle before insulin injection(CTRav) |
| | | Prostate primary cell line cancer(Cline8) |
| | | Prostate primary cell line normal(Cline7) |
| | | RVp |
| | | RV.Tbet |
| | | spleen |
| | | T cells control |
| | | T cells treated with synthetic androgen "R1881" |
| | | testes |
| | | Thymocytes |
| | | thymus |
| | | Total prostate RNA(Cline1) |
| 141 | 10562-L1-1 | Th1lH |
| | 10562-L1-2 | Th2eM |
| | | Th2lH |
| | | Th21H |
| 151 | 10615-L1-1 | Th2lH |
| | 10615-L1-2 | Th1lH |
| | 10615-R1-1 | Th2eM |
| 152 | 10617-R1-1 | Th1lH Th21H |
| 158 | 4155-L1-1 | ThleM |
| 163 | 3717-L2-1 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline 1) |
| 170 | 3140-R1-1 | Th2lH |
| 175 | 3755-L1-2 | Th1lH |
| | | Th2eH |
| | | Th2eM |
| | | Th2lH |
| 176 | 3758-R2-2 | Commercial prostate cell line androgen dependent(Cline4) |
| | | Commercial prostate celt line androgen independent (Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Thl |
| | | Total prostate RNA(Cline1) |
| 183 | 3784-R1-2 | ThlcH |
| 184 | 3788-R2-2 | T cells treated with synthetic androgen "R1881 |
| 191 | 3817-R1-1 | Th1lH |
| | | Th2eM |
| | | Th2lH |
| 192 | 3819-L2-2 | T cells treated with synthetic androgen "R1881" |
| 200 | 3849-L1-2 | Th1lH |
| | | Th2lH |
| 205 | 3872-L1-1 | testes |
| 219 | 3920-L1-1 | Th1eH |
| | | Th1lH |
| | | Th2eH |
| | | Th2eM |
| | | Th2lH |
| 223 | | fat |
| | | kidney |
| | | T cells control |
| | | T cells treated with synthetic androgen "R1881" |
| | | testes |
| | | Th1lH |
| 224 | 3931-L1-1 | Th2eH |
| 248 | 3995-L2-1 | T cells control |
| | 3995-L2-2 | T cells treated with synthetic androgen "R1881" |
| | | Total prostate RNA(Cline1) |
| | | Commercial prostate cell line androgen independent(Clinc2and3) |
| | | Total prostate RNA(Cline1) |
| 275 | 4088-L1-2 | Th1lM |
| | | Th2lM |
| 276 | 9977-R2-1 | T cells control |
| | 4097-R2-1 | T cells treated with synthetic androgen "R1881" |
| 323 | 4284-L1-1 | Th1lH |
| | 4284-L1-2 | Th2lH |
| | | Th2eH |
| 328 | 4303-R1-1 | bone_marrow |
| | 4303-R1-2 | brain |
| | | colon |
| | | Commercial prostate cell line androgen dependent(Clinc4) |
| | | Commercial prostate cell line androgen independent(Cline2and3) |
| | | Commercial prostate cell line immortalized(Cline 5) |
| | | fat |
| | | GFPp |
| | | GFP.RV |
| | | GFPs |
| | | kidney |
| | | liver |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line nomal(Cline7) |
| | | rat oestrogene stimulated |
| | | RVp |
| | | RVs |
| | | RV.Tbet |
| | | Spem(PACH) |
| | | Sperm(SR) |
| | | spleen |
| | | T cells control |
| | | T cells treated with synthetic androgen "R1881" |
| | | testes |
| | | Thl |
| | | Th1_early_human |
| | | Th1_early_mouse |
| | | Th1cH |
| | | Th1_latc_mouse |
| | | Th2_early_human |
| | | Th2_early_mouse |
| | | Th2eH |
| | | Th2_late_mouse |
| | | Thp |
| | | Thymocytes |
| | | thymus |
| | | Thymus |
| | | Total prostate RNA(Cline1) |
| 344 | 4352-L1-1 | Th1eH |
| | | Th1lH |
| | | Th2eH |
| | | Th2eM |
| | | Th2lH |
| 357 | 4392-L1-2 | bone_marrow |
| 363 | 4417-R1-1 | bone_marrow |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFP.RV |
| | | kidney |
| | | liver |
| | | muscle after insulin injection(GTRav) |
| | | muscle before insulin injection(CTRav) |
| | | Prostate primary cell line cancer(Cline8) |
| | | Prostate primary cell line normal + cancer(Cline6) |
| | | Prostate primary cell line normal(Cline7) |
| | | RV.Tbet |
| | | Sperm(SR) |
| | | spleen |
| | | T cells control |
| | | testes |
| | | Th1 |
| | | Th1_early_human |
| | | Th1_early_mouse |
| | | Th1_late_mouse |
| | | Th2_early_human |
| | | Th2_early_mouse |
| | | Th2_late_mouse |
| | | Thp |
| | | thymus |
| | | Thymus |
| | | Total prostate RNA(Cline1) |
| 366 | 4432-L1-1 | ThleH |
| | 4432-L1-2 | Th2eH |
| | 4432-R1-1 | |
| 385 | 4482-R1-1 | Th1eH |
| 400 | 4528-R1-1 | Th1lH |
| | | Th2lH |
| 406 | 4567-L1-1 | Commercial prostate cell line androgen independent (Cline2and3) |
| | | Commercial prostate cell line immortalized(Cline 5) |
| | | Prostate primary cell line normal(Cline7) |
| | | testes |
| | | Th1eH |
| | | Th2eH |
| | | Total prostate RNA(Cline1) |
| 407 | 5232-L2-1 | Commercial prostate cell line androgen independent(Cline2and3) |
| | | Total prostate RNA(Cline1) |
| 425 | 4638-L1-1 | Th2eM |
| 472 | 4829-R2-1 | Commercial prostate cell line androgen dependent(Cline4) |
| | 4829-R2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R 1881" |
| | | Thl |
| | | Total prostate RNA(Cline1) |
| | | T cells treated with synthetic androgen "R1881" |
| 481 | 4875-R2-2 | Total prostate RNA(Cline1) |
| 489 | 4892-L1-1 | Th1lH |
| | 4892-R1-1 | Th2lH |
| | 4892-R1-2 | |
| 500 | 4939-L1-2 | Th1lH |
| | 4939-R1-2 | Th2lH |
| | | fat |
| | | testes |
| 501 | 4946-L1-1 | Th2cH |
| | 4946-R1-1 | Th1lH |
| | | Th2cM |
| | | Th2lH |
| 503 | 4958-R2-2 | T cells control |
| | | T cells treated with synthetic androgen "R1881" |
| 512 | 4983-L1-1 | Th1lH |
| | | Th2eM |
| | | Th2lH |
| 527 | 5063-L2-2 | T cells treated with synthetic androgen "R1881" |
| 530 | 5070-L1-1 | Th1lH |
| | | Th2eM |
| | | Th2lH |
| 531 | 5071-R2-1 | Commercial prostate cell line androgen independent(Cline2and3) |
| | | T cells control |
| | | T cells treated with synthetic androgen "R1881" |
| | | Total prostate RNA(Cline1) |
| 563 | 5193-R1-1 | testes |
| 608 | 5380-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line normal(Cline7) |
| | | Total prostate RNA(Cline1) |
| 633 | 5478-L2-2 | T cells treated with synthetic androgen "R1881" |
| 640 | 5497-R1-1 | Th1eH |
| | | Th2eH |
| 651 | 5534-L1-1 | Th2eM |
| 653 | 5554-L2-1 | T cells control |
| | 5554-R2-1 | T cells treated with synthetic androgen "R1881" |
| | | Total prostate RNA(Cline1) |
| | | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line normal(Cline7) |
| | | Th1 |
| 664 | 5598-R2-2 | T cells control |
| | | T cells treated with synthetic androgen "R1881" |
| 678 | 5638-L1-1 | Th1eH |
| | 5638-R2-1 | Th2eH |
| | | Commercial prostate cell line androgen independent(Cline2and3) |
| | | muscle after insulin injection(CTRav) |
| | | Total prostate RNA(Cline1) |
| 728 | 5795-R1-1 | bone_marrow |
| | | brain |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFP.RV |
| | | GFPs |
| | | kidney |
| | | liver |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control |
| | | testes |
| | | Th1_early_human |
| | | Th1_early_mouse |
| | | ThleH |
| | | Th1_late_mouse |
| | | Th2_early_human |
| | | Th2_early_mouse |
| | | Th2eH |
| | | Th2_late_mouse |
| | | Thymocytes |
| | | thymus |
| | | Thymus |
| | | Total prostate RNA(Cline1) |
| 744 | 5872-L1-1 | Th1eH |
| | 5872-L1-2 | Th2eH |
| | | Th2eM |
| | | Th1eM |
| 753 | 5903-R1-1 | Th1eH |
| | | Th2eH |
| 784 | 6008-R1-1 | Th1eH |
| | | Th2eH |
| 785 | 6016-R2-1 | Commercial prostate cell line androgen independent(Cline2and3) |
| | 6016-R2-2 | Total prostate RNA(Cline1) |
| 786 | 6023-R1-1 | Th1eH |
| | | Th1lH |
| | | Th2eH |
| | | Th2lH |
| 826 | 6190-R1-1 | Th1eH |
| | | Th2eH |
| 876 | 6404-R1-1 | Th1lH |
| | | Th2lH |
| 903 | 6478-L2-2 | Commercial prostate cell line androgen independent(Cline2and3) |
| | 6478-R2-2 | Commercial prostate cell line immortalized(Cline 5) |
| | | Prostate primary cell line normal(Cline7) |
| | | T cells control |
| | | T cells treated with synthetic androgen "R1881" |
| 909 | 6509-L1-1 | Th1eH |
| | 6509-L1-2 | Th1lH |
| | | Th2cH |
| | | Th2lH |
| | | Th2cM |
| 911 | 6521-L1-1 | Th1lH |
| | 6521-R1-1 | Th2lH |
| | 6521-R1-2 | fat |
| | | testes |
| | | Th1eH |
| 926 | 6584-L1-1 | Commercial prostate cell line androgen independent(Cline2and3) |
| | | Sperm(SR) |
| | | Th2eH |
| | | Total prostate RNA(Cline1) |
| 927 | 6587-R1-1 | Th1eH |
| | | Th2eH |
| 940 | 6647-R2-1 | T cells treated with synthetic androgen "R1881" |
| 942 | 6658-L1-1 | Th1lH |
| | | Th21H |
| 944 | 6664-R2-1 | T cells control |
| | | T cells treated with synthetic androgen "R1881" |
| 946 | 6680-L1-1 | fat |
| | | kidney |
| | | testes |
| 947 | 6681-R1-1 | Commercial prostate cell line androgen dependent(Cline4) |
| | 6681-R2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Total prostate RNA(Cline1) |
| 951 | 6692-L1-1 | Th1eH |
| | | Th2eH |
| 955 | 6712-L2-1 | T cells treated with synthetic androgen "R1881" |
| 963 | 6752-R1-1 | bone_marrow |
| | | brain |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | GFPs |
| | | kidney |
| | | liver |
| | | lung |
| | | muscle |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Spcrm(PACH) |
| | | Spcrm(SR) |
| | | spleen |
| | | T cells control |
| | | T cells treated with synthetic androgen "R1881" |
| | | testes |
| | | Th1 |
| | | Th1_early_human |
| | | Th1_early_mouse |
| | | Th1eH |
| | | Th1eM |
| | | Th1_late_mouse |
| | | Th1lM |
| | | T2 early human |
| | | Th2 _early_mouse |
| | | Th2eH |
| | | Th2eM |
| | | Th2_late_mouse |
| | | Th2lM |
| | | Thp |
| | | Thymocytes |
| | | thymus |
| | | Thymus |
| | | Total prostate RNA(Cline1) |
| 967 | 6762-L1-1 | Th1lH |
| | | Th2lH |
| 975 | 6797-R1-1 | T cells treated with synthetic androgen "R1881" |
| 1055 | 7089-R1-1 | Commercial prostate cell line androgen independent(Cline2and3) |
| | | Commercial prostate cell line immortalized(Cline 5) |
| | | fat |
| | | testes |
| | | Th1_early_human |
| | | Th2_early_human |
| | | Total prostate RNA(Cline1) |
| 1060 | 7104-R2-1 | T cells treated with synthetic androgen "R1881" |
| 1118 | 7356-L2-1 | Total prostate RNA(Cline1) |
| 1129 | 7385-L1-1 | Th2eM |
| | 7385-L1-2 | Th1eH |
| | | Th2eH |
| 1137 | 7421-R2-1 | Commercial prostate cell line androgen independent(Cline2and3) |
| 1145 | 7440-R1-2 | Th1eH |
| | | Th2eH |
| 1160 | 7522-L1-1 | Th1eH |
| | | Th2eH |
| 1161 | 7527-R2-2 | T cells treated with synthetic androgen "R1881" |
| 1166 | 7548-L1-1 | Th1lH |
| | | Th2lH |
| 1173 | 7571-L1-1 | bone_marrow |
| | 7571-R1-1 | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP_RV |
| | | Prostate primary cell line cancer(Cline8) |
| | | Prostate primary cell line normal(Cline7) |
| | | RVs |
| | | RV.Tbet |
| | | Sperm(PACH) |
| | | Sperm(SR) |
| | | spleen |
| | | T cells control |
| | | testes |
| | | Th1 |
| | | Th1_late_mouse |
| | | Th2_late_mouse |
| | | Thymus |
| | | Total prostate RNA(Cline1) |
| 1174 | 7572-R2-1 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1175 | 7573-R1-1 | testes |
| 1196 | 7660-L2-1 | Total prostate RNA(Cline1) |
| 1198 | 7702-L2-1 | Commercial prostate cell line androgen independent(Cline2and3) |
| | 7702-L2-2 | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line notmal(Cline7) |
| | | T cells control |
| | | T cells treated with synthetic androgen "R1881" |
| | | Th1 |
| | | Total prostate RNA(Gline1) |
| 1205 | | kidney |
| | | testes |
| | | Th1eH |
| | | Th2eH |
| 1207 | 7755-R1-1 | Th1lH |
| | | Th2lH |
| 1217 | 7781-R1-1 | Th1eH |
| | | Th2eH |
| 1233 | 7828-L1-1 | Th1lH |
| | 7828-R1-1 | |
| 1238 | 7849-R2-1 | T cells treated with synthetic androgen "R1881" |
| 1251 | 7905-L2-1 | T cells treated with synthetic androgen "R1881" |
| 1267 | 7983-L1-1 | Th1lH |
| 1299 | 8107-R1-1 | Th1lH |
| | | Th2lH |
| 1321 | 8222-R1-2 | Th1lH |
| 1323 | 8231-L1-1 | Th1lH |
| | | Th2cM |
| | | Th2lH |
| 1343 | 8302-L1-1 | Th2H |
| 1352 | 8355-R1-1 | testes |
| 1396 | 8559-R2-1 | T cells treated with synthetic androgen "R1881" |
| 1403 | 8600-L1-1 | Th1eH |
| | 8600-R1-1 | Th2eH |
| 1485 | 9068-R2-1 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line nomial(Cline7) |
| | | T cells control |
| | | T cells treated with synthetic androgen "R1881" |
| | | Total prostate RNA(Cline1) |
| 1531 | 9245-R2-1 | Total prostate RNA(Cline1) |
| 1545 | 9334-L2-2 | T cells treated with synthetic androgen "R1881 " |
| 1547 | 9347-R2-1 | T cells control |
| | | T cells treated with synthetic androgen "R1881" |
| 1559 | 9387-R2-2 | Commercial prostate cell line androgen independent(Cline2and3) |
| | | Total prostate RNA(Cline1) |
| 1586 | 9541-L1-1 | Th1eH |
| 1592 | 9564-R1-1 | Th1eH |
| | | Total prostate RNA(Cline1) |
| 1601 | 9594-R2-1 | Total prostate RNA(Cline1) |
| 1610 | 9625-R1-2 | Th2eM |
| 1611 | 9627-L1-1 | testes |
| 1617 | 9644-L1-1 | Th1eH |
| | 9644-R2-2 | T cells treated with synthetic androgen "R 1881" |
| 1634 | 9700-R1-1 | Th1eH |
| | | Th2eH |
| | | Th2eM |
| 1645 | 9736-R1-1 | Th1eH |
| | | Th2eH |
| 1649 | 9753-R1-1 | Th1eH |
| | 9753-R1-2 | Th2eH |
| 1653 | 9767-L1-1 | Th1eH |
| | 9767-R1-1 | Th2eH |
| 1656 | 9774-R2-1 | Commercial prostate cell line androgen dependent(Cline4) |
| | 9774-R2-2 | Commercial prostate cell line androgen independeat(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1661 | 9794-L1-1 | Th1eH |
| | 9794-L1-2 | Th2eH |
| | 9794-R1-1 | |
| 1667 | 9816-R2-1 | Commercial prostate cell line androgen dependent(Cline4) |
| | | T cells control |
| | | T cells treated with synthetic androgen "R 1881" |
| | | Total prostate RNA(Gline1) |
| 1681 | 9905-L1-1 | Th1eH |
| | | Th2eH |
| 1686 | 9936-R2-1 | T cells treated with synthetic androgen "R1881" |
| 1688 | 9955-L1-1 | Th1eH |
| | 9955-R1-1 | Th1lH |
| | | Th2eH |
| | | Th21H |
| 1693 | 9987-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal(Cline7) |
| | | T cells control |
| | | T cells treated with synthetic androgen "R1881" |
| | | Total prostate RNA(Cline1) |
| 4773 | 5283-L1-1 | Fat |
| | | Testes |
| | | T cells |

| | | |
|---|---|---|
| *Th(1/2)eH = Th(1/12) early human Th(1/2)eM = Th(1/2) early mouse Th(1/2)lH = Th(1/2) late human Th(1/2)lM = Th(1/2) late mouse | | |

**TABLE 6-Validated microRNA Sequences**

| **SEQ ID NO:** | **OLIGO ID.** | **Tissue of Validation** |
|---|---|---|
| 1697 | 8587-R2-1 | Commercial prostate cell line androgen independent(Cline2and3) |
| | 8587-R2-2 | Commercial prostate cell line immortalized(Cline 5) |
| | 10010-R2-1 | muscle after insulin injection(CTRav) |
| | 10010-R2-2 | muscle before insulin injection(CTRav) |
| | | muscle after insulin injection to diabetic subject (DIABav) |
| | | muscle before insulin injection to diabetic subject (DIABav) |
| | | Prostate primary cell line cancer(Cline8) |
| | | Prostate primary cell line nomtal(C.line7) |
| | | T cells control |
| | | T cells treated with synthetic androgen "R1881" |
| | | Th1 |
| | | Total prostate RNA(Glinc1) |
| 1698 | 8587-R2-1 | Commercial prostate cell line androgen independent(Cline2and3) |
| | 8587-R2-2 | Commercial prostate cell line immortalized(Cline 5) |
| | 10010-R2-1 | muscle after insulin injection(CTRav) |
| | 10010-R2-2 | muscle before insulin injection(CTRav) |
| | | muscle after insulin injection to diabetic subject (DIABav) |
| | | muscle before insulin injection to diabetic subject (DIABav) |
| | | Prostate primary cell line cancer(Cline8) |
| | | Prostate primary cell line normal(Cline7) |
| | | T cells control |
| | | T cells treated with synthetic androgen "R1881" |
| | | Th1 |
| | | Total prostate RNA(Cline1) |
| 1700 | 10018-L1-1 | Th1eH |
| | | Th2eH |
| 1719 | 10058-R1-1 | Th1eH |
| 1720 | 10058-R1-1 | Th1eH |
| 1721 | 10058-R1-1 | Th1eH |
| 1730 | 10093-L2-1 | Commercial prostate cell line androgen dependent(Cline4) |
| | 10093-R2-1 | Commercial prostate cell line androgen independent(Cline2and3) |
| | 10093-R2-2 | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DLABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1731 | 10093-L2-1 | Commercial prostate cell line androgen dependent(Cline4) |
| | 10093-R2-1 | Commercial prostate cell line androgen independent(Cline2and3) |
| | 10093-R2-2 | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1732 | 10093-L2-1 | Commercial prostate cell line androgen dependent(Cline4) |
| | 10093-R2-1 | Commercial prostate cell line androgen independent(Cline2and3) |
| | 10093-R2-2 | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin inflection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1744 | 10138-L2-1 | Commercial prostate cell line androgen dependent(Cline4) |
| | 10138-L2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1745 | 10138-L2-1 | Commercial prostate cell line androgen dependent(Cline4) |
| | 10138-L2-2 | Commercial prostate cell line androgen independent(Cline2and3) |
| | | Commercial prostate cell line immortalized(Cline 5) |
| | | muscle after insulin injcction(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1746 | 10145-L2-1 | T cells control |
| | | T cells treated with synthetic androgen "R181" |
| | | Total prostate RNA(Cline1) |
| 1747 | 10149-R-1 | bone marrow |
| | | Th1eH |
| | | Th2eH |
| 1748 | 10149-R1-1 | bone marrow |
| | | Th1eH |
| | | Th2eH |
| 1749 | 10154-R1-1 | fat |
| | | testes |
| 1750 | 10154-R1-1 | fat testes |
| 1784 | 10233-L2-2 | T cells treated with synthetic androgen "R1881" |
| 1785 | 10233-L2-2 | T cells treated with synthetic androgen "R1881" |
| 1805 | 10273-L1-1 | Th1eH |
| | 10273-R1-2 | Th1lH |
| | | Th2eH |
| | | Th2lH |
| 1806 | 10273-L1-1 | Th1eH |
| | 10273-R1-2 | Th1lH |
| | | Th2eH |
| | | Tb2lH |
| 1807 | 10273-L1-1 | Th1eH |
| | 10273-R1-2 | Th1lH |
| | | Th2eH |
| | | Th2lH |
| 1815 | 10303-L1-1 | Th2eM |
| 1816 | 10303-L1-1 | Th2eM |
| 1817 | 10303-L1-1 | Th2eM |
| 1818 | 10303-L1-1 | Th2eM |
| 1824 | 10324-L1-1 | Th1eH |
| | 10324-L1-2 | Th2eM |
| 1825 | 10324-L1-1 | Th1eH |
| | 10324-L1-2 | Th2eM |
| 1826 | 10324-L1-1 | Th1eH |
| | 10324-L1-2 | Th2eM |
| 1827 | 10324-L1-1 | Th1eH |
| | 10324-L1-2 | Th2eM |
| 1828 | 10325-R2-1 | T cells treated with synthetic androgen "R1881" |
| 1837 | 10342-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | T cells control |
| | | T cells treated with synthetic androgen "R1881" |
| 1838 | 10342-R2-2 | Total prostate RNA(Cline1) Commercial prostate cell line androgen dependent(Cline4) |
| | | Commercial prostate cell line androgen independent(Cline2and3) |
| | | Commercial prostate cell line immonalized(Cline 5) |
| | | Prostate primary cell line cancer(Cline8) |
| | | T cells control |
| | | T cells treated with synthetic androgen "R 1881" |
| | | Total prostate RNA(Cline1) |
| 1839 | 10342-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cance(Cline8) |
| | | T cells control |
| | | T cells treated with synthetic androgen "R1881" |
| | | Total prostate RNA(Cline1) |
| 1842 | 10347-L1-2 | Th1eH |
| | | Th2eH |
| 1843 | 10347-L1-2 | Th1eH |
| | | Th2eH |
| 1896 | 10553-L1-1 | Th1lH |
| | 10553-R1-1 | Th21H |
| | | brain |
| | | Commercial prostate cell line androgen dependent(Cline4) |
| | | Commercial prostate cell line androgen indcpendent(Cline2and3) |
| | | Commercial prostate cell line immortatized(Cline 5) |
| | | GFP.RV |
| | | kidney |
| | | muscle after insulin injection(CTRav) |
| | | muscle before insulin injection(CTRav) |
| | | Prostate, primary cell line cancer(Cline8) |
| | | Prostate primary cell line normal(Cline7) |
| | | RVp |
| | | RV.Tbet |
| | | spleen |
| | | T cells control |
| | | T cells treated with synthetic androgen "R1881" |
| | | testes |
| | | Thymocytes |
| | | thymus |
| | | Total prostate RNA(Cline1) |
| 1897 | 10553-L1-1 | Th1lH |
| | 10553-R1-1 | Th21H |
| | | brain |
| | | Commercial prostate cell line androgen dependent(Cline4) |
| | | Commercial prostate cell line androgen independent(Cline2and3) |
| | | Commercial prostate cell line immortalized(Cline 5) |
| | | GFP.RV |
| | | kidney |
| | | muscle after insulin injeclion(CTRav) |
| | | muscle before insulin injection(CTRav) |
| | | Prostate primary cell line cancer(Cline8) |
| | | Prostate primary cell line normal(Cline7) |
| | | RVp |
| | | RV.Tbet |
| | | spleen |
| | | T cells control |
| | | T cells treated with synthetic androgen "R1881" |
| | | testes |
| | | Thymocytes |
| | | thymus |
| | | Total prostate RNA(Cline1) |
| 1898 | 10562-L1-1 | Th1lH |
| | 10562-L1-2 | Th2eM |
| | | Th2lH |
| | | Th2lH |
| 1899 | 10562-L1-1 | Th1lH |
| | 10562-L1-2 | Th2eM |
| | | Th2lH |
| | | Th2lH |
| 1900 | 10562-L1-1 | Th1lH |
| | 10562-L1-2 | Th2eM |
| | | Th2lH |
| | | Th2lH |
| 1911 | 10615-L1-1 | Th2lH |
| | 10615-L1-2 | Th1lH |
| | 10615-R1-1 | Th2eM |
| 1912 | 10615-L1-1 | Th2lH |
| | 10615-L1-2 | Th1lH |
| | 10615-R1-1 | Th2eM |
| 1913 | 16615-L1-1 | Th2lH |
| | 10615-L1-2 | Th1lH |
| | 10615-R1-1 | Th2eM |
| 1914 | 10617-R1-1 | Th1lH |
| | | Th2lH |
| 1915 | 10617-R1-1 | Th1lH |
| | | Th2lH |
| 1916 | 10617-R1-1 | Th1lH |
| | | Th2lH |
| 1917 | 10617-R1-1 | Th1lH |
| | | Th2lH |
| 1925 | 4155-L1-1 | Th1eM |
| 1926 | 4155-L1-1 | Th1eM |
| 1933 | 3717-L2-1 | Commercial prostate cell line androgen dependent(Cline4) . |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1934 | 3717-L2-1 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 1944 | 3740-R1-2 | Th2lH |
| 1945 | 3740-R1-1 | Th2lH |
| 1949 | 3755-L1-2 | Th2lH |
| | | Th2eH |
| | | Th2eM |
| | | Th2lH |
| 1950 | 3755-L1-2 | Th1lH |
| | | Th2eH |
| | | Th2eM |
| | | Th2lH |
| 1951 | 8231-L1-1 | Th1lH |
| | 3755-L1-2 | Th2eH |
| | | Th2eM |
| | | Th2lH |
| 1952 | 3758-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Thl |
| | | Total prostate RNA(Cline1) |
| 1953 | 3758-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Thl |
| | | Total prostate RNA(Cline1) |
| 1954 | 3758-R2-2 | Commercial prostate cell line androgen dependent(Cline4) |
| | | Commercial prostate cell line androgen independent (Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells conlrol |
| | | T cells treated with synthetic androgen "R1881" |
| | | Thl |
| | | Total prostate RNA(Cline1) |
| 1959 | 3784-R1-2 | Th1eH |
| 1960 | 3784-R1-2 | Th1eH |
| 1961 | 3784-R1-2 | Th1eH |
| 1962 | 3784-R1-2 | Th1eH |
| 1963 | 8222-R1-2 | Th1lH |
| 1963 | 3788-R2-2 | T cells treated with synthetic androgen "R1881" |
| 1964 | 8222-R1-2 | Th1lH |
| 1964 | 3788-R2-2 | T cells treated with synthetic androgen "R1881" |
| 1975 | 3817-R1-1 | Th1lH |
| | | Th2eM |
| | | Th2lH |
| 1976 | 3817-R1-1 | Th1lH |
| | | Th2eM |
| | | Th2lH |
| 1977 | 3819-L2-2 | T cells treated with synthetic androgen "R1881" |
| 1978 | 3819-L2-2 | T cells treated with synthetic androgen "R1881" |
| 1979 | 3819-L2-2 | T cells treated with synthetic androgen "R1881" |
| 1990 | 3849-L1-2 | Th1lH |
| | | Th2lH |
| 1991 | 3849-L1-2 | Th1lH |
| | | Th2lH |
| 1992 | 3849-L1-2 | Th1lH |
| | | Th2lH |
| 1993 | 3849-L1-2 | Th1lH |
| | | Th2lH |
| 2000 | 3872-L1-1 | testes |
| 2001 | 3872-L1-1 | testes |
| 2002 | 3872-L1-1 | testes |
| 2025 | 3926-R1-2 | fat |
| | | kidney |
| | | T cells control |
| | | T cells treated with synthetic androgen "R1881" |
| | | testes |
| | | Th1lH |
| 2026 | | fat |
| | | kidney |
| | | T cells control |
| | | T cells treated with synthetic androgen "R1881" |
| | | testes |
| | | Th1lH |
| 2027 | 3931-L1-1 | Th2eH |
| 2028 | 3931-L1-1 | Th2eH |
| 2068 | 3995-L2-1 | T cells control |
| | 3995-L2-2 | T cells treated with synthetic androgen "R1881" |
| | | Total prostate RNA(Cline1) |
| | | Commercial prostate cell line androgen independent(Clinc2and3) |
| | | Total prostate RNA(Cline1) |
| 2108 | 4088-L1-2 | Th1lM |
| | | Th2lM |
| 2109 | 4088-L1-2 | Th1lM |
| | | Th2lM |
| 2110 | 4088-L1-2 | Th1lM |
| | | Th2lM |
| 2111 | 9977-R2-1 | T cells control |
| | | T cells treated with synthetic androgen "R1881" |
| 2111 | 4097-R2-1 | T cells treated with synthetic androgen "R1881" |
| 2112 | 9977-R2-1 | T cells control |
| | | T cells treated with synthetic androgen "R1881" |
| 2112 | 4097-R2-1 | T cells treated with synthetic androgen "R 1881" |
| 2173 | 4284-L1-1 | Th1lH |
| | 4284-L1-2 | Th2lH |
| | | Th2eH |
| 2174 | 4284-L1-1 | Th1lH |
| | 4284-L1-2 | Th2lH |
| | | Th2eH |
| 2175 | 4284-L1-1 | Th1lH |
| | 4284-L1-2 | Th21H |
| | | Th2eH |
| 2180 | 4303-R1-1 | bone_marrow |
| | 4303-R1-2 | brain |
| | | colon |
| | | Commercial prostate cell line androgen dependent(Cline4) |
| | | Commercial prostate cell line androgen independent(Cline2and3) |
| | | Commercial prostate cell line immortatized(Cline 5) |
| | | fat |
| | | GFPp |
| | | GFP.RV |
| | | GFPs |
| | | kidney |
| | | liver |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control |
| | | T cells treated with synthetic androgen "R1881" |
| | | testes |
| | | Th1 |
| | | Th1_earty_human |
| | | Th1_early_mouse |
| | | Th1eH |
| | | Th1_late_mouse |
| | | Th2_early human |
| | | Th2_early_mouse |
| | | Th2eH |
| | | Th2_late_mouse |
| | | Thp |
| | | Thymocytes |
| | | thymus |
| | | Thymus |
| | | Total prostate RNA(Cline1) |
| | | Commercial prostate cell line androgen independent(Cline2and3) |
| | | Commercial prostate cell line immortalized(Cline 5) |
| | | Prostate primary cell line normal(Cline7) |
| | | RV.Tbet |
| | | T cells treated with synthetic androgen "R 1881" |
| | | thymus |
| | | Thymus |
| | | Total prostate RNA(Cline1) |
| 2181 | 4303-R1-1 | bone_marrow |
| | 4303-R1-2 | brain |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | GFPs |
| | | kidney |
| | | liver |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control |
| | | T cells treated with synthetic androgen "R1881" |
| | | testes |
| | | Th1 |
| | | Th1_early_human |
| | | Th1_early_mouse |
| | | Th1eH |
| | | Th1_late_mouse |
| | | Th2 early human |
| | | Th2_early_mouse |
| | | Th2eH |
| | | Th2_late_mouse |
| | | Thp |
| | | Thymocytes |
| | | thymus |
| | | Thymus |
| | | Total prostate RNA(Cline1) |
| | | Commercial prostate cell line androgen independent(Cline2and3) |
| | | Commercial prostate cell line immortalized(Cline5) |
| | | Prostate primary cell line normal(Cline7) |
| | | RV.Tbet |
| | | T cells treated with synthetic androgen "R1881" |
| | | thymus |
| | | Thymus |
| | | Total prostate RNA(Cline1) |
| 2201 | 4352-L1-1 | Th1eH |
| | | Th1lH |
| | | Th2eH |
| | | Th2eM |
| | | Th21H |
| 2202 | 4352-L1-1 | Th1eH |
| | | Th1lH |
| | | Th2eH |
| | | Th2eM |
| | | Th2lH |
| 2203 | 4352-L1-1 | Th1eH |
| | | Th1lH |
| | | Th2eH |
| | | Th2eM |
| | | Th2lH |
| 2219 | 4392-L1-2 | bone_marrow |
| 2220 | 4392-L1-2 | bone_marrow |
| 2221 | 4392-L1-2 | bone_marrow |
| 2228 | 4417-R1-1 | bone_marrow |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | Cat |
| | | GFP.RV |
| | | kidney |
| | | liver |
| | | muscle after insulin injection(CTRav) |
| | | muscle before insulin injection(CTRav) |
| | | Prostate primary cell line cancer(Cline8) |
| | | Prostate primary cell line normal +cancer(Cline6) |
| | | Prostate primary cell line normal(Cline7) |
| | | RV.Tbet |
| | | Sperm(SR) |
| | | spleen |
| | | T cells control |
| | | testes |
| | | Th1 |
| | | Th1_early_human |
| | | Th1_early_mouse |
| | | Th1_late_mouse |
| | | Th2_early_human |
| | | Th2_early_mouse |
| | | Th2_late_mouse |
| | | Thp |
| | | thymus |
| | | Thymus |
| | | Total prostate RNA(Cline1) |
| 2229 | 4417-R1-1 | bone_marrow |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFP.RV |
| | | kidney |
| | | liver |
| | | muscle after insulin injection(CTRav) |
| | | muscle before insulin injection(CTRav) |
| | | Prostate primary cell line cancer(Cline8) |
| | | Prostate primary cell line normal + cancer(Cline6) |
| | | Prostate primary cell line normal(Cline7) |
| | | RV.Tbet |
| | | Sperm(SR) |
| | | spleen |
| | | T cells control |
| | | testes |
| | | Th1 |
| | | Th1_early_human |
| | | Th1_early_mouse |
| | | Th1_late_mouse |
| | | Th2_early_human |
| | | Th2_early_mouse |
| | | Th2_late_mouse |
| | | Thp |
| | | thymus |
| | | Thymus |
| | | Total prostate RNA(Cline1) |
| 2231 | 4432-L1-1 | Th1eH |
| | 4432-L1-2 | Th2eH |
| | 4432-R1-1 | |
| 2232 | 4432-L1-1 | Th1eH |
| | 4432-L1-2 | Th2eH |
| | 4432-R1-1 | |
| 2233 | 4432-L1-1 | Th1eH |
| | 4432-L1-2 | Th2eH |
| | 4432-R1-1 | |
| 2234 | 4432-L1-1 | Th1ef |
| | 4432-L1-2 | Th2eH |
| | 4432-R1-1 | |
| 2260 | 4482-R1-1 | Th1eH |
| 2278 | 4528-R1-1 | Th 1lH |
| | | Th2lH |
| 2279 | 4528-R1-1 | Th1lH |
| | | Th2lH |
| 2280 | 4528-R1-1 | Th1lH |
| | | Th2lH |
| 2313 | 4638-L1-1 | Th2eM |
| 2314 | 4638-L1-1 | Th2eM |
| 2389 | 4829-R2-1 | Commercial prostate cell line androgen dependent(Cline4) |
| | 4829-R2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| | | T cells treated with synthetic androgen "R1881" |
| 2390 | 4829-R2-1 | Commercial prostate cell line androgen dependent(Cline4) |
| | 4829-R2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline 1) |
| | | T cells treated with synthetic androgen "R1881" |
| 2399 | 4875-R2-2 | Total prostate RNA(Cline1) |
| 2408 | 4892-L1-1 | Th1lH |
| | 4892-R1-1 | Th2lH |
| | 4892-R1-2 | |
| 2409 | 4892-L1-1 | Th1lH |
| | 4892-R1-1 | Th2lH |
| | 4892-R1-2 | |
| 2410 | 4892-L1-1 | Th1lH |
| | 4892-R1-1 | Th2lH |
| | 4892-R1-2 | |
| 2427 | 4939-L1-2 | Th1lH |
| | 4939-R1-2 | Th2lH |
| | | fat |
| | | testes |
| 2428 | 4939-L1-2 | Th1lH |
| | 4939-R1-2 | Th2lH |
| | | fat |
| | | testes |
| 2429 | 4939-L1-2 | Th1lH |
| | 4939-R1-2 | Th2lH |
| | | fat |
| | | testes |
| 2430 | 4939-L1-2 | Th1lH |
| | 4939-R1-2 | Th2lH |
| | | fat |
| | | testes |
| 2431 | 4946-L1-1 | Th2eH |
| | 4946-R1-1 | Th1lH |
| | | Th2eM |
| | | Th2lH |
| 2432 | 4946-L1-1 | Th2eH |
| | 4946-R1-1 | Th1lH |
| | | Th2eM |
| | | Tb2lH |
| 2433 | 4946-L1-1 | Th2eH |
| | 4946-R1-1 | Th1lH |
| | | Th2eM |
| | | Th2lH |
| 2434 | 4946-L1-1 | Th2eH |
| | 4946-R1-1 | Th1lH |
| | | Th2eM |
| | | Th2lH |
| 2437 | 4958-R2-2 | T cells control |
| | | T cells treated with synthetic androgen "R1881" |
| 2438 | 4958-R2-2 | T cells control |
| | | T cells treated with synthetic androgen "R1881" |
| 2450 | 4983-L1-1 | Th1lH |
| | | Th2eM |
| | | Th2lH |
| 2451 | 4983-L1-1 | Th1lH |
| | | Th2eM |
| | | Th2lH |
| 2469 | 5063-L2-2 | T cells treated with synthetic androgen "R1881" |
| 2470 | 5063-L2-2 | T cells treated with synthetic androgen "R1881" |
| 2471 | 5063-L2-2 | T cells treated with synthetic androgen "R1881" |
| 2476 | 5070-L1-1 | Th1lH |
| | | Th2eM |
| | | Th2lH |
| 2477 | 5071-R2-1 | Commercial prostate cell line androgen independent(Cline2and3) |
| | | T cells control |
| | | T cells treated with synthetic androgen "R1881" |
| | | Total prostate RNA(Cline1) |
| 2523 | 5193-R1-1 | testes |
| 2532 | 5232-L2-1 | Commercial prostate cell line androgen independent(Cline2and3) |
| | | Total prostate RNA(Ctine1) |
| 2584 | 5380-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line normal(Cline7) |
| | | Total prostate RNA(Cline1) |
| 2585 | 5380-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line normal(Cline7) |
| | | Total prostate RNA(Cline1) |
| 2586 | 5380-R2-2 | Commercial prostate cell line androgen dependent(Cline4) |
| | | Commercial prostate cell line androgen independent(Cline2and3) |
| | | Commercial prostate cell line immortalized(Cline5) |
| | | Prostate primary cell line cancer(Cline8) |
| | | Prostate primary cell line normal(Cline7) |
| | | Total prostate RNA(Cline1) |
| 2587 | 5380-R2-2 | Commercial prostate cell line androgen dependent(Cline4) |
| | | Commercial prostate cell line androgen independent(Cline2and3) |
| | | Commercial prostate cell line immortalized(Cline 5) |
| | | Prostate primary cell line cancer(Cline8) |
| | | Prostate primary cell line normal(Cline7) |
| | | Total prostate RNA(Cline1) |
| 2613 | 5478-L2-2 | T cells treated with synthetic androgen "R1881" |
| 2620 | 5497-R1-1 | Th1eH |
| | | Th2eH |
| 2621 | 5497-R1-1 | Th1eH |
| | | Th2eH |
| 2622 | 5497-R1-1 | Th1eH |
| | | Th2eH |
| 2637 | 5534-L1-1 | Th2eM |
| 2648 | 5598-R2-2 | T cells control |
| | | T cells treated with synthetic androgen "R 1881" |
| 2649 | 5598-R2-2 | T cells control |
| | | T cells treated with synthetic androgen "R1881" |
| 2730 | 5795-R1-1 | bone marrow |
| | | brain |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFP.RV |
| | | GFPs |
| | | kidney |
| | | liver |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Spem(PACH) |
| | | Sperm(SR) |
| | | spleen |
| | | T cells control |
| | | testes |
| | | Th1_early_human |
| | | Th1_early_mouse |
| | | Th1eH |
| | | Th1_late mouse |
| | | Th2_carly_human |
| | | Th2_early_mouse |
| | | Th2eH |
| | | Th2_late_mouse |
| | | Thymocytes |
| | | thymus |
| | | Thymus |
| | | Total prostate RNA(Cline1) |
| 2754 | 5872-L1-1 | Th1eH |
| | 5872-L1-2 | Th2eH |
| | | Th2eM |
| | | *Th1eM |
| 2755 | 5872-L1-1 | Th1eH |
| | 5872-L1-2 | Th2eH |
| | | Th2eM |
| | | Th1eM |
| 2756 | 5872-L1-1 | Th1eH |
| | 5872-L1-2 | Th2eH |
| | | Th2eM |
| | | Th1eM |
| 2771 | 5903-R1-1 | Th1eH |
| | | Th2eH |
| 2809 | 6008-R1-1 | Th1eH |
| | | Th2eH |
| 2810 | 6008-R1-1 | Th1eH |
| | | Th2eH |
| 2811 | 6016-R2-1 | Commercial prostate cell line androgen Independent(Cline2and3) |
| | 6016-R2-2 | Total prostate RNA(Cline1) |
| 2812 | 6016-R2-1 | Commercial prostate cell line androgen independent(Cline2and3) |
| | 6016-R2-2 | Total prostate RNA(C-line1) |
| 2813 | 6016-R2-1 | Commercial prostate cell line androgen independent(Cline2and3) |
| | 6016-R2-2 | Total prostate RNA(Cline1) |
| 2814 | 6023-R1-1 | Th1eH |
| | | Th1lH |
| | | Th2eH |
| | | Th2lH |
| 2815 | 6023-R1-1 | Th1eH |
| | | Th1lH |
| | | Th2eH |
| | | Th2lH |
| 2862 | 6190-R1-1 | Th1eH |
| | | Th2cH |
| 2863 | 6190-R1-1 | Th1eH |
| | | Th2eH |
| 2864 | 6190-R1-1 | Th1eH |
| | | Th2eH |
| 2928 | 6404-R1-1 | Th1lH |
| | | Th2lH |
| 2929 | 6404-R1-1 | Th1lH |
| | | Th2lH |
| 2958 | 6478-L2-2 | Commercial prostate cell line androgen indepeadent(Cline2and3) |
| | 6478-R2-2 | Commercial prostate cell line immortalized(Cline5) |
| | | Prostate primary cell line normal(Cline7) |
| | | T cells control |
| | | T cells treated with synthetic androgen "R1881" |
| 2959 | 6478-L2-2 | Commercial prostate cell line androgen independent(Cline2and3) |
| | 6478-R2-2 | Commercial prostate cell line immortalized(Cline 5) |
| | | Prostate primary cell line normal(Ctine7) |
| | | T cells control |
| | | T cells treated with synthetic androgen "R 1881" |
| 2960 | 6478-L2-2 | Commercial prostate cell line androgen independent(Cline2and3) |
| | 6478-R2-2 | Commercial prostate cell line immortalized(Cline 5) |
| | | Prostate primary cell line normal(Cline7) |
| | | T cells control |
| | | T cells treated with synthetic androgen "R1881" |
| 2961 | 6478-L2-2 | Commercial prostate cell line androgen independent(Cline2and3) |
| | 6478-R2-2 | Commercial prostate cell line immortalized(Cline 5) |
| | | Prostate primary cell line normal(Cline7) |
| | | T cells control |
| | | T cells treated with synthetic androgen "R1881" |
| 2966 | 6509-L1-1 | Th1eH |
| | 6509-L1-2 | Th1lH |
| | | Th2eH |
| | | Th2lH |
| | | Th2eM |
| 2967 | 6509-L1-1 | Th1eH |
| | 6509-L1-2 | Th1lH |
| | | Th2eH |
| | | Th2lH |
| | | Th2eM |
| 2968 | 6509-L1-1 | Th1eH |
| | 6509-L1-2 | Th1lH |
| | | Th2eH |
| | | Th2lH |
| | | Th2eM |
| 2971 | 6521-L1-1 | Th1lH |
| | 6521-R1-1 | Th2lH |
| | 6521-R1-2 | fat |
| | | testes |
| | | Th1eH |
| 2972 | 6521-L1-1 | Th1lH |
| | 6521-R1-1 | Th2lH |
| | 6521-R1-2 | fat |
| | | testes |
| | | Th1eH |
| 2973 | 6521-L1-1 | Th1lH |
| | 6521-R1-1 | Th2lH |
| | 6521-R1-2 | fat |
| | | testes |
| | | Th1eH |
| 2974 | 6521-L1-1 | Th1lH |
| | 6521-R1-1 | Th2lH |
| | 6521-R1-2 | fat |
| | | testes |
| | | Th1eH |
| 2999 | 6584-L1-1 | Commercial prostate cell line androgen independent(Cline2and3) |
| | | Sperm(SR) |
| | | Th2eH |
| | | Total prostate RNA(Cline1) |
| 3000 | 6587-R1-1 | Th1eH |
| | | Th2eH |
| 3001 | 6587-R1-1 | Th1eH |
| | | Th2eH |
| 3015 | 6647-R1-1 | T cells treated with synthetic androgen "R1881" |
| 3017 | 6658-L1-1 | Th1lH |
| | | Th2lH |
| 3018 | 6658-L1-1 | Th1lH |
| | | |
| 3019 | 6658-L1-1 | Th2lH Th1lH |
| | | Th2lH |
| 3021 | 6664-R2-1 | T cells control |
| | | T cells treated with synthetic androgen "R1881" |
| 3023 | 6680-L1-1 | fat |
| | | kidney |
| | | testes |
| 3024 | 6680-L1-1 | fat |
| | | kidney |
| | | testes |
| 3025 | 6681-R1-1 | Commercial prostate cell line androgen dependent(Cline4) |
| | 6681-R2-2 | Commercial prostate cell line androgen independent(Cline2and3) |
| | | Commercial prostate cell line immortalized(Cline5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Total prostate RNA(Cline1) |
| 3026 | 6681-R1-1 | Commercial prostate cell line androgen dependent(Cline4) |
| | 6681-R2-2 | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Total prostate RNA(Cline1) |
| 3027 | 6681-R1-1 | Commercial prostate cell line androgen dependent(Cline4) |
| | 6681-R2-2 | Commercial prostate cell line androgen independent(Cline2and3) |
| | | Commercial prostate cell line immortalized(Cline5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Total prostate RNA(Cline1) |
| 3029 | 6692-L1-1 | Th1ef |
| | | Th2eH |
| 3030 | 6692-L1-1 | Th1eH |
| | | Th2eH |
| 3038 | 6712-L2-1 | T cells treated with synthetic androgen "R1881" |
| 3039 | 6712-L2-1 | T cells treated with synthetic androgen "R1881" |
| 3048 | 6752-R1-1 | bone marrow |
| | | brain |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFPRV |
| | | GFPs |
| | | kidney |
| | | liver |
| | | lung |
| | | muscle |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control |
| | | T cells treated with synthetic androgen "R 1881 |
| | | testes |
| | | Th1 |
| | | Th1_early_ humnan |
| | | Th1_early_mouse |
| | | Th1eH |
| | | Th1eM |
| | | Th1_late_mouse |
| | | Th1lM |
| | | Th2_early_human |
| | | Th2_early_mouse |
| | | Th2eH |
| | | Th2eM |
| | | Th2 late mouse |
| | | Th2lM |
| | | Thp |
| | | Thymocytes |
| | | thymus |
| | | Thymus |
| | | Total prostate RNA(Cline 1) |
| 3049 | 6752-R1-1 | bone_marrow |
| | | brain |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | GFPs |
| | | kidney |
| | | liver |
| | | lung |
| | | muscle |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control |
| | | T cells treated with synthetic androgen "R1881" |
| | | testes |
| | | rh1 |
| | | Th1_early_human |
| | | Th1_early_mouse |
| | | Th1eH |
| | | Th1eM |
| | | Th1_late_mouse |
| | | Th1lM |
| | | Th2_earty_human |
| | | Th2_early_mouse |
| | | Th2eH |
| | | Th2eM |
| | | Th2_late_mouse |
| | | Th2lM |
| | | Thp |
| | | Thymocytes |
| | | thymus |
| | | Thymus |
| | | Total prostate RNA(Cline1) |
| 3050 | 6752-R1-1 | bone_marrow |
| | | brain |
| | | colon |
| | | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | GFPs |
| | | kidney |
| | | liver |
| | | lung |
| | | muscle |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) . |
| | | Prostate primary cell line cancer(Cline8) |
| | | Prostate primary cell line normal + cancer(Cline6) |
| | | Prostate primary cell line normal(Cline7) |
| | | rat oestrogene stimulated |
| | | RVp |
| | | RVs |
| | | RV.Tbet |
| | | Sperm(PACH) |
| | | Sperm(SR) |
| | | spleen |
| | | T cells control. |
| | | T cells treated with synthetic androgen "R1881" |
| | | testes |
| | | Th1 |
| | | Th1_early_human |
| | | Th1_early_mouse |
| | | Th1eH |
| | | Th1eM |
| | | Th1_late_mouse |
| | | Th1lM |
| | | Th2_early_human |
| | | Th2 _early_mouse |
| | | Th2eH |
| | | Th2eM |
| | | Th2_late_mouse |
| | | Th2lM |
| | | Thp |
| | | Thymocytes |
| | | thymus |
| | | Thymus |
| | | Total prostate RNA(Cline1) |
| 3052 | 6762-L1-1 | Th1lH |
| | | Th2lH |
| 3053 | 6762-L1-1 | Th1lH |
| | | Th2lH |
| 3054 | 6762-L1-1 | Th1lH |
| | | Th2lH |
| 3061 | 6797-R1-1 | T cells treated with synthetic androgen "R1881" |
| 3062 | 6797-R1-1 | T cells treated with synthetic androgen "R1881" |
| 3162 | 7104-L1-1 | T cells treated with synthetic androgen "R1881" |
| 3232 | 7356-L2-1 | Total prostate RNA(Cline1) |
| 3233 | 7356-L2-1 | Total prostate RNA(Cline1) |
| 3249 | 7385-L1-1 | Th2eM |
| | 7385-L1-2 | Th1eH |
| | | Th2eH |
| 3250 | 7385-L1-1 | Th2eM |
| | 7385-L1-2 | Th1eH |
| | | Th2eH |
| 3261 | 7421-R2-1 | Commercial prostate cell line androgen independent(Cline2and3) |
| 3271 | 7440-R1-2 | Th1eH |
| | | Th2eH |
| 3272 | 7440-R1-2 | Th1eH |
| | | Th2eH |
| 3273 | 7440-R1-2 | Th1eH. |
| | | Th2eH |
| 3274 | 7440-R1-2 | Th1eH |
| | | Th2eH |
| 3300 | 7522-L1-1 | Th1eH |
| | | Th2eH |
| 3301 | 7522-L1-1 | Th1eH |
| | | Th2eH |
| 3302 | 7527-R2-2 | T cells treated with synthetic androgen "R 1881" |
| 3303 | 7527-R2-2 | T cells treated with synthetic androgen "R1881" |
| 3304 | 7527-R2-2 | T cells treated with synthetic androgen "R1881" |
| 3320 | 7571-L1-1 | bone_marrow |
| | 7571-R1-1 | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | Prostate primary cell line cancer(Cline8) |
| | | Prostate primary cell line normal(Cline7) |
| | | RVs |
| | | RV.Tbet |
| | | Sperm(PACH) |
| | | Sperm(SR) |
| | | spleen |
| | | T cells control |
| | | testes |
| | | Th1 |
| | | Th1_late_mouse |
| | | Th2_late_mouse |
| | | Thymus |
| | | Total prostate RNA(Cline1) |
| 3321 | 7571-L1-1 | bone_marrow |
| | 7571-R1-1 | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | Prostate primary cell line cancer(Cline8) |
| | | Prostate primary cell line normal(Cline7) |
| | | RVs |
| | | RV.Tbet |
| | | Sperm(PACH) |
| | | Sperm(SR) |
| | | spleen |
| | | T cells control |
| | | testes |
| | | Th1 |
| | | Th1_late_mouse |
| | | Th2_late_mouse |
| | | Thymus |
| | | Total prostate RNA(cline1) |
| 3322 | 7571-L1-1 | bone marrow |
| | 7571-R1-1 | Commercial prostate cell line androgen dependent(Cline4) |
| | | Commercial prostate cell line androgen independent(Cline2and3) |
| | | Commercial prostate cell line immortalized(Cline 5) |
| | | fat |
| | | GFPp |
| | | GFP.RV |
| | | Prostate primary cell line cancer(Cline8) |
| | | Prostate primary cell line normal(Cline7) |
| | | RVs |
| | | RV.Tbet |
| | | Sperm(PACH) |
| | | Sperm(SR) |
| | | spleen |
| | | T cells control |
| | | testes |
| | | Th1 |
| | | Th1_late_mouse |
| | | Th2_late_mouse |
| | | Thymus |
| | | Total prostate RNA(Cline1) |
| 3323 | 7572-R2-1 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R 1881" |
| | | Th1 |
| | | Total prostate RNA(Cline1) |
| 3324 | 7572-R2-1 | Commercial prostate cell line androgen dependent(Cline4) |
| | | Commercial prostate cell line androgen independent(Cline2and3) |
| | | Commercial prostate cell line immortalized(Cline5) |
| | | muscle after insulin injection(CTRav) |
| | | muscle after insulin injection to diabetic subject (DIABav) |
| | | muscle before insulin injection(GTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 3325 | 7572-R2-1 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle after insulin injection to diabetic subject (DIABav) |
| | | muscle before insulin injection(CTRav) |
| | | muscle before insulin injection to diabetic subject (DIABav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 3326 | 7573-R1-1 | testes |
| 3327 | 7573-R1-1 | testes |
| 3328 | 7573-R1-1 | testes |
| 3329 | 7573-R1-1 | testes |
| 3358 | 7660-L2-1 | Total prostate RNA(Cline1) |
| 3359 | 7660-L2-1 | Total prostate RNA(Cline1) |
| 3360 | 7660-L2-1 | Total prostate RNA(Cline1) |
| 3363 | 7702-L2-1 | Commercial prostate cell line androgen independent(Cline2and3) |
| | 7702-L2-2 | Commercial prostate cell line immortalized(Cline 5) |
| | | muscle after insulin injection(CTRav) |
| | | muscle after insulin injection to diabetic subject (DLABav) |
| | | muscle before insulin injection(CTRav) |
| | | muscle before insulin injection to diabetic subject (DIABav) |
| | | Prostate primary cell line cancer(Cline8) |
| | | Prostate primary cell line normal(Cline7) |
| | | T cells control |
| | | T cells treated with synthetic androgen "R1881" |
| | | Th1 |
| | | Total prostate RNA(Cline1) |
| 3372 | 7747-R1-1 | kidney |
| | | testes |
| | | Th1eH |
| | | Th2eH |
| 3373 | 7747-R1-1 | kidney |
| | | testes |
| | | Th1eH |
| | | Th2eH |
| 3374 | 7747-R1-1 | kidney |
| | | testes |
| | | Th1eH |
| | | Th2eH |
| 3375 | 7747-R1-1 | kidney |
| | | testes |
| | | Th1eH |
| | | Th2eH |
| 3378 | 7755-R1-1 | Th1lH |
| | | Th2lH |
| 3379 | 7755-R1-1 | Th1lH |
| | | Th2lH |
| 3391 | 7781-R1-1 | Th1eH |
| | | Th2eH |
| 3392 | 7781-R1-1 | Th1eH |
| | | Th2eH |
| 3416 | 7828-L1-1 | Th1lH |
| | 7828-R1-1 | Th2lH |
| 3417 | 7828-L1-1 | Th1lH |
| | 7828-R1-1 | Th2lH |
| 3422 | 7849-R2-1 | T cells treated with synthetic androgen "R1881" |
| 3423 | 7849-R2-1 | T cells treated with synthetic androgen "R1881" |
| 3424 | 7849-R2-1 | T cells treated with synthetic androgen "R1881" |
| 3440 | 7905-L2-1 | T cells treated with synthetic androgen "R1881" |
| 3441 | 7905-L2-1 | T cells treated with synthetic androgen "R1881" |
| 3442 | 7905-L2-1 | T cells treated with synthetic androgen "RL881" |
| 3461 | 7983-L1-1 | Th1lH |
| 3497 | 8107-R1-1 | Th1lH |
| | | Th2lH |
| 3498 | 8107-R1-1 | Th1lH |
| | | Th2lH |
| 3499 | 8107-R1-1 | Th1lH |
| | | Th2lH |
| 3528 | 8231-L1-1 | Th1lH |
| | | Th2eM |
| | | Th2lH |
| 3555 | 8302-L1-1 | Th2lH |
| 3556 | 8302-L1-1 | Th2lH |
| 3557 | 8302-L1-1 | Th2lH |
| 3567 | 8355-R1-1 | testes |
| 3619 | 8559-R2-1 | T cells treated with synthetic androgen "R1881" |
| 3620 | 8559-R2-1 | T cells treated with synthetic androgen "R1881" |
| 3621 | 8559-R2-1 | T cells treated with synthetic androgen "R1881" |
| 3627 | 8600-L1-1 | Th1eH |
| | 8600-R1-1 | Th2eH |
| 3628 | 8600-L1-1 | Th1eH |
| | 8600-R1-1 | Th2eH |
| 3786 | 9245-R2-1 | Total prostate RNA(Cline1) |
| 3804 | 9334-L2-2 | T cells treated with synthetic androgen "R1881" |
| 3805 | 9334-L2-2 | T cells treated with synthetic androgen "R1881" |
| 3806 | 9334-L2-2 | T cells treated with synthetic androgen "R1881" |
| 3807 | 9334-L2-2 | T cells treated with synthetic androgen "R1881" |
| 3808 | 9347-R2-1 | T cells control |
| | | T cells treated with synthetic androgen "R1881" |
| 3825 | 9387-R2-2 | Commercial prostate cell line androgen independent(Cline2and3) |
| | | Total prostate RNA(Cline1) |
| 3862 | 9541-L1-1 | Th1eH |
| 3863 | 9541-L1-1 | Th1eH |
| 3870 | 9564-R1-1 | Th1eH |
| | | Total prostate RNA(Gline1) |
| 3871 | 9564-R1-1 | Th1eH |
| | | Total prostate RNA(Cline1) |
| 3880 | 9594-R2-1 | Total prostate RNA(Cline1) |
| 3881 | 9594-R2-1 | Total prostate RNA(Cline1) |
| 3882 | 9594-R2-1 | Total prostate RNA(Cline1) |
| 3896 | 9625-R1-2 | Th2eM |
| 3897 | 9625-R1-2 | Th2eM |
| 3898 | 9625-R1-2 | Th2eM |
| 3912 | 9644-L1-1 | Th1eH |
| | 9644-R2-2 | T cells treated with synthetic androgen "R 1881 |
| 3931 | 9700-R1-1 | Th1eH |
| | | Th2eH |
| | | Th2eM |
| 3943 | 9736-R1-1 | Th1eH |
| | | Th2eH |
| 3950 | 9753-R1-1 | Th1eH |
| | 9753-R1-2 | Th2eH |
| 3951 | 9753-R1-1 | Th1eH |
| | 9753-R1-2 | Th2eH |
| 3956 | 9767-L1-1 | Th1eH |
| | 9767-R1-1 | Th2eH |
| 3957 | 9767-L1-1 | Th1eH |
| | 9767-R1-1 | Th2eH |
| 3959 | 9774-R2-1 | Commercial prostate cell line androgen dependent(Cline4) |
| | 9774-R2-2 | Commercial prostate cell line androgen independent(Cline2and3) |
| | | Commercial prostate cell line immortalized(Cline 5) |
| | | muscle after insulin injection(CTRav): |
| | | muscle before insulin injection(CTRav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 3960 | 9774-R2-1 | Commercial prostate cell line androgen dependent(Cline4) |
| | 9774-R2-2 | Commercial prostate cell line androgen independent(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal + cancer(Cline6) |
| | | Prostate primary cell line normal(Cline7) |
| | | T cells control |
| | | T cells treated with synthetic androgen "R1881" |
| | | Th1 |
| | | Total prostate RNA(Cline1) |
| 3966 | 9794-L1-1 | Th1eH |
| | 9794-L1-2 | Th2eH |
| | 9794-R1-1 | |
| 3967 | 9744-L1-1 | Th1eH |
| | 9794-L1-2 | Th2eH |
| | 9794-R1-1 | |
| 3968 | 9794-L1-1 | Th1eH |
| | 9794-L1-2 | Th2eH |
| | 9794-R1-1 | |
| 3973 | 9816-R2-1 | Commercial prostate cell line androgen dependent(Cline4) |
| | | T cells control |
| | | T cells treated with synthetic androgen "R1881" |
| | | Total prostate RNA(Cline1) |
| 3974 | 9816-R2- | Commercial prostate cell line androgen dependent(Cline4) |
| | | T cells control |
| | | T cells treated with synthetic androgen "R1881" |
| | | Total prostate RNA(Cline1) |
| 3975 | 9816-R2-1 | Commercial prostate cell line androgen dependent(Cline4) |
| | | T cells control |
| | | T cells treated with synthetic androgen "R1881" |
| | | Total prostate RNA(Cline1) |
| 3976 | 9816-R2-1 | Commercial prostate cell line androgen dependent(Cline4) |
| | | T cells control |
| | | T cells treated with synthetic androgen "R1881" |
| | | Total prostate RNA(Cline1) |
| 3986 | 9905-L1-1 | Th1eH |
| | | Th2eH |
| 3987 | 9905-L1-1 | Th1eH |
| | | Th2eH |
| 3991 | 9936-R2-1 | T cells treated with synthetic androgen "R1881" |
| 3992 | 9936-R2-1 | T cells treated with synthetic androgen "R 1881" |
| 3993 | 9955-L1-1 | Th1eH |
| | 9955-R1-1 | Th1lH |
| | | Th2eH |
| | | Th2lH |
| 3994 | 9955-L1-1 | Th1eH |
| | 9955-R1-1 | Th1lH |
| | | Th2eH |
| | | Th2lH |
| 4001 | 9987-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal(Cline7) |
| | | T cells control |
| | | T cells treated with synthetic androgen "R1881" |
| | | Total prostate RNA(Cline1) |
| 4002 | 9987-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal(Cline7) |
| | | T cells control |
| | | T cells treated with synthetic androgen "R1881" |
| | | Total prostate RNA(Cline1) |
| 4003 | 9987-R2-2 | Commercial prostate cell line androgen dependent(Cline4) |
| | | Commercial prostate cell line androgen independent(Cline2and3) |
| | | Commercial prostate cell line immortalized(Cline 5) |
| | | muscle after insulin injection(CTRav) |
| | | muscle before insulin injection(CTRav) |
| | | Prostate primary cell line cancer(Cline8) |
| | | Prostate primary cell line normal(Cline7) |
| | | T cells control |
| | | T cells treated with synthetic androgen "R1881" |
| | | Total prostate RNA(Cline1) |
| 4004 | 9627-L1-1 | testes |

### REFERENCES

Bartel P., Cell, 116, 281-297, 2004.
Baskerville and al., RNA., Mar;11(3):241-7, 2005.
Bejerano G. and al., Bioinformatics, 4;20 Suppl 1:140-148, 2004.
Bentwich and al., Nat Genet., Jul;37(7):766-70 2005.
Berezikov and al., Cell., Jan 14; 120(1):21-4, 2005.
Blume and al., Exp Cell Res., 288(1):131-42, 2003.
Brennecke and al., Cell., 113(1):25-36, 2003.
Brennecke and al., PLoS Biol., Mar;3(3):e85, 2005.
Burgler and al., BMC Genomics., Jun 8;6(1):88, 2005.
Calin and al., Proc Natl Acad Sci, U S A, 26;99(24):15524-9, 2002.
Calin et al., Proc Natl Acad Sci, U S A, 10;101(32):11755-60, 2004.
Calin and al., 2004.
Chen and al., Science, 303(5654):83-6, 2004.
Doench and al., Genes Dev., Mar 1;18(5):504-11, Epub, Mar 10, 2004.
Dostie J, Mourelatos Z, Yang M, Sharma A, Dreyfuss G., "Numerous microRNPs in neuronal cells containing novel microARNs", RNA, 9(2):180-6, 2003. Erratum in: RNA, 9(5):631-2, 2003.
Enright and al., Genome Biol., 5(1):R1, 2003.
Esau and al., J Biol Chem., 10;279(50):52361-5, 2004.
Grun D and al., PLoS Comput Biol., Jun; 1(1):e 13, 2005.
He and al., Nat Rev Genet., 5(7):522-31, Review, 2004.
He L. and al., Nature, Jun 9;435(7043):828-33, 2005.
Houbaviy and al., Dev Cell. 5(2):351-8, 2003.
Ji and aL, Oncogene, 11;22(39):8031-41, 2003.
John and al., Biochem Biophys Res Commun., 17;322(2):403-10, 2004.
Kawasaki and al., Proc Natl Acad Sci, U S A, 6;101(1):360-5, 2004.
Kiriakidou and al., Genes Dev., May 15;18(10):1165-78, 2004.
Krek and al., Nat Genet, May;37(5):495-500, 2005.
Krichevsky and al., RNA, 9(10):1274-81, 2003.
Kuwabara, Cell., 19; 116(6):779-93, 2004.
Lai and al., Nat Genet., Apr;30(4):3634, 2002.
Lai and al., Genome Biol., 4(7):R42, 2003.
Lewis and al., Cell., Dec 26;115(7):787-98, 2003.
Lewis and al., Cell., Jan 14;120(1):15-20, 2005.
Lim and al., Science, Mar 7;299(5612):1540, 2003.
Lim and al., Nature, 30, 2005.
Lu and al., Nature, Jun 9;435(7043):834-8 2005.
Mattick and al., Bioessays, 25(10):930-9, 2003.
Metzler and al., Genes Chromosomes Cancer, 39(2):167-9, 2004.
Michael and al., Mol Cancer Res., 1(12):882-91, 2003.
Miska and al., Genome Biol., 5(9):R68, 2004.
Multer-Tidow et al.. Lung Cancer, 45 Suppl 2:S 145-50, 2004.
Nam and aL, Nucleic Acids Res., Jun 24;33(11);3570-81, 2005.
O'Donnell and al., Nature, Jun 9;435(7043):839-43, 2005.
Poy and al., Nature, 11; 432(7014):226-30, 2004.
Rehmsmeier and al., RNA, Oct; 10(10): 1507-17, 2004.
Reinhart and al.. Nature, 403(6772):901-6, 2000.
Sempere and al., Genome BioL, 5(3):R13, 2004.
Stark and al., PLoS Biol., Dec;1(3):E60, 2003.
Takamizawa J. and al., Cancer Res., 1;64(11):3753-6, 2004.
Tinzl and al., Trends Genet., 20(12):617-24, 2004, Review.

## Claims

1. A computer-implemented method of identifying microRNA precursor candidates in non-coding and coding regions of a genome, comprising:
receiving at least a first and a second genome from two different species, each containing a plurality of sequences including highly repetitive DNA;
masking a first set of highly repetitive DNA sequences in the first genome, wherein the first set includes SINE and LINE interspersed repeat sequences, but does not include at least 75% of other interspersed repeat sequences;
masking a second set of highly repetitive DNA sequences in the second genome, wherein the second set includes SINE and LINE interspersed repeat sequences, but does not include at least 75% of the other interspersed repeat sequences;
comparing the masked first genome to the masked second genome to determine pairs of sequences that are homologous between the two genomes;
creating a list of microRNA precursor candidates from the homologous pairs; and
analyzing the list of candidates to:
eliminate sequences having less than 60 nucleotides; and
identify sequences that have a stem-loop secondary structure with a 5' strand stem having from 20 to 45 nucleotides and with a 3' strand stem having from 20 to 45 nucleotides; and
remove, from the list, sequences not having said stem-loop secondary structure.

2. The computer-implemented method of claim 1, wherein the other interspersed repeat sequences include processed pseudogenes, retrotranscripts, DNA transposons, and retrovirus retrotransposons.

3. The computer-implemented method of claim 1, further comprising:
creating a plurality of assemblies, each created from at least one pair of homologous sequences, wherein at least one assembly is created from a first group of at least two pairs of homologous sequences, wherein each pair of the first group has one sequence in common;
clustering the assemblies into a plurality of clusters, wherein each assembly of a cluster shares at least one sequence with at least one other assembly of that cluster, wherein a cluster includes one or more assemblies;
analyzing the properties of each cluster to eliminate sequences from the list of candidates.

4. The computer-implemented method of claim 3, further comprising:
analyzing the properties of each cluster to determine new sequences from the clusters to be added to the first set and second set of sequences that are masked, further comprising:
adding the new sequences to the first set and to the second set; and
repeating masking with the first set and to the second set, comparing the masked genomes, creating a plurality of assemblies, and clustering the assemblies.

5. The method of claim 4, wherein analyzing the properties of a cluster to determine new sequences comprises:
determining the number of sequences of a cluster; and
if the number of sequences of a cluster is greater than a predetermined number, selecting the sequences of that cluster to be added to the first set and to the second set.

6. The computer-implemented method of claim 3, wherein at least one assembly is created from a second group of at least two pairs of homologous sequences, wherein the two sequences from the first genome of the second group overlap to form a first sequence, wherein the two sequences from the first genome of the second group overlap to form a second sequence, wherein the size variation between the first sequence and the second sequence is less than 10%, and wherein the alignment between the first sequence and the second sequence shows more than 85% sequence similarity.

7. The computer-implemented method of claim 3, wherein at least one assembly is created from a second group of at least two pairs of homologous sequences, wherein the two sequences from the first genome of the second group are consecutive and form a first sequence containing an intervening sequence, wherein the two sequences from the first genome of the second group are consecutive and form a second sequence containing an intervening sequence, wherein the size variation between the first sequence and the second sequence is less than 10%, and wherein the alignment between the first sequence and the second sequence shows more than 90% sequence similarity.

8. The computer-implemented method of claim 3, wherein analyzing the properties of a cluster includes:
if a cluster has more than a predetermined number of sequences, eliminating the sequences of that cluster from the list of candidates.

9. The computer-implemented method of claim 8, wherein the predetermined number is four.

10. The computer-implemented method of claim 8, wherein analyzing the properties of the cluster further includes:
flagging sequences that correspond to a coding gene as 'coding';
eliminating all of the sequences of the cluster if one of the sequences is flagged as 'coding'; and
eliminating the sequences of an assembly if the assembly is not identical by at least 85% within a minimal window of 60 nucleotides.

11. The computer-implemented method of claim 1, wherein analyzing comprises:
eliminating a pair of homologous sequences if a secondary structure resulting from a folding of the sequences does not satisfy each of a first set of criteria including:
a number of nucleotides in a pre-miRNA stem-loop;
a terminal hairpin being of a certain length;
a percentage similarity of the sequences between the two genomes; and
a Z score less than a specified amount; and
eliminating a pair of homologous sequences if a secondary structure resulting from a folding of the sequences does not satisfy a predetermined number of a second set of criteria including:
a minimal free energy (MFE) of less than a specified amount;
a GC content being within a certain percent range;
a base-paring number being within a certain percent range; and
perfect conservation of a sequence of a specified length along arms of the stem-loop.

12. The computer-implemented method of claim 11, wherein the predetermined number is three.

13. The computer-implemented method of claim 11, wherein the first set and-the second set of criteria have the following values:
the pre-miRNA stem-loop is between 60 to 120 nucleotides;
the percentage similarity of the sequences between the two genomes is at least 85 %;
at least one 17nt-long sequence is perfectly conserved along the arms of the stem-loop;
the terminal hairpin-loop is between 4 and 15 nucleotides;
the GC content is from 30 % to 51 %;
the base-pairing number is between 30 and 40 %;
the MFE is lower than -25 kcal/mol; and
the Z score is less than 0.06.

14. The computer-implemented method of claim 11, wherein analyzing further comprises:
when a sequence has a secondary structure satisfying the first set of criteria and a predetermined number of the second set of criteria, extracting exact positions of the 5' strand start and exact positions of the 3' strand end to make a new sequence;
folding the new sequence to from a new secondary structure; and
parsing the secondary structure of the new structure to determine if the secondary structure satisfies the first set of criteria and a predetermined number of the second set of criteria.

15. A computer-implemented method of identifying non-coding RNA candidates, comprising:
receiving at least a first and a second genome from two different species, each containing a plurality of sequences including highly repetitive DNA;
masking a first set of highly repetitive DNA sequences in the first genome, wherein the first set includes SINE and LINE interspersed repeat sequences, but does not include at least 75 % of other interspersed repeat sequences;
masking a second set of highly repetitive DNA sequences in the second genome, wherein the second set includes SINE and LINE interspersed repeat sequences, but does not include at least 75 % of the other interspersed repeat sequences;
comparing the masked first genome to the masked second genome to determine pairs of sequences that are homologous between the two genomes;
creating a list of non-coding RNA candidates from the homologous pairs;
creating a plurality of assemblies, each created from at least one pair of homologous sequences, wherein at least one assembly is created from a first group of at least two pairs of homologous sequences, wherein each pair of the first group has one sequence in common;
clustering the assemblies into a plurality of clusters, wherein each assembly of a cluster shares at least one sequence with at least one other assembly of that cluster, wherein a cluster includes one or more assemblies;
analyzing the properties of each cluster to eliminate sequences from the list of candidates;
analyzing the list of candidates to:
eliminate sequences having less than 60 nucleotides; and
identify sequences that have a stem loop secondary structure with a 5' strand stem having from 20 to 45 nucleotides and with a 3' strand stem having from 20 to 45 nucleotides; and
remove, from the list, sequences not having said stem-loop secondary structure;
eliminating a pair of homologous sequences if the secondary structure does not satisfy each of a first set of criteria; and
eliminating a pair of homologous sequences if the secondary structure does not satisfy a predetermined number of a second set of criteria.

## Patentansprüche

1. Computer-implementierte Methode zur Identifizierung von Mikro-RNA-Vorläufer-Kandidaten in nicht-kodierenden und kodierenden Regionen eines Genoms, umfassend:
Gewinnen wenigstens eines ersten und eines zweiten Genoms von zwei verschiedenen Spezies, das jeweils eine Vielzahl von Sequenzen einschließlich hochrepetitiver DNA enthält;
Maskieren eines ersten Satzes von hochrepetitiven DNA-Sequenzen in dem ersten Genom, wobei der erste Satz eingestreute SINE- und LINE-Wiederholungssequenzen enthält, jedoch mindestens 75% der anderen eingestreuten Wiederholungssequenzen nicht enthält;
Maskieren eines zweiten Satzes von hochrepetitiven DNA-Sequenzen in dem zweiten Genom, wobei der zweite Satz eingestreute SINE- und LINE-Wiederholungssequenzen enthält, jedoch mindestens 75% der anderen eingestreuten Wiederholungssequenzen nicht enthält;
Vergleichen des maskierten ersten Genoms mit dem maskieren zweiten Genom, um Paare von Sequenzen zu bestimmen, die zwischen den beiden Genomen homolog sind;
Erstellen einer Liste von Mikro-RNA-Vorläufer-Kandidaten aus den homologen Paaren; und
Analysieren der Liste von Kandidaten, um:
Sequenzen zu eliminieren, die weniger als 60 Nukleotide aufweisen; und
Sequenzen zu identifizieren, die eine Stamm-Schleife-Sekundärstruktur besitzen, die einen 5'-Strang im Stamm mit 20 bis 45 Nukleotiden und einen 3'-Strang im Stamm mit 20 bis 45 Nukleotiden aufweist; und
Entfernen von der Liste von Sequenzen, die diese Stamm-Schleife-Sekundärstruktur nicht aufweisen.

2. Computer-implementierte Methode nach Anspruch 1, wobei die anderen eingestreuten Wiederholungssequenzen prozessierte Pseudogene, Retrotranskripte, DNA-Transposons und retrovirale Retrotransposons enthalten.

3. Computer-implementierte Methode nach Anspruch 1, außerdem umfassend:
Schaffen einer Vielzahl von Anordnungen, die jeweils aus wenigstens einem Paar von homologen Sequenzen geschaffen sind, wobei wenigstens eine Anordnung aus einer ersten Gruppe aus wenigstens zwei Paaren von homologen Sequenzen geschaffen ist, wobei jedes Paar der ersten Gruppe eine gemeinsame Sequenz besitzt;
Clustern der Anordnungen zu einer Vielzahl von Clustern, wobei jede Anordnung eines Clusters wenigstens eine Sequenz mit wenigstens einer anderen Anordnung aus jenem Cluster teilt, wobei ein Cluster ein oder mehrere Anordnungen umfasst;
Analysieren der Eigenschaften jedes Clusters, um Sequenzen aus der Liste der Kandidaten zu eliminieren.

4. Computer-implementierte Methode nach Anspruch 3, außerdem umfassend:
Analysieren der Eigenschaften jedes Clusters, um neue Sequenzen aus den Clustern zu bestimmen, die dem ersten Satz und zweiten Satz der Sequenzen, die maskiert sind, hinzugefügt werden sollen, außerdem umfassend:
Hinzufügen der neuen Sequenzen zu dem ersten Satz und zu dem zweiten Satz; und
Wiederholen des Maskierens mit dem ersten Satz und mit dem zweiten Satz, Vergleichen der maskieren Genome, Schaffen einer Vielzahl von Anordnungen und Clustern der Anordnungen.

5. Methode nach Anspruch 4, wobei das Analysieren der Eigenschaften eines Clusters zur Bestimmung neuer Sequenzen umfasst:
Bestimmen der Anzahl von Sequenzen eines Clusters; und
dann, wenn die Anzahl der Sequenzen eines Clusters größer ist als eine zuvor festgelegte Anzahl, Auswählen der Sequenzen jenes Clusters, die dem ersten Satz und dem zweiten Satz hinzuzufügen sind.

6. Computer-implementierte Methode nach Anspruch 3, wobei wenigstens eine Anordnung aus einer zweiten Gruppe von wenigstens zwei Paaren von homologen Sequenzen geschaffen ist, worin die beiden Sequenzen aus dem ersten Genom der zweiten Gruppe überlappen und so eine erste Sequenz bilden, worin die beiden Sequenzen aus dem ersten Genom der zweiten Gruppe überlappen und so eine zweite Sequenz bilden, wobei die Größenvariation zwischen der ersten Sequenz und der zweiten Sequenz weniger als 10% beträgt, und wobei das Alignment zwischen der ersten Sequenz und der zweiten Sequenz mehr als 85% Sequenzähnlichkeit zeigt.

7. Computer-implementierte Methode nach Anspruch 3, wobei wenigstens eine Anordnung aus einer zweiten Gruppe aus wenigstens zwei Paaren von homologen Sequenzen geschaffen ist, worin die beiden Sequenzen aus dem ersten Genom der zweiten Gruppe konsekutiv sind und eine erste Sequenz bilden, die eine dazwischen liegende Sequenz enthält, worin die beiden Sequenzen aus dem ersten Genom der zweiten Gruppe konsekutiv sind und eine zweite Sequenz bilden, die eine dazwischen liegende Sequenz enthält, wobei die Größenvariation zwischen der ersten Sequenz und der zweiten Sequenz weniger als 10% beträgt und wobei das Alignment zwischen der ersten Sequenz und der zweiten Sequenz mehr als 90% Sequenzähnlichkeit zeigt.

8. Computer-implementierte Methode nach Anspruch 3, wobei das Analysieren der Eigenschaften eines Clusters beinhaltet:
dann, wenn ein Cluster mehr als eine zuvor festgelegte Anzahl von Sequenzen aufweist, das Eliminieren der Sequenzen aus jenem Cluster von der Liste der Kandidaten.

9. Computer-implementierte Methode nach Anspruch 8, wobei die zuvor festgelegte Anzahl Vier ist.

10. Computer-implementierte Methode nach Anspruch 8, wobei das Analysieren der Eigenschaften des Clusters außerdem beinhaltet:
mit Flag Versehen der Sequenzen, die einem kodierenden Gen entsprechen, als ,Kodierend';
Eliminieren alle der Sequenzen des Clusters, wenn eine der Sequenzen mit dem Flag ,Kodierend' versehen ist; und
Eliminieren der Sequenzen einer Anordnung, wenn die Anordnung nicht zu wenigstens 85% innerhalb eines Minimalfensters von 60 Nukleotiden identisch ist.

11. Computer-implementierte Methode nach Anspruch 1, wobei das Analysieren umfasst:
Eliminieren eines Paars von homologen Sequenzen, wenn eine Sekundärstruktur, die aus einer Faltung der Sequenzen resultiert, nicht jedes aus einem ersten Satz von Kriterien erfüllt, einschließlich:
einer Anzahl von Nukleotiden in einer pre-miRNA-Stamm-Schleife;
einer terminale Haarnadel, die von einer bestimmten Länge ist;
einer prozentualen Ähnlichkeit der Sequenzen zwischen den beiden Genomen; und
einem Z-Score von weniger als einer spezifizierten Menge; und
Eliminieren eines Paars von homologen Sequenzen, wenn eine Sekundärstruktur, die aus einer Faltung der Sequenzen resultiert, nicht eine zuvor festgelegte Anzahl von einem zweiten Satz von Kriterien erfüllt, einschließlich:
einer minimalen freien Energie (MFE) von weniger als einer spezifizierten Menge;
einem GC-Gehalt, der innerhalb eines bestimmten prozentualen Bereichs liegt;
einer Anzahl der Basenpaarungen, die innerhalb eines bestimmten prozentualen Bereichs liegt; und
der perfekten Konservierung einer Sequenz einer spezifizierten Länge entlang der Arme der Stamm-Schleife.

12. Computer-implementierte Methode nach Anspruch 11, wobei die zuvor festgelegte Anzahl Drei ist.

13. Computer-implementierte Methode nach Anspruch 11, wobei der erste Satz und der zweite Satz von Kriterien die folgenden Werte aufweist:
die pre-miRNA-Stamm-Schleife beträgt zwischen 60 und 120 Nukleotide;
die prozentuale Ähnlichkeit der Sequenzen zwischen den beiden Genomen beträgt wenigstens 85%;
wenigstens eine 17 nt lange Sequenz ist perfekt konserviert entlang der Arme der Stamm-Schleife;
die terminale Haarnadelschleife beträgt zwischen 4 und 15 Nukleotide;
der GC-Gehalt beträgt von 30% bis 51 %;
die Anzahl der Basenpaarungen beträgt zwischen 30 und 40%;
die MFE ist kleiner als -25 kcal/mol; und
der Z-Score beträgt weniger als 0,06.

14. Computer-implementierte Methode nach Anspruch 11, wobei das Analysieren außerdem umfasst:
dann, wenn eine Sequenz eine Sekundärstruktur aufweist, die den ersten Satz von Kriterien und eine zuvor festgelegte Anzahl aus dem zweiten Satz von Kriterien erfüllt, das Extrahieren der exakten Positionen des 5'-Strangstarts und der exakten Positionen des 3'-Strangendes, um eine neue Sequenz zu erhalten;
Falten der neuen Sequenz, um eine neue Sekundärstruktur zu bilden; und
Parse-Analysieren der Sekundärstruktur der neuen Sequenz, um zu bestimmen, ob die Sekundärstruktur den ersten Satz von Kriterien und eine zuvor festgelegte Anzahl aus dem zweiten Satz von Kriterien erfüllt.

15. Computer-implementierte Methode zur Identifizierung von nicht-kodierenden RNA-Kandidaten, umfassend:
Gewinnen wenigstens eines ersten und eines zweiten Genoms von zwei verschiedenen Spezies, das jeweils eine Vielzahl von Sequenzen einschließlich hochrepetitiver DNA enthält;
Maskieren eines ersten Satzes von hochrepetitiven DNA-Sequenzen in dem ersten Genom, wobei der erste Satz eingestreute SINE- und LINE-Wiederholungssequenzen enthält, jedoch mindestens 75% der anderen eingestreuten Wiederholungssequenzen nicht enthält;
Maskieren eines zweiten Satzes von hochrepetitiven DNA-Sequenzen in dem zweiten Genom, wobei der zweite Satz eingestreute SINE- und LINE-Wiederholungssequenzen enthält, jedoch mindestens 75% der anderen eingestreuten Wiederholungssequenzen nicht enthält;
Vergleichen des maskierten ersten Genoms mit dem maskieren zweiten Genom, um Paare von Sequenzen zu bestimmen, die zwischen den beiden Genomen homolog sind;
Erstellen einer Liste von nicht-kodierenden Vorläufer-Kandidaten aus den homologen Paaren;
Schaffen einer Vielzahl von Anordnungen, die jeweils aus wenigstens einem Paar von homologen Sequenzen geschaffen sind, wobei wenigstens eine Anordnung aus einer ersten Gruppe aus wenigstens zwei Paaren von homologen Sequenzen geschaffen ist, wobei jedes Paar der ersten Gruppe eine gemeinsame Sequenz besitzt;
Clustern der Anordnungen zu einer Vielzahl von Clustern, wobei jede Anordnung aus einem Clusters wenigstens eine Sequenz mit wenigstens einer anderen Anordnung aus jenem Cluster teilt, wobei ein Cluster ein oder mehrere Anordnungen enthält;
Analysieren der Eigenschaften jedes Clusters, um Sequenzen aus der Liste der Kandidaten zu eliminieren;
Analysieren der Liste von Kandidaten, um:
Sequenzen zu eliminieren, die weniger als 60 Nukleotide aufweisen; und
Sequenzen zu identifizieren, die eine Stamm-Schleife-Sekundärstruktur besitzen, die einen 5'-Strang im Stamm mit 20 bis 45 Nukleotiden und einen 3'-Strang im Stamm mit 20 bis 45 Nukleotiden aufweist; und
Entfernen von der Liste von Sequenzen, die die Stamm-Schleife-Sekundärstruktur nicht aufweisen;
Eliminieren eines Paars von homologen Sequenzen, wenn die Sekundärstruktur nicht jedes aus einem ersten Satz von Kriterien erfüllt; und
Eliminieren eines Paars von homologen Sequenzen, wenn die Sekundärstruktur nicht eine zuvor festgelegte Anzahl aus einem zweiten Satz von Kriterien erfüllt.

## Revendications

1. Procédé implémenté par ordinateur d'identification de candidats de précurseur de micro ARN dans des régions non codantes et codantes d'un génome, comprenant les étapes consistant à :
recevoir au moins un premier et un second génome de deux espèces différentes, contenant chacun une pluralité de séquences incluant de l'ADN hautement répétitif ;
masquer un premier jeu de séquences d'ADN hautement répétitif dans le premier génome, où le premier jeu inclut des séquences répétitives entremêlées SINE et LINE, mais n'inclut pas au moins 75 % des autres séquences répétitives entremêlées ;
masquer un second jeu de séquences d'ADN hautement répétitif dans le second génome, où le second jeu inclut des séquences répétitives entremêlées SINE et LINE, mais n'inclut pas au moins 75 % des autres séquences répétitives entremêlées ;
comparer le premier génome masqué au second génome masqué pour déterminer des paires de séquences qui sont homologues entre les deux génomes ;
créer une liste de candidats de précurseur de micro ARN à partir des paires homologues ; et
analyser la liste de candidats pour :
éliminer des séquences ayant moins de 60 nucléotides ; et
identifier des séquences qui ont une structure secondaire tige-boucle avec une tige de brin en 5' comportant de 20 à 45 nucléotides et avec une tige de brin en 3' comportant de 20 à 45 nucléotides ; et
enlever, de la liste, les séquences n'ayant pas la structure secondaire tige-boucle.

2. Procédé implémenté par ordinateur selon la revendication 1, dans lequel les autres séquences répétitives entremêlées incluent des pseudogènes traités, des produits de rétrotranscription, des transposons d'ADN et des rétrotransposons de rétrovirus.

3. Procédé implémenté par ordinateur selon la revendication 1, comprenant en outre les étapes consistant à :
créer une pluralité d'ensembles, chacun créé au moins à partir d'une paire de séquences homologues, où au moins un ensemble est créé à partir d'un premier groupe d'au moins deux paires de séquences homologues, où chaque paire du premier groupe a une séquence en commun ;
agréger les ensembles en une pluralité d'agrégats, où chaque ensemble d'agrégats partage au moins une séquence avec au moins un autre ensemble de cet agrégat, où un agrégat inclut un ou plusieurs ensembles ;
analyser les propriétés de chaque agrégat pour éliminer des séquences de la liste de candidats.

4. Procédé implémenté par ordinateur selon la revendication 3, comprenant en outre les étapes consistant à :
analyser les propriétés de chaque agrégat pour déterminer de nouvelles séquences à partir des agrégats à ajouter au premier jeu et au second jeu de séquences qui sont masqués, comprenant en outre les étapes consistant à :
ajouter les nouvelles séquences au premier jeu et au second jeu ; et
répéter le masquage avec le premier jeu et le second jeu, comparer les génomes masqués, créer une pluralité d'ensembles, et agréger les ensembles.

5. Procédé selon la revendication 4, dans lequel l'analyse des propriétés d'un agrégat pour déterminer de nouvelles séquences comprend les étapes consistant à :
déterminer le nombre de séquences d'un agrégat ; et
si le nombre de séquences d'un agrégat est plus grand qu'un nombre prédéterminé, sélectionner les séquences de cet agrégat à ajouter au premier jeu et au second jeu.

6. Procédé implémenté par ordinateur selon la revendication 3, dans lequel au moins un ensemble est créé à partir d'un second groupe d'au moins deux paires de séquences homologues, où les deux séquences du premier génome du second groupe se chevauchent pour former une première séquence, où les deux séquences du premier génome du second groupe se chevauchent pour former une seconde séquence, où la variation de taille entre la première séquence et la seconde séquence est inférieure à 10 %, et où l'alignement entre la première séquence et la seconde séquence montre plus de 85 % de similitude de séquence.

7. Procédé implémenté par ordinateur selon la revendication 3, dans lequel au moins un ensemble est créé à partir d'un second groupe d'au moins deux paires de séquences homologues, où les deux séquences du premier génome du second groupe sont consécutives et forment une première séquence contenant une séquence d'intervention, où les deux séquences provenant du premier génome du second groupe sont consécutives et forment une seconde séquence contenant une séquence d'intervention, où la variation de taille entre la première séquence et la seconde séquence est inférieure à 10 %, et où l'alignement entre la première séquence et la seconde séquence présente plus de 90 % de similitude de séquence.

8. Procédé implémenté par ordinateur selon la revendication 3, dans lequel l'analyse des propriétés d'un agrégat inclut l'étape consistant à :
si un agrégat a plus d'un nombre prédéterminé de séquences, éliminer les séquences de cet agrégat de la liste de candidats.

9. Procédé implémenté par ordinateur selon la revendication 8, dans lequel le nombre prédéterminé est de quatre.

10. Procédé implémenté par ordinateur selon la revendication 8, dans lequel l'analyse des propriétés de l'agrégat inclut en outre les étapes consistant à:
baliser les séquences qui correspondent à un gène codant comme « codantes » ;
éliminer toutes les séquences de l'agrégat si l'une des séquences est balisée comme « codante » ; et
éliminer les séquences d'un ensemble si l'ensemble n'est pas identique à hauteur d'au moins 85 % dans une fenêtre minimale de 60 nucléotides.

11. Procédé implémenté par ordinateur selon la revendication 1, dans lequel l'analyse comprend les étapes consistant à :
éliminer une paire de séquences homologues si une structure secondaire résultant d'un pliage des séquences ne satisfait pas chacun d'un premier jeu de critères comprenant :
un nombre de nucléotides dans une tige-boucle de pré-miARN ;
une boucle en épingle à cheveux terminale d'une certaine longueur ;
une similitude en pourcentage des séquences entre les deux génomes ; et
une note Z inférieure à une quantité spécifiée ; et
éliminer une paire de séquences homologues si une structure secondaire résultant d'un pliage des séquences ne satisfait pas un nombre prédéterminé d'un second jeu de critères incluant :
une énergie libre minimale (MFE) inférieure à une quantité spécifiée;
une teneur en GC dans une certaine plage de pourcentage ;
un nombre d'appariements de base dans une certaine plage de pourcentage ; et
une conservation parfaite d'une séquence d'une longueur spécifiée le long des brins de la tige-boucle.

12. Procédé implémenté par ordinateur selon la revendication 11, dans lequel le nombre prédéterminé est de trois.

13. Procédé implémenté par ordinateur selon la revendication 11, dans lequel le premier et le second jeu de critères ont les valeurs suivantes:
la tige-boucle de pré-miARN est comprise entre 60 et 120 nucléotides;
la similitude en pourcentage des séquences entre les deux génomes est d'au moins 85%;
au moins une séquence longue de 17nt est parfaitement conservée le long des brins de la tige-boucle;
la boucle en épingle à cheveux terminale est comprise entre 4 et 15 nucléotides;
la teneur en GC est de 30 % à 51%;
le nombre d'appariements de base est compris entre 30 et 40%;
la MFE est inférieure à -25 kcal/mol ; et
la note Z est inférieure à 0,06.

14. Procédé implémenté par ordinateur selon la revendication 11, dans lequel l'analyse comprend en outre les étapes consistant à :
lorsqu'une séquence a une structure secondaire satisfaisant le premier jeu de critères et un nombre prédéterminé du second jeu de critères, extraire des positions exactes du début du brin 5' et des positions exactes de la fin du brin 3' pour faire une nouvelle séquence ;
plier la nouvelle séquence pour former une nouvelle structure secondaire ; et
analyser la structure secondaire de la nouvelle structure pour déterminer si la structure secondaire satisfait le premier jeu de critères et un nombre prédéterminé du second jeu de critères.

15. Procédé implémenté par ordinateur d'identification de candidats d'ARN non codants, comprenant les étapes consistant à :
recevoir au moins un premier et un second génome de deux espèces différentes, chacun contenant une pluralité de séquences incluant de l'ADN hautement répétitif ;
masquer un premier jeu de séquences d'ADN hautement répétitif dans le premier génome, où le premier jeu inclut des séquences répétitives entremêlées SINE et LINE, mais n'inclut pas au moins 75 % d'autres séquences répétitives entremêlées;
masquer un second jeu de séquences d'ADN hautement répétitif dans le second génome, où le second jeu inclut des séquences répétitives entremêlées SINE et LINE, mais n'inclut pas au moins 75% des autres séquences répétitives entremêlées ;
comparer le premier génome masqué au second génome masqué pour déterminer des paires de séquences qui sont homologues entre les deux génomes ;
créer une liste de candidats d'ARN non codants à partir des paires homologues ;
créer une pluralité d'ensembles, chacun créé à partir d'au moins une paire de séquences homologues, où au moins un ensemble est créé à partir d'un premier groupe d'au moins deux paires de séquences homologues, où chaque paire du premier groupe a une séquence en commun ;
agréger les ensembles en une pluralité d'agrégats, où chaque ensemble d'agrégats partage au moins une séquence avec au moins un autre ensemble de cet agrégat, où un agrégat inclut un ou plusieurs ensembles ;
analyser les propriétés de chaque agrégat pour éliminer des séquences de la liste de candidats ;
analyser la liste de candidats pour :
éliminer des séquences ayant moins de 60 nucléotides ; et
identifier des séquences qui ont une structure secondaire tige-boucle avec une tige de brin en 5' comportant de 20 à 45 nucléotides et avec une tige de brin en 3' comportant de 20 à 45 nucléotides ; et
enlever, de la liste, les séquences n'ayant pas la structure secondaire tige-boucle;
éliminer une paire de séquences homologues si la structure secondaire ne satisfait pas chacun à un premier jeu de critères; et
éliminer une paire de séquences homologues si la structure secondaire ne satisfait pas un nombre prédéterminé d'un second jeu de critères.
